(19) Europäisches Patentamt
European Patent Office
Office européen des brevets

(11) EP 4 317 162 B1

(12) # EUROPEAN PATENT SPECIFICATION

(45) Date of publication and mention of the grant of the patent:
**04.02.2026 Bulletin 2026/06**

(21) Application number: **22778714.0**

(22) Date of filing: **24.03.2022**

(51) International Patent Classification (IPC):
***C07D 487/04*** (2006.01) ***A61K 31/519*** (2006.01)
***A61P 19/02*** (2006.01) ***A61P 37/00*** (2006.01)

(52) Cooperative Patent Classification (CPC):
**C07D 487/04; A61P 19/02; A61P 37/00**

(86) International application number:
**PCT/CN2022/082684**

(87) International publication number:
**WO 2022/206531 (06.10.2022 Gazette 2022/40)**

(54) **1,3-BENZODIOXOLANE-CONTAINING COMPOUND, AND PREPARATION METHOD THEREFOR AND USE THEREOF**

1,3-BENZODIOXOLANHALTIGE VERBINDUNG UND HERSTELLUNGSVERFAHREN DAFÜR UND VERWENDUNG DAVON

COMPOSÉ CONTENANT DU 1,3-BENZODIOXOLANE, SON PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.04.2021 CN 202110357313**

(43) Date of publication of application:
**07.02.2024 Bulletin 2024/06**

(73) Proprietor: **Xi'an Xintong Pharmaceutical Research Co., Ltd.**
**Xi'an, Shaanxi 710076 (CN)**

(72) Inventors:
- **XU, Yungen**
  **Shaanxi 710000 (CN)**
- **LIANG, Tingting**
  **Shaanxi 710000 (CN)**
- **ZHU, Qihua**
  **Shaanxi 710000 (CN)**
- **WANG, Junjie**
  **Shaanxi 710000 (CN)**
- **CEN, Lifang**
  **Shaanxi 710000 (CN)**
- **WANG, Wenjie**
  **Shaanxi 710000 (CN)**
- **JIN, Jiaming**
  **Shaanxi 710000 (CN)**

(74) Representative: **Murgitroyd & Company**
**165-169 Scotland Street**
**Glasgow G5 8PL (GB)**

(56) References cited:
**WO-A1-2011/090760** **WO-A1-2016/019233**
**WO-A1-2020/231990** **CN-A- 108 329 321**
**CN-A- 108 484 609** **CN-A- 109 970 740**
**US-A1- 2011 039 868**

- **YUAN YIN ET AL: "Structure-based design and synthesis of 1H-pyrazolo[3,4-d]pyrimidin-4-amino derivatives as Janus kinase 3 inhibitors", BIOORGANIC & MEDICINAL CHEMISTRY, vol. 26, no. 17, 1 September 2018 (2018-09-01), AMSTERDAM, NL, pages 4774 - 4786, XP055568887, ISSN: 0968-0896, DOI: 10.1016/j.bmc.2018.04.005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical field

The invention is within the field of medicinal chemistry, and specifically relates to a type of Bruton's tyrosine kinase (BTK) and Janus kinase subtype 3 (JAK3) kinase inhibitors containing 1,3-benzodioxol structures, and preparation methods therefor; it also includes pharmaceutical compositions containing these compounds and use of the compounds in the preparation of therapeutic drugs used for the treatment of autoimmune diseases such as rheumatoid arthritis or B cell lymphoma, or the drugs used to treat B cell malignancies. The references to methods of treatment in the subsequent paragraphs are to be interpreted as references to the compounds, pharmaceutical compositions and medicaments of the present invention for use in a method for treatment of the human (or animal) body by therapy.

### Technical background

Rheumatoid arthritis (RA) is a chronic, inflammatory synovitis-based autoimmune disease of unknown etiology, whose main pathological manifestations are inflammatory cell infiltration in synovial tissue, pannus formation, and progressive articular cartilage and bone deterioration, eventually leading to joint deformity or even loss of function.

Janus Kinase (JAK) includes non-receptor tyrosine kinases, including four subtypes, i.e. JAK1, JAK2, JAK3, and TYK2. A variety of JAK inhibitors are currently on the market, such as Tofacitinib, Ruxolitinib and Baricitinib. Tofacitinib was released in 2012 for the treatment of moderate to severe rheumatoid arthritis, and was approved for the treatment of psoriatic arthritis in 2017. However, tofacitinib has serious side effects including infection and anemia. This mainly occurs because tofacitinib is a pan-JAK inhibitor. While inhibiting JAK1 and JAK3 to produce therapeutic effects, tofacitinib also inhibits JAK2 activity and induces anemia as a side effect. Therefore, selective JAK1 or JAK3 inhibitors should be developed in the treatment of rheumatoid arthritis. Since JAK1 can form dimers with JAK3 and dimers/multimers with JAK2 or TYK2, it can cause side effects. Therefore, the side effects caused by the selective inhibition of JAK3 may be less intense than those caused by the inhibition of JAK1.

Bruton's tyrosine kinase (BTK) is a member of the TEC family and belongs to the group of non-receptor tyrosine kinases. It is mainly expressed in B cells, myeloid cells, mast cells, and platelets, but does not occur in T cells and NK cells. Bruton's tyrosine kinase plays a vital role in the B cell receptor signaling pathway. In addition, BTK is also involved in the conduction of many other signaling pathways. Abnormal activation of B cells can lead to B-cell lymphomas and autoimmune diseases. Three BTK inhibitors are currently on the market including Ibrutinib, Acalabrutinib and Zanubrutinib.

It has been reported that the combined use of BTK inhibitor LFM-A13 and JAK3 inhibitor JANEX-1 in the treatment of graft-versus-host reaction (GVHR) is much better than using LFM-A13, JANEX-1 or methotrexate alone. In addition, the JAK3 selective inhibitor PF-06651600 has shown good anti-RA effects in clinical practice. The reason is not only related to the inhibition of JAK3, but also closely related to the inhibition of TEC family kinases such as BTK. Therefore, the inventors believe that the simultaneous inhibition of BTK and JAK3 has a synergistic effect on the treatment of autoimmune diseases such as RA. However, those skilled in the art would prefer to combine existing BTK and JAK3 inhibitors; however, it is difficult to obtain compounds with excellent BTK and JAK3 inhibitory activities at the same time according to previous research. However, the inventors unexpectedly developed a series of compounds, which not only have inhibitory activity on BTK, but also have highly selective inhibitory activity on JAK3.

Yuan Yin et al: "Structure-based design and synthesis of 1Hpyrazolo[3,4-d]pyrimidin-4-amino derivatives as Janus kinase 3 inhibitors", Bioorg. & Med. Chem., vol. 26, no. 17, 1 September 2018 pages 4774-4786, reports the discovery and optimization of 1H-pyrazolo[3,4-d]pyrimidin-4- as covalent JAK3 inhibitors that exploit a unique cysteine (Cys909) residue in JAK3.

CN109970740 A relates to a 4-amino-pyrimidoazacycle derivative, a preparation method and application thereof. WO 2011/090760 A1 provides compounds, pharmaceutically acceptable compositions thereof, and methods of using the same.

CN108329321 A provides a drug capable of being used for preventing, treating and/or improving JAK related diseases such as inflammatory diseases and autoimmune diseases (such as psoriasis, rheumatoid arthritis, inflammatory bowel diseases, Sjogren's syndrome, Behcet's disease, disseminated sclerosis and systemic lupus erythematosus) and cancels (such as Castleman's disease, lymphoma, leukemia, multiple myeloma or myeloproliferation disease. The drug has excellent JAK kinase (janus kinase) inhibition activity.

CN108484609 A provides medicine for preventing, treating and/or relieving autoimmunity diseases such as Psoriasis, rheumatoid arthritis, inflammatory enteritis diseases, sjogren's syndrome, behcet's diseases, multiple sclerosis and systemic lupus erythematosus. The medicine has excellent JAK inhibitory activity.

US 2011/039868 A discloses compounds that form covalent bonds with Bruton's tyrosine kinase (Btk).

WO2016/019233 A1 discloses amido compounds that form covalent bonds with Bruton's tyrosine kinase (Btk).

WO 2020/231990 A1 relates to novel compounds and pharmaceutical compositions thereof, and methods for inhibiting the activity of FGFR enzymes with the compounds and compositions of the disclosure.

**Scope of the Invention**

The invention provides a class of compounds containing a 1,3-benzodioxol structure, which can simultaneously inhibit BTK and JAK3. In addition, the invention also provides specific preparation methods of the compounds and pharmaceutical application of the compounds as BTK and JAK3 kinase inhibitors.

The invention is defined by the claims and any other aspects, configurations or embodiments set forth herein not falling within the scope of the claims are for information only.

Specifically, the present invention provides a class of 1,3-benzodioxol derivatives represented by general formula (I) or pharmaceutically acceptable salts thereof:

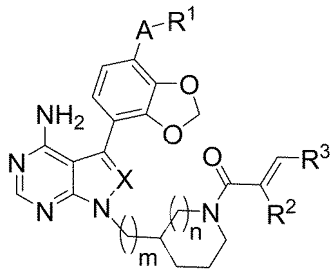

(I)

where X represents N or CH; A represents -NHCO or -$NHSO_2$-; *m* represents 0, and *n* represents 1; $R^1$ represents:

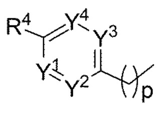, 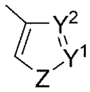, 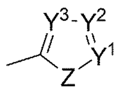, 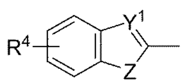, 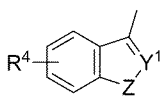, 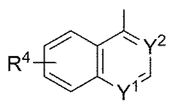

or

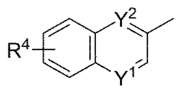,

where *p* represents 0 or 1; $R^4$ represents H, F, Cl, Br, I, $C_1$-$C_6$ alkyl, $CF_3$, OH, $C_1$-$C_6$ alkoxy, $OCF_3$, CN, $NO_2$, $NH_2$, $C_1$-$C_6$ alkylamino, $N(CH_3)_2$, $N(C_2H_5)_2$, $NHCOCH_3$, $CONH_2$, $CONHCH_3$, $CONHCH_2CF_3$, $OCH_2CH_2OCH_3$, $C_6H_5$, $R^4$ can be monosubstituted, disubstituted or trisubstituted; $Y^1$, $Y^2$, $Y^3$ or $Y^4$ represent N or C-$R^5$, $R^5$ represents H, F, Cl, Br, I, $CH_3$, $CF_3$, OH, $OCH_3$, $OCF_3$ or CN; Z represents O, S or N-$R^6$, $R^6$ represents H, $CH_3$ or $C_2H_5$ or cyclopropyl; $R^2$ and $R^3$ represents H,.

Preferably, in the general formula (I), , A is -NHCO-.

Preferably, the compounds of the invention have the following general formula (II):

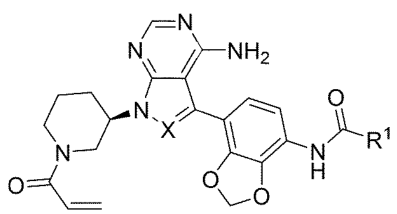

(II)

where X and $R^1$ are as defined above.

Preferably, in general formula (II), $R^1$ represents

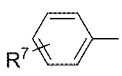, 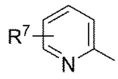, 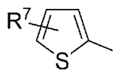, 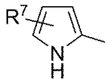,

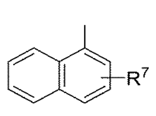, 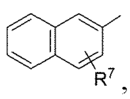, 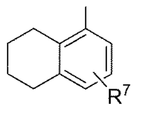, 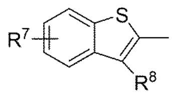 or 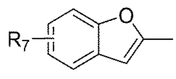,

where $R^7$ represents H, F, Cl, Br, CH3, t-Bu, $CH_3$, t-Bu, $CF_3$, CN, OH, $OCH_3$, $OCF_3$, $NH_2$, $N(CH_3)_2$, $N(C_2H_5)_2$, $NHCOCH_3$, $CONH_2$, $CONHCH_3$, $CONHCH_2CF_3$, $C_6H_5$,

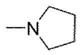, 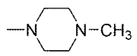 or, 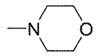;

and $R^7$ can be mono-, di- or tri-substituted, and $R^8$ represents H, Cl or $CH_3$.

Further, $R^7$ preferably represents H, Cl, $CH_3$, t-Bu, $CF_3$, $OCH_3$, $N(CH_3)_2$, $N(C_2H_5)_2$ or $CONHCH_2CF_3$; $R^7$ can be mono-, di- or tri-substituted; and $R^8$ preferably represents Cl.

More preferably, the 34 types of 1,3-benzodioxol derivatives of the invention are numbered sequentially as follows: I-1, I-2, I-3, and I-34. For example, the 1,3-benzodioxol derivatives of the invention are numbered sequentially as follows: I-1, I-2, I-3, and I-32.

Further preferably, the 1,3-benzodioxol derivatives of the invention are selected from the following compounds:

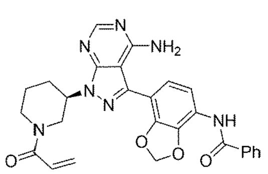
I-1

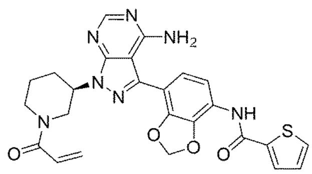
I-2

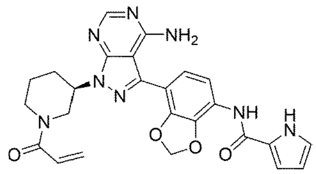
I-3

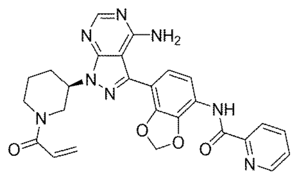
I-4

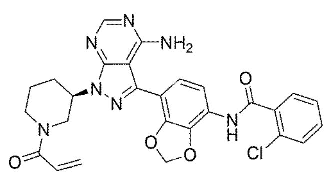
I-5

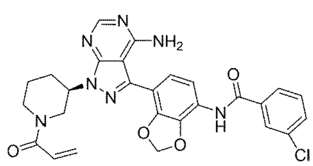
I-6

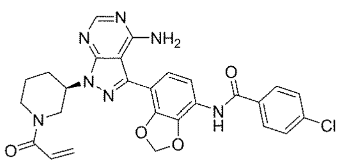
I-7

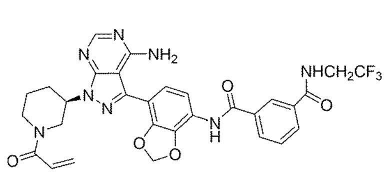
I-8

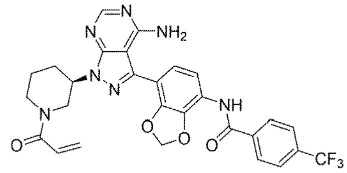
I-9

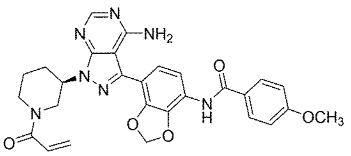
I-10

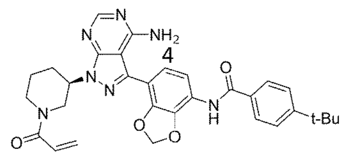
I-11

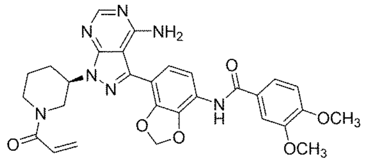
I-12

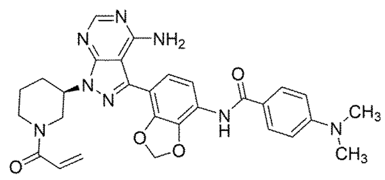

I-13

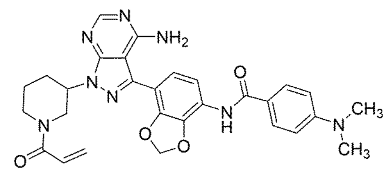

I-14

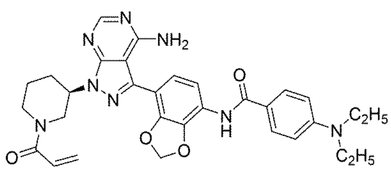

I-15

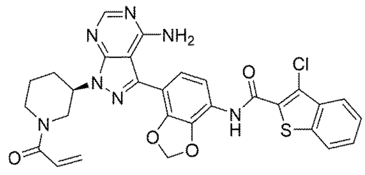

I-16

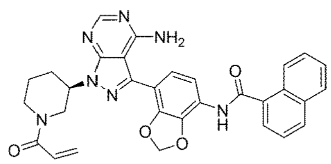

I-17

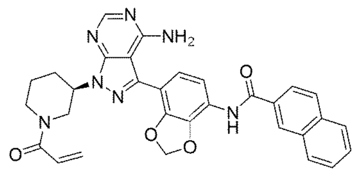

I-18

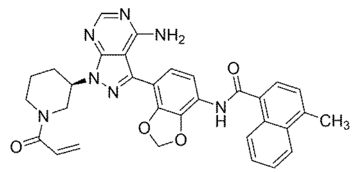

I-19

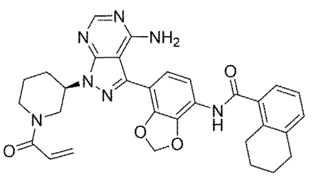

I-20

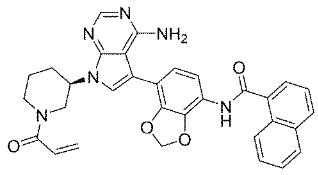

I-21

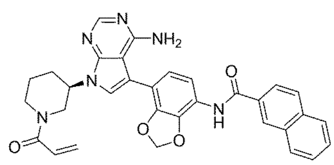

I-22

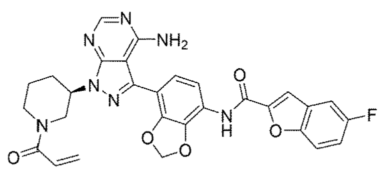

I-31

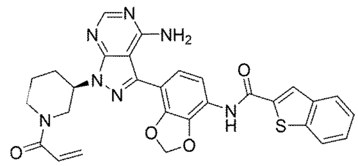

I-32

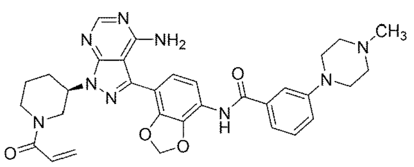

I-33 (reference example)

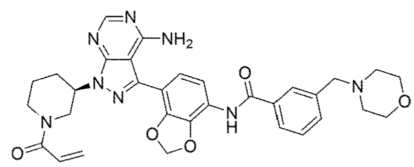

I-34 (reference example)

The pharmaceutically acceptable salt of the compound in the invention is the acid addition salt of the compound of general formula (I), where the acids used to form the salt are: hydrogen chloride, and hydrogen bromide, as well as the following acids: sulfuric, carbonic, oxalic, citric, succinic, tartaric, phosphoric, lactic, pyruvic, acetic, maleic, methanesulfonic, benzenesulfonic, p-toluenesulfonic, or ferulic acids.

The compound of general formula (I) of the invention can be prepared by the following methods:

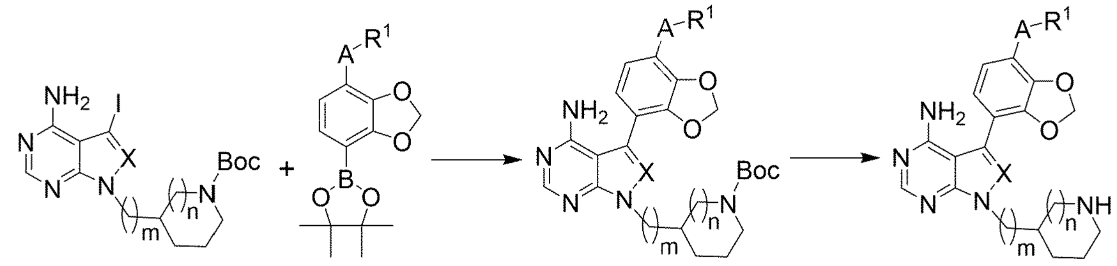

(III) (IV) (V) (VI)

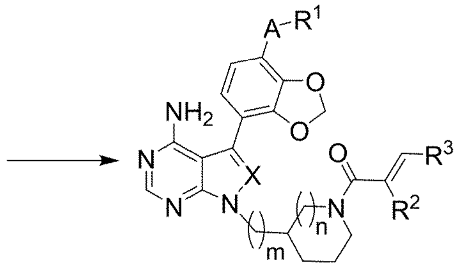

(I)

where definitions of $R^1$, $R^2$, $R^3$, X, A, m and n are the same as before.

Compound (V) is prepared by a Suzuki coupling reaction between compounds (III) and (IV); the solvent used is selected from toluene, *N, N*-dimethylformamide (DMF), ethylene glycol dimethyl ether, 1,4 -dioxane, tetrahydrofuran, methanol, ethanol, acetonitrile, acetone, water or a mixed solvent composed of any two of the above solvents, and preferably including ethylene glycol dimethyl ether. The base used is selected from sodium ethylate, sodium acetate, potassium acetate, potassium phosphate, sodium hydroxide, potassium hydroxide, potassium carbonate, sodium carbonate or triethylamine, and preferably sodium carbonate or potassium carbonate; the catalyst used is selected from tetrakis (triphenylphosphine) palladium (Pd $(PPh_3)_4$), [1, 1'-bis (diphenylphosphino)ferrocene]palladium dichloride ($Pd(dppf)Cl_2$), bis(triphenylphosphino)palladium dichloride ($Pd(PPh_3)_2Cl_2$), palladium acetate (Pd $(OAc)_2$) or (1,1'-bis(diphenylphosphino)ferrocene)nickel dichloride ($NiCl_2(dppf)$), and preferably $Pd(dppf)Cl_2$. The reaction temperature is selected from room temperature to 120 °C, and preferably 70-100°C.

Compound (VI) is prepared from compound (V) by removing the tert-butoxycarbonyl (Boc) protecting group, using a reagent selected from trifluoroacetic acid, concentrated hydrochloric acid, hydrogen chloride/ethyl acetate solution or hydrogen chloride/methanol solution, and preferably trifluoroacetic acid or hydrogen chloride/ethyl acetate solution. The reaction solvent is selected from acetone, dichloromethane, ethyl acetate, 1,4-dioxane or acetonitrile, and preferably ethyl acetate or dichloromethane.

Compound (I) is prepared by reacting compound (VI) with the corresponding acid chloride; the acid-binding agent used is selected from triethylamine, pyridine, N,N-diisopropylethylamine, 4-dimethylaminopyridine, potassium carbonate or sodium carbonate, and preferably triethylamine. The solvent used is selected from dichloromethane, tetrahydrofuran, 1,4-dioxane, ethyl acetate, acetone, toluene or DMF, and preferably dichloromethane.

Compound (I) can also be prepared by reacting compound (VI) with the corresponding acid in the presence of a condensing agent selected from N, N'-carbonyldiimidazole (CDI), 1-(3-dimethylaminopropyl)-3-ethylcarbodiimide hydrochloride (EDCI)/1-hydroxybenzotriazole (HOBt) or dicyclohexylcarbodiimide (DCC)/4-dimethylaminopyridine (DMAP), while EDCI/HOBt is preferred.

When A represents -NHCO-, *m* represents 0, n represents 1, $R^2$, $R^3$ represents H, and when the C atom at the 3-position of piperidine is in the R configuration, the compound of the general formula (II) in the invention can be prepared by the following method:

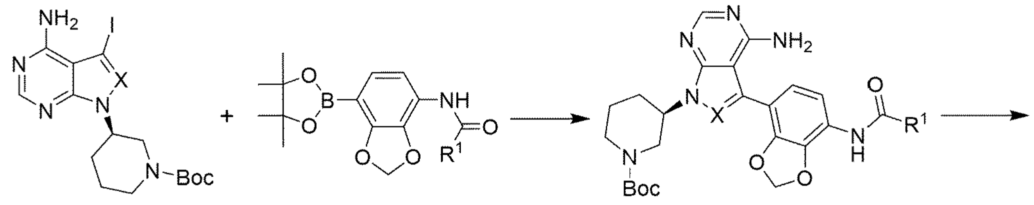

(III-A) (IV-A) (V-A)

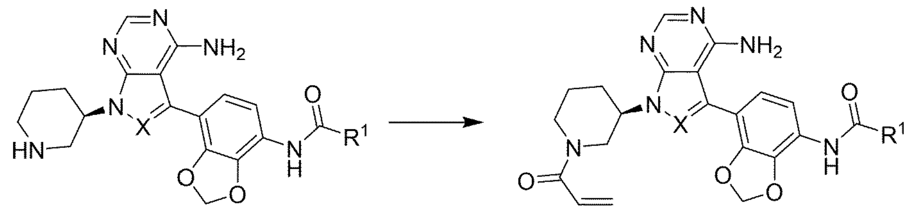

(VI-A) (II)

where definitions of $R^1$ and X are the same as before.

Compound (V-A) is prepared by the Suzuki coupling reaction between compounds (III-A) and (IV-A). The solvent used is selected from toluene, DMF, ethylene glycol dimethyl ether, 1,4-dioxane, tetrahydrofuran, methanol, ethanol, acetonitrile, acetone, water or a mixed solvent of any two of the above solvents, and preferably ethylene glycol dimethyl ether. The base used is selected from sodium ethylate, sodium acetate, potassium acetate, potassium phosphate, sodium hydroxide, hydrogen potassium oxide, potassium bicarbonate, potassium carbonate, sodium carbonate or triethylamine, and preferably sodium carbonate or potassium carbonate. The catalyst used is selected from $Pd(PPh_3)_4$, $Pd(dppf)Cl_2$, $Pd(PPh_3)_2Cl_2$, $Pd(OAc)_2$ or $NiCl_2(dppf)$, and preferably $Pd(dppf)Cl_2$. The reaction temperature is selected from 25-120°C, and preferably 70-100°C. The reaction time is selected from 2-24 h, and preferably 12-18 h.

Compound (VI-A) is prepared from compound (V-A) by removing the Boc protecting group using a reagent selected from trifluoroacetic acid, concentrated hydrochloric acid, hydrogen chloride/ethyl acetate solution or hydrogen chloride/methanol solution, and preferably hydrogen chloride/ethyl acetate solution. The reaction solvent is selected from acetone, dichloromethane, ethyl acetate, 1,4-dioxane or acetonitrile, and preferably ethyl acetate or dichloromethane.

Compound (II) is prepared by reacting compound (VI-A) with acryloyl chloride. The acid-binding agent used is selected from triethylamine, pyridine, *N,N*-diisopropylethylamine, 4-dimethylaminopyridine, potassium carbonate or sodium carbonate, and preferably triethylamine. The solvent used is selected from dichloromethane, tetrahydrofuran, acetone, toluene or DMF, and preferably dichloromethane.

Compound (II) can also be prepared by reacting compound (VI-A) with acrylic acid in the presence of a condensing agent selected from CDI, EDCI/HOBt or DCC/DMAP, and preferably EDCI/HOBt.

Intermediate (III) can be prepared by the following method:

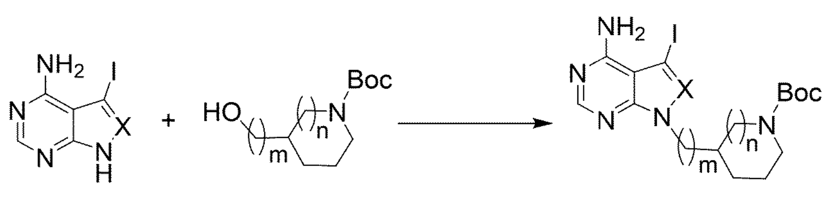

(VII) (VIII) (III)

where definitions of X, *m* and *n* are the same as before.

Compound (III) is prepared by the Mitsunobu reaction of compounds (VII) and (VIII). The phosphine ligand used is selected from triphenylphosphine ($PPh_3$) or tri-n-butylphosphine ($n\text{-}Bu_3P$), and preferably triphenylphosphine. The azo reagent used is selected from diethyl azodicarbonate (DEAD), diisopropyl azodicarbonate (DIAD) or *N,N,N',N'*-tetramethylazodicarbonamide (TMAD), and preferably DIAD. The solvent used is selected from tetrahydrofuran, 1,4-dioxane, diethyl ether, dichloromethane, toluene, acetonitrile or DMF, and preferably tetrahydrofuran.

Intermediate (IV) can be prepared by the following methods:

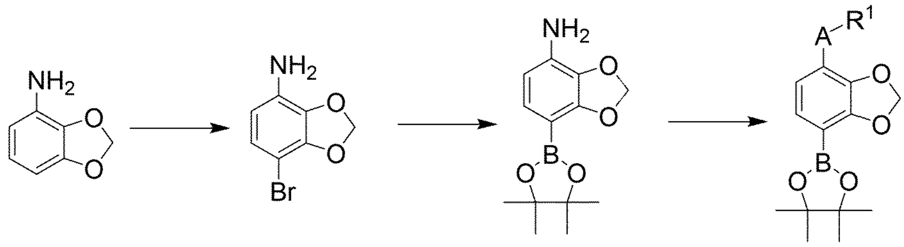

(IX) (X) (XI) (IV)

where A is selected from -NHCO-, $-NHCOCH_2-$ or $-NHSO_2-$, and $R^1$ is as defined above.

Benzo[d][1,3]dioxo heterocyclopenten-4-amine (IX) is dissolved in DMF, and N-bromosuccinimide (NBS) is added for bromination to obtain 7-bromo benzo[d][1,3]dioxo heterocyclopenten-4-amine (X).

Compound (X) and biboronic acid pinacol ester are dissolved in 1,4-dioxane, and react in the presence of a catalyst, $Pd(dppf)Cl_2$, and potassium acetate to obtain 7-(4,4,5,5-tetra methyl-1,3,2-dioxaborolan-2-)benzo[d][1,3]dioxo heterocyclopenten-4-amine (XI).

The intermediate (IV) is prepared by reacting compound (XI) with the corresponding acid chloride. The acid-binding agent used is selected from triethylamine, pyridine, *N,N*-diisopropylethylamine, 4-dimethylaminopyridine, potassium carbonate or sodium carbonate, and preferably *N,N*-diisopropylethylamine or triethylamine. The solvent used is selected from dichloromethane, tetrahydrofuran, 1,4-dioxane, ethyl acetate, acetone, toluene or DMF, and preferably dichloromethane.

The invention also discloses a pharmaceutical composition, which contains the compound of general formula (I) or pharmaceutically acceptable salt thereof and a pharmaceutically acceptable carrier. The compound can be made into common pharmaceutical preparations by adding pharmaceutically acceptable carriers, such as tablets, capsules, syrups, suspensions, and injections as well; spices, sweeteners, liquid or solid fillers or diluents can be added as medical excipients.

The pharmaceutical composition of the invention can be administered by methods known to those skilled in the art, such as oral, rectal, sublingual, pulmonary, transdermal, iontophoresis, vaginal and intranasal administration. The pharmaceutical composition of the invention is preferably administered parenterally, such as by subcutaneous, intramuscular or intravenous injection. The dose varies according to the form of the preparation, the expected duration of action, and the condition of the subject to be treated. The therapeutically effective dose required for actual treatment can be determined by a doctor according to the actual situation (such as the patient's condition, body weight, etc.). For normal adults, the dose of the pharmaceutical composition of the invention based on the compound of general formula (I) may be 1 ng to 10 g per kg body weight of an adult, and preferably 10 $\mu$g to 100 mg per kg body weight.

The use of the compound of general formula (I) or stereoisomers, hydrates, solvates, crystals or pharmaceutically acceptable salts thereof in the preparation of BTK and/or JAK3 inhibitor drugs is also protected by the invention. Correspondingly, the invention also provides a treatment method, which includes administering a therapeutically effective amount of the compound of general formula (I) of the invention or stereoisomer, hydrated substances, solvates, crystals or pharmaceutically acceptable salts thereof.

Preferably, the invention provides the compound of general formula (I) or the stereoisomer, hydrate, solvate, crystal or pharmaceutically acceptable salt thereof in the preparation of BTK and JAK3 dual-target inhibitor drug.

Further, the BTK and JAK3 inhibitors therein are used for the treatment of autoimmune diseases and B-cell malignancies, and preferably, the autoimmune diseases include rheumatoid arthritis, psoriasis and alopecia areata, and others, and the B-cell malignancies include B-cell lymphoma and so on. Correspondingly, the invention also provides a method for treating an autoimmune disease or a B-cell malignancy, which includes administering a therapeutically effective dose of the compound of general formula (I) or the stereoisomer, hydrate, solvate, crystal or pharmaceutically acceptable salt thereof in the invention to a subject suffering from an autoimmune disease or a B-cell malignancy.

The beneficial effect of the invention is to provide 1,3-benzodioxol derivatives represented by the general formula (I). The results of pharmacological trials show that the compound (I) of the invention can produce excellent inhibition effects on both BTK and JAK3 kinases, and can be used to prepare drugs for rheumatoid arthritis caused by over-activation of BTK and JAK3 pathways. The 1,3-benzodioxol derivatives of the invention have high selectivity for inhibiting the activity of JAK3, and basically no inhibitory activity on JAK1 and JAK2. The invention also provides a highly efficient preparation method of the 1,3-benzodioxol derivatives.

## Specific Implementations

The present application will be described in detail below in conjunction with specific implementations.

Example 1

Synthesis of ((*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-)benzamide (I-1)

Synthesis of (*R*)-3-(4-amino-3-iodo-1*H*-pyrazolo[3,4-*d*]pyrimidin-1)piperidine-1-tert-butyl formate (III-1)

4-Amino-3-iodo-1*H*-pyrazolo[3,4-*d*]pyrimidine (VII-1) (1.80 g, 6.90 mmol), (*S*)-1-tert-butoxycarbonyl-3-hydroxy piperidine (VIII-1) (2.78 g, 13.79 mmol), $PPh_3$ (3.61 g, 13.79 mmol), and anhydrous THF (100 mL) were added to a 250 mL three-necked flask under nitrogen protection. The mixture was stirred for 30 min under ice bath, and DIAD (2.40 g, 13.79 mmol) was added; the mixture was reacted at room temperature for 18 h. Next, THF was distilled off under reduced pressure, the residue was dissolved in ethyl acetate (100 mL), and washed successively with saturated sodium carbonate (30 mL × 3), water (30 mL × 3), saturated sodium chloride (30 mL × 3), and was dried by anhydrous $Na_2SO_4$. After suction filtration, the filtrate was evaporated to remove the solvent under reduced pressure to obtain yellow oil, which was separated by column chromatography (eluent: Dichloromethane: Methanol=150-90:1), 2.06 g of white solid was obtained. The yield was 67.2%, m.p. at 178-180°C. $^1$H-NMR (300 MHz, $CDCl_3$) $\delta$ (ppm): 8.34 (s, 1H, ArH), 6.27 (s, 2H, $NH_2$), 4.85-4.66 (m, 1H, $CHC\underline{H_2}N$), 4.36-4.00 (m, 2H, $C\underline{H}CH_2N$), 3.47-3.21 (m, 1H, $CHCH_2CH_2C\underline{H_2}N$), 2.92-2.72 (m, 1H, $CHCH_2CH_2C\underline{H_2}N$), 2.28-2.01 (m, 2H, $CHC\underline{H_2}CH_2CH_2N$), 1.97-1.79 (m, 1H, $CHCH_2C\underline{H_2}CH_2N$), 1.76-1.57 (m, 1H, $CHCH_2C\underline{H_2}CH_2N$), 1.45 (s, 9H, NBoc).

Synthesis of 7-bromobenzo[d][1,3]dioxo heterocyclopenten-4-amine (X)

Benzo[*d*][1,3]dioxo heterocyclopenten-4-amine (IX) (1.24 g, 9.04 mmol) and DMF (30 mL) were mixed; then, the mixture was stirred to conduct dissolution and cooling to -30°C. Next, NBS (1.69 g, 9.50 mmol) was added in batches; after the addition was completed, the mixture was incubated and reacted for 1 h. Water (60 mL) was added, the mixture was extracted with ethyl acetate (15 mL × 3), the organic phase was washed successively with water (10 mL × 3) and saturated sodium chloride solution (10 mL × 3), and then dried over anhydrous sodium sulfate. After suction filtration, the filtrate was evaporated to dryness under reduced pressure, and the residue was subjected to column chromatography (eluent: Petroleum ether: Ethyl acetate=30-10:1) to obtain 1.01 g of white solid with a yield of 51.7%, m.p. at 99-100°C. $^1$H-NMR (300 MHz, $CDCl_3$) $\delta$ (ppm): 6.80 (d, *J* = 8.67 Hz, 1H, ArH), 6.23 (d, *J* = 8.67 Hz, 1H, ArH), 6.02 (s, 2H, $OCH_2O$), 3.58 (s, 2H, $NH_2$).

Synthesis of 7-(4,4,5,5-Tetramethyl-1,3,2-dioxaborolan-2-yl)benzo[*d*][1,3]dioxo heterocyclopenten-4-amine (XI)

Compound X (3.14 g, 14.54 mmol), bis(pinacolato)diboron (7.39 g, 29.07 mmol), Pd(dppf)$Cl_2$ (0.53 g, 0.73 mmol), potassium acetate (4.28 g, 43.62 mmol) and 1,4-dioxane (25 mL) were mixed under $N_2$ protection. Next, the temperature was raised to 95°C for reaction for 12 h. Suction filtration was completed by using diatomaceous earth. The filtrate was distilled off the solvent under reduced pressure, and was then dissolved in ethyl acetate (20 mL), washed with water (10 mL × 3) and saturated sodium chloride solution (10 mL × 3) successively. Next, the filtrate was dried over anhydrous sodium sulfate; then, suction filtration was used and the filtrate was evaporated to dryness under reduced pressure. The residue was subjected to column chromatography (eluent: Petroleum ether: Dichloromethane = 1:5-10) to obtain 2.70 g of white solid with a yield of 70.6%, mp. 84-86°C. $^1$H-NMR (300 MHz, $CDCl_3$) $\delta$ (ppm): 7.09 (d, *J* = 8.16 Hz, 1H, ArH), 6.31 (d, *J*= 8.19 Hz, 1H, ArH), 5.99 (s, 2H, $OCH_2O$), 3.71 (br, 2H, $NH_2$), 1.34 (s, 12H, $4CH_3$).

Synthesis of *N*-(7-(4,4,5,5-tetramethyl-1,3,2-dioxaborolan-2-yl)benzo[*d*][1,3]dioxo heterocyclopenten-4-)benzamide (IV-1)

Compound XI (0.56 g, 2.14 mmol) was dissolved in dichloromethane (10 mL); then, *N,N*-diisopropylethylamine (0.83 g, 6.42 mmol) was added, and benzoyl chloride (0.45 g, 3.21 mmol) in dichloromethane solution was added dropwise, after which the mixture was reacted at a room temperature for 30 min. The reaction solution was washed successively with 1 mol/L hydrochloric acid (10 mL × 3), saturated sodium bicarbonate solution (10 mL × 3), water (10 mL × 3) and saturated sodium chloride solution (10 mL × 3), and was then dried over anhydrous $Na_2SO_4$. After suction filtration, the filtrate was evaporated to dryness under reduced pressure, and the residue was subjected to column chromatography (eluent: Petroleum ether: Dichloromethane = 1:1-8) to obtain 409 mg of white solid with a yield of 52.1%, m.p. at 202-204°C. $^1$H-NMR (300 MHz, $CDCl_3$) $\delta$ (ppm): 7.89-7.88 (m, 4H, ArH), 7.57-7.47 (m, 3H, ArH, NH), 7.29 (d, *J* = 8.73 Hz, 1H, ArH), 6.07 (s, 2H, $OCH_2O$), 1.36 (s, 12H, $4CH_3$).

Synthesis of (*R*)-3-(4-amino-3-(7-benzamidobenzo[*d*] [1,3]dioxo heterocyclopenten-4)-1*H*-pyrazolo[3,4-*d*]pyrimidin-1-) piperidine-1-tert-butyl formate (V-1)

Compound III-1 (1.50 g, 3.38 mmol) and IV-1 (1.49 g, 4.05 mmol) were added into a 100 mL single-neck flask. Next, 2 mol/L sodium carbonate solution (3.38 mL), Pd(dppf)$Cl_2$ (0.12 g, 0.17 mmol) and ethylene glycol dimethyl ether (40 mL) were added under nitrogen protection, a reaction of the mixture was conducted at 85 °C for 17 h. Cooling was conducted to a room temperature, suction filtration was conducted through diatomaceous earth, the filtrate was concentrated under reduced pressure, then the filtrate was dissolved in ethyl acetate (40 mL), and washed with water (10 mL × 3), and saturated sodium chloride (10 mL × 3), and the product was concentrated under reduced pressure to obtain 2.56 g crude. Column chromatography (eluent: Dichloromethane: Methanol=100-70:1),after separation 926 mg of light-yellow solid was obtained with a yield of 49.2%, m.p. at 112-114°C. $^1$H-NMR (300 MHz, $CDCl_3$+$D_2O$) $\delta$ (ppm): 8.36 (s, 1H, ArH), 8.05-7.98 (m, 1H, ArH), 7.92 (d, *J* = 7.23 Hz, 2H, ArH), 7.64-7.49 (m, 3H, ArH), 7.15 (d, *J* = 8.19 Hz, 1H, ArH), 6.13 (s, 2H, $OCH_2O$), 4.92-4.79 (m, 1H, $CHC\underline{H}_2NBoc$), 4.42-4.23 (m, 1H, $C\underline{H}CH_2NBoc$), 3.57-3.28 (m, 2H, $C\underline{H}_2NC\underline{H}_2$), 2.92-2.76 (m, 1H, $NC\underline{H}_2CH_2$), 2.32-2.16 (m, 2H, $NCH_2CHC\underline{H}_2$), 1.93-1.81 (m, 2H, $NCH_2C\underline{H}_2$), 1.45 (s, 9H, NBoc).

Synthesis of (*R*)-N-(7-(4-amino-1-(piperidin-3)-1*H*-pyrazolo[3,4-*d*]pyrimidin-3-)benzo[*d*][1,3]dioxo heterocyclopenten-4-)benzamide (VI-1)

Compound V-1 (0.93 g, 1.66 mmol) was dissolved in ethyl acetate (6 mL); next, a saturated solution of hydrogen chloride in ethyl acetate (10 mL) was added and the mixture was stirred at a room temperature for 4 h during which a large amount of solids precipitated out. 20 mL of water was added, the layers were separated, the aqueous layer was washed twice with ethyl acetate, the pH was adjusted to 8-9 with 1 mol/L NaOH, a solid then precipitated, the solid was subjected to suction filtration, and dried to obtain 721 mg of a light-yellow solid with a yield of 94.8% . The solid was directly submitted to the next step without further purification.

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)benzamide (I-1)

Compound VI-1 (93 mg, 0.20 mmol) was dissolved in 5 mL of anhydrous dichloromethane. Next, triethylamine (31 mg, 0.30 mmol) was added, and the anhydrous dichloromethane solution of acryloyl chloride (22 mg, 0.24 mmol) was added dropwise under ice bath; the dropwise addition was completed, and the mixture was stirred at a room temperature for 3.5 h. Washing was conducted with water (10 mL × 3) and saturated sodium chloride solution (10 mL × 3), and the mixture was concentrated under reduced pressure to obtain a crude product. Residue column chromatography was conducted (eluent: Dichloromethane: Methanol=100-35:1), and 77 mg of white solid was isolated with a yield of 74.0%, m.p. at 141-142.5°C. $^1$H-NMR (300 MHz, $CDCl_3$) $\delta$ (ppm): 8.39 (s, 1H, ArH), 8.35-8.25 (m, 1H, NH), 7.93 (d, *J* = 7.26 Hz, 2H, ArH), 7.86-7.76 (m, 1H, ArH), 7.60-7.44 (m, 3H, ArH), 7.13-7.03 (m, 1H, ArH), 6.67-6.52 (m, 1H, $CH=CH_2$), 6.34-6.18 (m, 2H, $CH=CH_2$, $NH_2$), 6.09 (s, 2H, $OCH_2O$), 5.75-5.60 (m, 1H, $CH=CH_2$), 4.93-4.74 (m, 1.5H, $NC\underline{H}_2C\underline{H}$), 4.65-4.51 (m, 0.5H, $NCH_2C\underline{H}$), 4.27-4.14 (m, 0.5H, $NC\underline{H}_2CH$), 4.08-3.94 (m, 0.5H, $NC\underline{H}_2CH$), 3.85-3.68 (m, 0.5H, $NC\underline{H}_2CH_2$), 3.40-3.26 (m, 0.5H, $NC\underline{H}_2CH_2$), 3.24-3.08 (m, 0.5H, $NC\underline{H}_2CH_2$), 2.92-2.85 (m, 0.5H, $NC\underline{H}_2CH_2$), 2.81 (s, 1H, $NH_2$), 2.46-2.30 (m, 1H, $NCH_2CHC\underline{H}_2$), 2.29-2.18 (m, 1H, $NCH_2CHC\underline{H}_2$), 2.06-1.91 (m, 1H, $NCH_2C\underline{H}_2$), 1.79-1.61 (m, 1H, $NCH_2C\underline{H}_2$). HRMS (ESI): m/z $[M+H]^+$. Calcd for $C_{27}H_{26}N_7O_4$: 512.2046; found: 512.2045.

Example 2

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4-)thiophene-2-formamide (I-2)

Synthesis of *N*-(7-(4,4,5,5-tetramethyl-1,3,2-dioxaborolan-2-)benzo[*d*][1,3]dioxo heterocyclopenten-4-)thiophene-2-formamide (IV-2)

Thiophene-2-formic acid (0.73 g, 5.70 mmol) was dissolved in anhydrous dichloromethane (12 mL), 3 drops of DMF was added, and the anhydrous dichloromethane solution of oxalyl chloride (1.09 g, 8.55 mmol) was added dropwise under ice bath. After drop addition, stirring was conducted at a room temperature. TLC (petroleum ether: Ethyl acetate = 3:1) was used for monitoring. After the reaction of the raw materials was completed, the solvent was removed under reduced pressure, and anhydrous dichloromethane (15 mL) was added to dissolve the residue for use.

Compound XI (1.00 g, 3.80 mmol) and dichloromethane (20 mL) were added into a 50 mL eggplant-shaped flask, stirring was conducted for dissolution, *N,N*-diisopropylethylamine (1.47 g, 11.40 mmol) was added, and under ice-bath, the above-mentioned oxalyl chloride was added dropwise, and the mixture was reacted at room temperature for 6 h. The

organic phase was washed successively with 1 mol/L hydrochloric acid (10 mL × 3), water (10 mL), saturated sodium bicarbonate solution (10 mL), saturated sodium chloride solution (10 mL × 3), and was dried over anhydrous sodium sulfate. Suction filtration was conducted, and the filtrate was concentrated under reduced pressure to obtain a light-yellow solid. Petroleum ether: ethyl acetate was beaten at a ratio of 8:1 (1 g/6 mL) to obtain 1.25 g of white solid with a yield of 88.1%, which was directly used in the next step without further purification.

Synthesis of (*R*)-3-(4-amino-3-(7-(thiophene-2-carboxamido)benzo[*d*][1,3]dioxo heterocyclopenten-4)-1*H* -pyrazolo [3,4-*d*]pyrimidin-1-)piperidine-1-tert-butyl formate (V-2)

Compound III-1 (0.90 g, 2.03 mmol) and IV-2 (0.91 g, 2.16 mmol) were used as raw materials, the operation process was the same as that of compound V-1 to obtain 583 mg of light-yellow solid with a yield of 51.1%, m.p. at 142-144°C. $^1$H-NMR (300 MHz, $CDCl_3$) $\delta$ (ppm): 8.35 (s, 1H, ArH), 7.94-7.87 (m, 2H, ArH, NH), 7.69 (d, *J* = 3.66 Hz, 1H, ArH), 7.60 (d, *J* = 5.01 Hz, 1H, ArH), 7.20-7.08 (m, 2H, ArH), 6.12 (s, 2H, $OCH_2O$), 5.93 (br, 2H, $NH_2$), 4.93-4.78 (m, 1H, $CHC\underline{H}_2NBoc$), 4.46-4.07 (m, 2H, $C\underline{H}C\underline{H}_2NBoc$), 3.56-3.29 (m, 1H, $NC\underline{H}_2CH_2$), 2.94-2.75 (m, 1H, $NC\underline{H}_2CH_2$), 2.35 -2.13 (m, 2H, $NCH_2CHC\underline{H}_2$), 1.98-1.82 (m, 1H, $NCH_2C\underline{H}_2$), 1.77-1.59 (m, 1H, $NCH_2C\underline{H}_2$), 1.45 (s, 9H, NBoc).

Synthesis of (*R*)-*N*-(7-(4-amino-1-(piperidin-3)-1*H*-pyrazolo[3,4-*d*]pyrimidin-3-)benzo[*d*][1,3]dioxo heterocyclopenten-4)-thiophene-2-formamide (VI-2)

Compound V-2 (400 mg, 0.71 mmol) was used as the raw material, the procedure was the same as that of compound VI-1 to obtain 329 mg of light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4-)thiophene-2-formamide (I-2)

Compound VI-2 (300 mg, 0.65 mmol) and acryloyl chloride (64 mg, 0.71 mmol) were used as raw materials, the procedure was the same as that of compound I-1 to obtain 131 mg of white solid with a yield of 39.1%, m.p. at 140-142°C. $^1$H-NMR (300 MHz, $CDCl_3$) $\delta$ (ppm): 8.37 (s, 1H, ArH), 8.02 (s, 1H, NH), 7.89 (d, *J* = 7.98 Hz, 1H, ArH), 7.73 (d, *J* = 3.27 Hz, 1H, ArH), 7.62 (d, *J* = 4.74 Hz, 1H, ArH), 7.18 (d, *J* = 3.60 Hz, 1H, ArH), 7.12 (d, *J* = 8.70 Hz, 1H, ArH), 6.72-6.54 (m, 1H, $CH=CH_2$), 6.39-6.25 (m, 1H, $CH=CH_2$), 6.14 (s, 2H, $OCH_2O$), 5.96 (s, 1H, $NH_2$), 5.79-5.63 (m, 1H, $CH=CH_2$), 4.99-4.82 (m, 1.5H, $NC\underline{H}_2C\underline{H}$), 4.71-4.58 (m, 0.5H, $NCH_2C\underline{H}$), 4.32-4.18 (m, 0.5H, $NC\underline{H}_2CH$), 4.12-3.99 (m, 0.5H, $NC\underline{H}_2CH$), 3.89-3.73 (m, 0.5H, $NC\underline{H}_2CH_2$), 3.47-3.32 (m, 0.5H, $NC\underline{H}_2CH_2$), 3.30-3.14 (m, 0.5H, $NC\underline{H}_2CH_2$), 2.97-2.79 (m, 0.5H, $NC\underline{H}_2CH_2$), 2.49-2.34 (m, 1H, $NCH_2CHC\underline{H}_2$), 2.33-2.23 (m, 1H, $NCH_2CHC\underline{H}_2$), 2.20 (s, 1H, $NH_2$), 2.07-1.96 (m, 1H, $NCH_2C\underline{H}_2$), 1.83-1.67 (m, 1H, $NCH_2C\underline{H}_2$); HRMS (ESI): m/z $[M+H]^+$. Calcd for $C_{25}H_{24}N_7O_4S$: 518.1610; found: 518.1611.

Example 3

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4)-1*H*-pyrrole-2-formamide (I-3)

Synthesis of *N*-(7-(4,4,5,5-tetramethyl-1,3,2-dioxaborolan-2-yl)benzo[d][1,3]dioxo heterocyclopenten-4)-1*H*-pyrrole-formamide (IV-3)

Compound XI (1.00 g, 3.80 mmol) and pyrrole-2-carboxylic acid (0.63 g, 5.70 mmol) were used as raw materials, the operation process was the same as that of compound IV-2 to obtain 1.17 g of white solid with a yield of 86.4%, which was directly fed into next step without further purification.

Synthesis of (*R*)-3-(3-(7-(1*H*-pyrrole-2-formamide)benzo[*d*][1,3]dioxo heterocyclopenten-4)-4-amino- 1*H*-pyrazolo [3,4-*d*]pyrimidin-1-)piperidine-1-tert-butyl formate (V-3)

Compound III-1 (0.90 g, 2.03 mmol) and IV-3 (1.36 g, 3.85 mmol) were used as raw materials, the operation process was the same as that of compound V-1 to obtain 550 mg of light-yellow solid with a yield of 49.5%, m.p. at 138-140°C. $^1$H-NMR (400 MHz, $CDCl_3$) $\delta$ (ppm): 11.67 (s, 1H, NH), 8.42 (s, 1H, ArH), 7.77 (d, *J* = 8.52 Hz, 1H, ArH), 7.70 (s, 1H, NH), 7.15-7.10 (m, 1H, ArH), 7.08 (d, *J* = 8.60 Hz, 1H, ArH), 6.87-6.82 (m, 1H, ArH), 6.38-6.31 (m, 1H, ArH), 6.11 (s, 2H, $OCH_2O$), 5.30 (s, 1H, $NH_2$), 4.92-4.78 (m, 1H, $CHC\underline{H}_2NBoc$), 4.50-4.24 (m, 1H, $C\underline{H}CH_2NBoc$), 4.24-4.03 (m, 1H, $CHC\underline{H}_2NBoc$), 3.58-3.29 (m, 1H, $NC\underline{H}_2CH_2$), 2.91-2.75 (m, 1H, $NC\underline{H}_2CH_2$), 2.36-2.16 (m, 3H, $NCH_2CHC\underline{H}_2$, $NH_2$), 1.96-1.82 (m, 1H, $NCH_2C\underline{H}_2$), 1.78-1.63 (m, 1H, $NCH_2C\underline{H}_2$), 1.45 (s, 9H, NBoc).

Synthesis of (*R*)-*N*-(7-(4-amino-1-(piperidin-3)-1*H*-pyrazolo[3,4-*d*]pyrimidin-3-)benzo[*d*][1,3]dioxo heterocyclopenten-4)-1*H*-pyrrole-2-formamide (VI-3)

Compound V-3 (450 mg, 0.82 mmol) was used as the raw material, the procedure was the same as that of compound VI-1 to obtain 383 mg of light-yellow solid, which was directly used in the next step without further purification.

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4)-1*H*-pyrrole-2-formamide (I-3)

Compound VI-3 (360 mg, 0.81 mmol) and acryloyl chloride (77 mg, 0.85 mmol) were used as raw materials, the procedure was the same as that of compound I-1 to obtain 212 mg of white solid with a yield of 52.5%, m.p. at 156-158°C. $^1$H-NMR (300 MHz, $CDCl_3$+$D_2O$) $\delta$ (ppm): 8.40 (s, 1H, ArH), 7.80-7.60 (m, 1H, ArH), 7.18-6.99 (m, 2H, ArH), 6.94-6.81 (m, 1H, ArH), 6.70-6.52 (m, 1H, CH=$CH_2$), 6.43-6.22 (m, 2H, ArH, CH=$CH_2$), 6.09 (s, 2H, $OCH_2O$), 5.80-5.60 (m, 1H, CH=$CH_2$), 5.07-4.54 (m, 4H, N$C\underline{H}_2$C$\underline{H}$, $NH_2$), 4.31-4.15 (m, 0.5H, N$C\underline{H}_2$CH), 4.10-3.93 (m, 0.5H, N$C\underline{H}_2$CH), 3.87-3.65 (m, 0.5H, N$C\underline{H}_2CH_2$), 3.46-3.30 (m, 0.5H, N$C\underline{H}_2CH_2$), 3.26-3.11 (m, 0.5H, N$C\underline{H}_2CH_2$), 2.95-2.76 (m, 0.5H, N$C\underline{H}_2CH_2$), 2.48-2.14 (m, 2H, $NCH_2$CH$C\underline{H}_2$), 2.05-1.89 (m, 1H, $NCH_2C\underline{H}_2$), 1.83-1.60 (m, 1H, $NCH_2C\underline{H}_2$); HRMS (ESI): m/z $[M+H]^+$. Calcd for $C_{25}H_{25}N_8O_4$: 501.1999; found: 501.2003.

Example 4

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4)pyridine-2-formamide (I-4)

Synthesis of *N*-(7-(4,4,5,5-tetramethyl-1,3,2-dioxaborolan-2-yl)benzo[*d*][1,3]dioxo heterocyclopenten-4)-pyridine-2-formamide (IV-4)

Compound XI (0.70 g, 2.66 mmol) and pyridine-2-formyl chloride hydrochloride (0.57 g, 3.19 mmol) were used as raw materials, the operation process was the same as that of compound IV-1 to obtain 612 mg of off-white solid with a yield of 62.5%, m.p. at 190-192°C. $^1$H-NMR (300 MHz, $CDCl_3$) $\delta$ (ppm): 10.11 (s, 1H, NH), 8.70-8.66 (m, 1H, ArH), 8.29 (d, *J* = 7.65 Hz, 1H, ArH), 8.00 (d, *J* = *8.43* Hz, 1H, ArH), 7.93 (t, *J* = 7.56 Hz, 1H, ArH), 7.54-7.44 (m, 1H, ArH), 7.30 (d, *J* = 8.55 Hz, 1H, ArH), 6.11 (s, 2H, $OCH_2O$), 1.37 (s, 12H, $4CH_3$).

Synthesis of (*R*)-3-(4-amino-3-(7-(pyridine-2-formamidyl)benzo[*d*][1,3]dioxo heterocyclopenten-4)-1*H*-pyrazolo[3,4-*d*] pyrimidin-1-)piperidine-1-tert-butyl formate (V-4)

Compound III-1 (0.80 g, 1.80 mmol) and compound IV-4 (0.80 g, 2.16 mmol) were used as raw materials, the operation process was the same as that of compound V-1 to obtain 476 mg of a light-yellow solid with a yield of 47.4%, m.p. at 118-120°C. $^1$H-NMR (300 MHz, $CDCl_3$) $\delta$ (ppm): 10.17 (s, 1H, NH), 8.70-8.66 (m, 1H, ArH), 8.39 (s, 1H, ArH), 8.33 (d, *J* = 7.77 Hz, 1H, ArH), 8.17 (d, *J* = 8.70 Hz, 1H, ArH), 7.96 (td, $J_1$ = 7.74 Hz, $J_2$ = 1.65 Hz, 1H, ArH), 7.58-7.51 (m, 1H, ArH), 7.18 (d, *J* = 8.70 Hz, 1H, ArH), 6.18 (s, 2H, $OCH_2O$), 5.79 (s, 2H, $NH_2$), 4.94-4.81 (m, 1H, CH$C\underline{H}_2$NBoc), 4.43-4.27 (m, 1H, C$\underline{H}$$CH_2$NBoc), 4.23-4.10 (m, 1H, CH$C\underline{H}_2$NBoc), 3.52-3.39 (m, 1H, N$C\underline{H}_2CH_2$), 2.94-2.79 (m, 1H, N$C\underline{H}_2CH_2$), 2.34-2.27 (m, 1H, $NCH_2$CH$C\underline{H}_2$), 2.26-2.20 (m, 1H, $NCH_2$CH$C\underline{H}_2$), 1.98-1.90 (m, 2H, $NCH_2C\underline{H}_2$), 1.47 (s, 9H, NBoc).

Synthesis of (*R*)-*N*-(7-(4-amino-1-(piperidin-3)-1*H*-pyrazolo[3,4-*d*]pyrimidin-3-)benzo[d][1,3]dioxo heterocyclopenten-4-)pyridine-2-formamide (VI-4)

Compound V-4 (0.40 g, 0.72 mmol) was dissolved in anhydrous dichloromethane (5 mL), trifluoroacetic acid (5 mL) was added, and stirring was conducted at a room temperature for 10 h. The solvent was evaporated under reduced pressure, the residue was dissolved in ethyl acetate, the pH was adjusted to 8-9 with 1 mol/L NaOH, the layers were separated, the aqueous layer was extracted once with ethyl acetate, the organic layer was washed with saturated sodium chloride (10 mL $\times$ 3), then drying was conducted over anhydrous sodium sulfate. After suction filtration, the filtrate was concentrated under reduced pressure to obtain 280 mg of a light-yellow product with a yield of 85.37%. The product was directly submitted to the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)pyridine-2-formamide (I-4)

Compound VI-4 (230 mg, 0.50 mmol), acrylic acid (43 mg, 0.60 mmol), HOBt (102 mg, 0.75 mmol), EDCI (145

mg, 0.75 mmol) and TEA (102 mg, 1.00 mmol) were dissolved in 6 mL DMF, and then stirred at room temperature for 12 h. Next, 30 mL of water was added, and extraction was conducted with ethyl acetate (10 mL × 3), and the organic layer was sequentially washed with 1 mol/L hydrochloric acid (10 mL × 3), saturated sodium carbonate (10 mL × 3) and saturated sodium chloride (10 mL × 3) and then was dried over anhydrous sodium sulfate. Suction filtration was conducted, and the filtrate was concentrated under reduced pressure to obtain a crude product. Column chromatography (eluent: Dichloromethane: Methanol=100-50:1) to obtain 201 mg of white solid with a yield of 78.2%, m.p. at 154-156°C. $^1$H-NMR (300 MHz, $CDCl_3$) $\delta$ (ppm): 10.11 (s, 1H, NHCO), 8.61 (s, 1H, ArH), 8.36-8.21 (m, 2H, ArH), 8.16-8.03 (m, 1H, ArH), 7.97-7.84 (m, 1H, ArH), 7.55-7.40 (m, 1H, ArH), 7.16-7.02 (m, 1H, ArH), 6.64-6.46 (m, 1H, $CH{=}CH_2$), 6.34-6.19 (m, 1H, $CH{=}CH_2$), 6.12 (s, 2H, $OCH_2O$), 5.78 (s, 1H, $NH_2$), 5.68-5.57 (m, 1H, $CH{=}CH_2$), 4.96-4.74 (m, 1.5H, $NC\underline{H}_2C\underline{H}$), 4.65-4.52 (m, 0.5H, $NCH_2C\underline{H}$), 4.24-4.12 (m, 0.5H, $NC\underline{H}_2CH$), 4.05-3.92 (m, 0.5H, $NC\underline{H}_2CH$), 3.80-3.67 (m, 0.5H, $NC\underline{H}_2CH_2$), 3.42-3.26 (m, 0.5H, $NC\underline{H}_2CH_2$), 3.20-3.07 (m, 0.5H, $NC\underline{H}_2CH_2$), 2.90-2.73 (m, 0.5H, $NC\underline{H}_2CH_2$), 2.44-2.15 (m, 2H, $NCH_2CHC\underline{H}_2$), 2.04-1.84 (m, 3H, $NCH_2C\underline{H}_2$, $NH_2$); HRMS (ESI): m/z $[M+H]^+$. Calcd for $C_{26}H_{25}N_8O_4$: 513.1999; found: 513.1999.

Example 5

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-2-chlorobenzamide (I-5)

Synthesis of 2-Chloro-*N*-(7-(4,4,5,5-tetramethyl-1,3,2-dioxaborolan-2-)benzo[d][1, 3]dioxo heterocyclopenten -4-)benzamide (IV-5)

Compound XI (0.80 g, 3.04 mmol) and o-chlorobenzoyl chloride (0.80 g, 4.56 mmol) were used as raw materials, the operation process was the same as that of compound IV-1 to obtain 800 mg of white solid with a yield of 65.5%, m.p. at 96-98°C. $^1$H-NMR (300 MHz, $CDCl_3$) $\delta$ (ppm): 8.16 (s, 1H, NHCO), 7.91 (d, *J*= 8.37 Hz, 1H, ArH), 7.81 (d, *J*= 6.57 Hz, 1H, ArH), 7.48-7.37 (m, 3H, ArH), 7.31 (d, *J* = 8.22 Hz, 1H, ArH), 6.08 (s, 2H, $OCH_2O$), 1.39 (s, 12H, $4CH_3$).

Synthesis of (R)-3-(4-amino-3-(7-(2-chlorobenzamido)benzo[d][1,3]dioxo heterocyclopenten-4)-1*H*-tert-butyl-pyrazolo [3,4-*d*]pyrimidin-1-)piperidine-1-tert-butyl formate (V-5)

Compound III-1 (0.65 g, 1.46 mmol) and IV-5 (0.71 g, 1.76 mmol) were used as raw materials, the operation process was the same as that of compound V-1 to obtain 651 mg of light-yellow solid with a yield of 75.2%, m.p. at 124-126°C. $^1$H-NMR (300 MHz, $CDCl_3$) $\delta$ (ppm): 8.36 (s, 1H, ArH), 8.16 (s, 1H, NH), 8.06 (d, *J* = 8.58 Hz, 1H, ArH), 7.86 (d, *J* = 6.24 Hz, 1H, ArH), 7.52-7.40 (m, 3H, ArH), 7.15 (d, *J* = 8.70 Hz, 1H, ArH), 6.12 (s, 2H, $OCH_2O$), 5.75 (s, 2H, $NH_2$), 4.93-4.77 (m, 1H, $CHC\underline{H}_2NBoc$), 4.44-4.23 (m, 1H, $C\underline{H}CH_2NBoc$), 4.22-4.04 (m, 1H, $CHC\underline{H}_2NBoc$), 3.54-3.35 (m, 1H, $NC\underline{H}_2CH_2$), 2.91-2.76 (m, 1H, $NC\underline{H}_2CH_2$), 2.32-2.18 (m, 2H, $NCH_2CHC\underline{H}_2$), 1.78-1.61 (m, 2H, $NCH_2C\underline{H}_2$), 1.45 (s, 9H, NBoc).

Synthesis of (R)-N-(7-(4-amino-1-(piperidin-3)-1H-pyrazolo[3,4-d]pyrimidin-3)benzo[d][1,3]dioxo heterocyclopenten-4)-2-chlorobenzamide (VI-5)

Compound V-5 (600 mg, 1.01 mmol) was used as the raw material, the procedure was the same as that of compound VI-1 to obtain 524 mg of light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-2-chlorobenzamide (I-5)

Compound VI-5 (450 mg, 0.91 mmol) and acryloyl chloride (87 mg, 0.96 mmol) were used as raw materials, the procedure was the same as that of compound I-1 to obtain 270 mg of a white solid with a yield of 54.0%, m.p. at 156.5-158°C. $^1$H-NMR (300 MHz, $CDCl_3$) $\delta$ (ppm): 8.39-8.27 (m, 2H, ArH, CONH), 8.03 (d, *J* = 8.61 Hz, 1H, ArH), 7.83 (d, *J* = 7.47 Hz, 1H, ArH), 7.52-7.36 (m, 3H, ArH), 7.13 (d, *J* = 8.55 Hz, 1H, ArH), 6.68-6.52 (m, 1H, $CH{=}CH_2$), 6.34-6.22 (m, 1H, $CH{=}CH_2$), 6.12 (s, 2H, $OCH_2O$), 6.04 (s, 1H, $NH_2$), 5.76-5.61 (m, 1H, $CH{=}CH_2$), 4.95-4.79 (m, 1.5H, $NC\underline{H}_2C\underline{H}$), 4.70-4.56 (m, 0.5H, $NCH_2C\underline{H}$), 4.28-4.16 (m, 0.5H, $NC\underline{H}_2CH$), 4.09-3.96 (m, 0.5H, $NC\underline{H}_2CH$), 3.84-3.70 (m, 0.5H, $NC\underline{H}_2CH_2$), 3.44-3.29 (m, 0.5H, $NC\underline{H}_2CH_2$), 3.25-3.11 (m, 0.5H, $NC\underline{H}_2CH_2$), 2.92-2.77 (m, 0.5H, $NC\underline{H}_2CH_2$), 2.52-2.19 (m, 3H, $NCH_2CHC\underline{H}_2$, $NH_2$), 2.04-1.91 (m, 1H, $NCH_2C\underline{H}_2$), 1.82-1.63 (m, 1H, $NCH_2C\underline{H}_2$); HRMS (ESI): m/z $[M+H]^+$. Calcd for $C_{27}H_{25}ClN_7O_4$: 546.1657; found: 546.1656.

Example 6

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-3-chlorobenzamide (I-6)

Synthesis of 3-chloro-*N*-(7-(4,4,5,5-tetramethyl-1,3,2-dioxaborolan-2)benzo[*d*][1,3]dioxo heterocyclopenten -4-)benzamide (IV-6)

Compound XI (0.80 g, 3.04 mmol) and m-chlorobenzoyl chloride (0.80 g, 4.56 mmol) were used as raw materials, the operation process was the same as that of compound IV-1. In this case, 987 mg of white solid was obtained, the yield was 80.8%, and m.p. was at 150-152°C. $^1$H-NMR (300 MHz, $CDCl_3$) $\delta$ (ppm): 7.88-7.86 (m, 1H, ArH), 7.84-7.80 (m, 2H, ArH, NH), 7.74 (d, *J*= 7.68 Hz, 1H, ArH), 7.53 (d, *J*= 8.04 Hz, 1H, ArH), 7.43 (t, *J*= 7.80 Hz, 1H, ArH), 7.28 (d, *J*= 8.31 Hz, 1H, ArH), 6.07 (s, 2H, $OCH_2O$), 1.36 (s, 12H, $4CH_3$).

Synthesis of (*R*)-3-(4-amino-3-(7-(3-chlorobenzamido)benzo[d][1,3]dioxo heterocyclopenten-4)-1*H*-tert-butyl-pyrazolo [3,4-*d*]pyrimidin-1-)piperidine-1-tert-butyl formate (V-6)

Compound III-1 (0.80 g, 1.80 mmol) and IV-6 (0.87 g, 2.16 mmol) were used as raw materials, the operation process was the same as that of compound V-1 to obtain 1.02 g (as crude product) of a light-yellow solid with a yield of 95.6%, m.p. at 128-130°C. $^1$H-NMR (300 MHz, $CDCl_3$) $\delta$ (ppm): 8.35(s, 1H, ArH), 8.03(s, 1H, NH), 7.95-7.85(m, 2H, ArH), 7.79 (d, *J*=7.29 Hz, 1H, ArH), 7.55(d, *J*=7.98Hz, 1H, ArH), 7.44(t, *J*=7.71Hz, 1H, ArH), 7.13(d, *J*= 8.58 Hz, 1H, ArH), 6.12(s, 2H, $OCH_2O$), 5.89(s, 2H, $NH_2$), 4.93-4.75(m, 1H, $CHC\underline{H}_2NBoc$), 4.47-4.22 (m, 1H, $C\underline{H}CH_2NBoc$), 4.21-4.02 (m, 1H, $CHC\underline{H}_2NBoc$), 3.56-3.29 (m, 1H, $NC\underline{H}_2CH_2$), 2.93-2.75 (m, 1H, $NC\underline{H}_2CH_2$), 2.36-2.19 (m, 2H, $NCH_2CHC\underline{H}_2$), 1.97-1.79 (m, 1H, $NCH_2C\underline{H}_2$), 1.77-1.60 (m, 1H, $NCH_2C\underline{H}_2$), 1.45 (s, 9H, Boc).

Synthesis of (*R*)-*N*-(7-(4-amino-1-(piperidin-3)-1H-pyrazolo[3,4-d]pyrimidin-3-)benzo[d][1,3]dioxo heterocyclopenten-4)-3-chlorobenzamide (VI-6)

Compound V-6 (1.00 g, 1.69 mmol) was used as the raw material, the operation process was the same as that of compound VI-1 to obtain 882 mg of light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-3-chlorobenzamide (I-6)

Compound VI-6 (500 mg, 1.02 mmol) and acryloyl chloride (97 mg, 1.07 mmol) were used as raw materials, the procedure was the same as that of compound I-1 to obtain 372 mg of white solid with a yield of 67.0%, m.p. at 143-144.5°C. $^1$H-NMR (300 MHz, $CDCl_3$) $\delta$ (ppm): 8.34 (d, *J* = 11.04 Hz, 1H, ArH), 8.21 (d, *J* = 9.12 Hz, 1H, ArH), 7.92 (s, 1H, NH), 7.90-7.77 (m, 2H, ArH), 7.55 (d, *J* = 7.77 Hz, 1H, ArH), 7.45 (t, *J* = 7.65 Hz, 1H, ArH), 7.16-7.05 (m, 1H, ArH), 6.68-6.52 (m, 1H, $CH=CH_2$), 6.34-6.22 (m, 1H, $CH=CH_2$), 6.12 (s, 2H, $OCH_2O$), 6.02 (s, 1H, $NH_2$), 5.76-5.61 (m, 1H, $CH=CH_2$), 4.95-4.78 (m, 1.5H, $NC\underline{H}_2C\underline{H}$), 4.68-4.55 (m, 0.5H, $NCH_2C\underline{H}$), 4.28-4.16 (m, 0.5H, $NC\underline{H}_2CH$), 4.10-3.97 (m, 0.5H, $NC\underline{H}_2CH$), 3.85-3.69 (m, 0.5H, $NC\underline{H}_2CH_2$), 3.45-3.29 (m, 0.5H, $NC\underline{H}_2CH_2$), 3.26-3.11 (m, 0.5H, $NC\underline{H}_2CH_2$), 2.93-2.79 (m, 0.5H, $NC\underline{H}_2CH_2$), 2.49-2.18 (m, 3H, $NCH_2CHC\underline{H}_2$, $NH_2$), 2.07-1.91 (m, 1H, $NCH_2C\underline{H}_2$), 1.81-1.64 (m, 1H, $NCH_2C\underline{H}_2$); HRMS (ESI): m/z $[M+H]^+$. Calcd for $C_{27}H_{25}ClN_7O_4$: 546.1657; found: 546.1662.

Example 7

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4)-4-chlorobenzamide (I-7)

Synthesis of 4-chloro-*N*-(7-(4,4,5,5-tetramethyl-1,3,2-dioxaborolan-2)benzo[*d*][1, 3]dioxo heterocyclopenten-4-)benzamide (IV-7)

Compound XI (0.80 g, 3.04 mmol) and p-chlorobenzoyl chloride (0.80 g, 4.56 mmol) were used as raw materials, the operation process was the same as that of compound IV-1 to obtain 1.02 g of white solid with a yield of 83.6%, m.p. at 210-212°C. $^1$H-NMR (300 MHz, $CDCl_3$) $\delta$ (ppm): 7.99-7.76 (m, 4H, ArH), 7.60-7.45 (m, 2H, ArH, NH), 7.32-7.26 (m, 1H, ArH), 6.07 (s, 2H, $OCH_2O$), 1.36 (s, 12H, $4CH_3$).

Synthesis of (R)-3-(4-amino-3-(7-(4-chlorobenzamido)benzo[*d*] [1,3]dioxo heterocyclopenten-4)-1*H* -tert-butyl-pyrazolo[3,4-*d*]pyrimidin-1-)piperidine-1-tert-butyl formate (V-7)

Compound III-1 (0.80 g, 1.80 mmol) and IV-7 (0.87 g, 2.16 mmol) were used as raw materials, the operation process was the same as that of compound V-1 to obtain 1.14 g of light-yellow solid, m.p. at 120-122°C. $^1$H-NMR (300 MHz, $CDCl_3$) $\delta$ (ppm): 8.35 (s, 1H, ArH), 7.99 (s, 1H, NH), 7.96-7.81 (m, 3H, ArH), 7.48 (d, $J$ = 7.83 Hz, 2H, ArH), 7.13 (d, $J$ = 8.43 Hz, 1H, ArH), 6.12 (s, 2H, $OCH_2O$), 5.87 (s, 2H, $NH_2$), 4.92-4.77 (m, 1H, $CHC\underline{H}_2NBoc$), 4.46-4.23 (m, 1H, $C\underline{H}CH_2NBoc$), 4.20-4.02 (m, 1H, $CHC\underline{H}_2NBoc$), 3.52-3.28 (m, 1H, $NC\underline{H}_2CH_2$), 2.93-2.74 (m, 1H, $NC\underline{H}_2CH_2$), 2.37-2.18 (m, 2H, $NCH_2CHC\underline{H}_2$), 1.98-1.80 (m, 1H, $NCH_2C\underline{H}_2$), 1.79-1.59 (m, 1H, $NCH_2C\underline{H}_2$), 1.45 (s, 9H, NBoc).

Synthesis of (R)-N-(7-(4-amino-1-(piperidin-3)-1H-pyrazolo[3,4-d]pyrimidin-3-)benzo[d][1,3]dioxo heterocyclopenten-4)-4-chlorobenzamide (VI-7)

Compound V-7 (1.00 g, 1.69 mmol) was used as the raw material, the operation process was the same as that of compound VI-1 to obtain 777 mg of light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-chlorobenzamide (I-7)

Compound VI-7 (500 mg, 1.02 mmol) and acryloyl chloride (97 mg, 1.07 mmol) were used as raw materials, the procedure was the same as that of compound I-1 to obtain 240 mg of white solid with a yield of 43.2%, m.p. at 159.5-161°C. $^1$H-NMR (400 MHz, $CDCl_3$) $\delta$ (ppm): 8.32 (d, $J$ = 11.08 Hz, 1H, ArH), 8.13 (s, 1H, CONH), 7.98-7.90 (m, 1H, ArH), 7.87 (d, $J$= 6.92 Hz, 2H, ArH), 7.52 (d, $J$ = 7.12 Hz, 2H, ArH), 7.10 (d, $J$ = 6.04 Hz, 1H, ArH), 6.67-6.53 (m, 1H, $CH=CH_2$), 6.35-6.23 (m, 1H, $CH=CH_2$), 6.11 (br, 3H, $OCH_2O$, NH), 5.76-5.61 (m, 1H, $CH=CH_2$), 4.95-4.82 (m, 1.5H, $NC\underline{H}_2C\underline{H}$), 4.67-4.56 (m, 0.5H, $NCH_2C\underline{H}$), 4.28-4.15 (m, 0.5H, $NC\underline{H}_2CH$), 4.08-3.97 (m, 0.5H, $NC\underline{H}_2CH$), 3.85-3.72 (m, 0.5H, $NC\underline{H}_2CH_2$), 3.42-3.29 (m, 0.5H, $NC\underline{H}_2CH_2$), 3.25-3.13 (m, 0.5H, $NC\underline{H}_2CH_2$), 2.93-2.79 (m, 0.5H, $NC\underline{H}_2CH_2$), 2.65 (s, 1H, $NH_2$), 2.47-2.32 (m, 1H, $NCH_2CHC\underline{H}_2$), 2.31-2.18 (m, 1H, $NCH_2CHC\underline{H}_2$), 2.04-1.92 (m, 1H, $NCH_2C\underline{H}_2$), 1.80-1.64 (m, 1H, $NCH_2C\underline{H}_2$); HRMS (ESI): m/z $[M+H]^+$. Calcd for $C_{27}H_{25}ClN_7O_4$: 546.1657; found: 546.1655.

Example 8

Synthesis of (*R*)-*N*$^1$-(7-(1-(1-acryloylpiperidin-3-yl)-4-amino-1*H*-pyrazolo[3,4-*d*]pyrimidin-3-)benzo[*d*][1,3]dioxo heterocyclopenten-4)-*N*$^3$-(2,2,2-trifluoroethyl)isophthalamide (I-8)

Synthesis of 3-((7-(4,4,5,5-tetramethyl-1,3,2-dioxaborolan-2-yl)benzo[d][1,3]dioxo heterocyclopenten-4)-carbamoyl) methyl benzoate (IV-8)

Compound XI (1.00 g, 3.80 mmol) and monomethyl isophthalate (1.03 g, 5.72 mmol) were used as raw materials, the operation process was the same as that of compound IV-2 to obtain 1.45 g of white solid with a yield of 89.7%, and it was directly used in next step without further purification. $^1$H-NMR (300 MHz, $CDCl_3$) $\delta$ (ppm): 8.51 (s, 1H, NH), 8.22 (d, $J$ = 7.77 Hz, 1H, ArH), 8.12 (d, $J$ = 7.89 Hz, 1H, ArH), 7.91 (s, 1H, ArH), 7.84 (d, $J$ = 8.43 Hz, 1H, ArH), 7.59 (t, $J$ = 7.71 Hz, 1H, ArH), 7.29 (d, $J$ = 8.49 Hz, 1H, ArH), 6.08 (s, 2H, $OCH_2O$), 3.96 (s, 3H, $CH_3$), 1.37 (s, 12H, $4CH_3$).

Synthesis of (*R*)-3-(4-amino-3-(7-(3-methoxycarbonylbenzamide) benzo[*d*][1,3]dioxo heterocyclopenten-4)-1*H*-pyrazolo[3,4-*d*]pyrimidin-1-)piperidine-1-tert-butyl formate (V-8)

Compound III-1 (0.80 g, 1.80 mmol) and IV-8 (1.15 g, 2.70 mmol) were used as raw materials, the operation process was the same as that of compound V-1 to obtain 1.52 g of gray solid, which was directly used in the next step without purification.

Synthesis of (*R*)-3-((7-(4-amino-1-(1-tert-butoxycarbonyl)piperidin-3)-1*H*-pyrazolo[3,4-*d*]pyrimidine-3-benzo[*d*][ 1,3]dioxo heterocyclopenten-4-)carbamoyl)benzoic acid (V-8-1)

Compound V-8 (1.40 g, 2.27 mmol), lithium hydroxide monohydrate (0.14 g, 3.41 mmol) and methanol/water (12 mL/4 mL) were mixed, and the mixture was stirred overnight at a room temperature. Methanol was distilled off under reduced pressure, water and ethyl acetate were added, the organic layer was removed, the pH of the aqueous layer was adjusted to 3-4 with 2 mol/L hydrochloric acid, suction filtration was conducted, and drying was conducted to obtain 1.13 g of white solid with a yield of 82.6%, m.p. at 168- 170°C.

Synthesis of (*R*)-3-(4-amino-3-(7-(3-((2,2,2-trifluoroethyl) carbamoyl)benzamide)benzo[*d*][1,3] dioxo heterocyclopenten-4)-1*H*-pyrazolo[3,4-*d*]pyrimidin-1)piperidine-1-tert-butyl formate (V-8-2)

V-8-1 (900 mg, 1.50 mmol), HOBt (243 mg, 1.80 mmol) and EDCI (344 mg, 1.80 mmol) were dissolved in 5 mL DMF, triethylamine (227 mg, 2.24 mmol) and trifluoroethylamine (156 mg, 1.57 mmol) were added; the mixture was stirred overnight at a room temperature. 25 mL of water was added, extraction was conducted with ethyl acetate, the organic layer was washed with water (10 mL × 3) and then washed with saturated sodium chloride (10 mL × 3), and drying was conducted over anhydrous sodium sulfate. Suction filtration was conducted, the filtrate was evaporated to dryness under reduced pressure, and the residue was purified by column chromatography (eluent: Dichloromethane: Methanol=100-40:1) to obtain 667 mg of white solid with a yield of 65.3%, m.p. at 143-145°C. $^1$H-NMR (300 MHz, $CDCl_3$) $\delta$ (ppm): 8.40 (s, 1H, ArH), 8.32 (s, 1H, NH), 8.27 (s, 1H, ArH), 8.04 (q, *J* = 7.92 Hz, 2H, ArH), 7.76 (d, *J* = 7.92 Hz, 1H, ArH), 7.56 (t, *J* = 7.71 Hz, 1H, ArH), 7.22 (s, 1H, NH), 7.09 (d, *J* = 8.79 Hz, 1H, ArH), 6.06 (s, 2H, $OCH_2O$), 5.87 (s, 2H, $NH_2$), 4.88-4.74 (m, 1H, $CHC\underline{H}_2NBoc$), 4.37-4.25 (m, 1H, $C\underline{H}CH_2NBoc$), 4.21-4.05 (m, 3H, $CHC\underline{H}_2NBoc$, $C\underline{H}_2CF_3$), 3.49-3.30 (m, 1H, $NC\underline{H}_2CH_2$), 2.90-2.74 (m, 1H, $NC\underline{H}_2CH_2$), 2.29-2.14 (m, 2H, $NCH_2CHC\underline{H}_2$), 1.94-1.81 (m, 1H, $NCH_2C\underline{H}_2$), 1.76-1.60 (m, 1H, $NCH_2C\underline{H}_2$), 1.43 (s, 9H, 3CH3).

Synthesis of (*R*)-*N*[1]-(7-(4-amino-1-(piperidin-3)-1*H*-pyrazolo[3,4-*d*]pyrimidin-3-)benzo[*d*][1,3]dioxo heterocyclopenten-4)-*N*[3]-(2,2,2-trifluoroethyl)isophthalamide (VI-8)

Compound V-8-2 (550 mg, 0.81 mmol) was used as the raw material, the operation process was the same as that of compound VI-1 to obtain 430 mg of a light-yellow solid with a yield of 91.62%, which was directly used in the next step without further purification.

Synthesis of (*R*)-*N*1-(7-(1-(1-acryloylpiperidin-3)-4-amino-1*H*-pyrazolo[3,4-*d*]pyrimidin-3-)benzo[*d*][1,3]dioxo heterocyclopenten-4)-*N*[3]-(2,2,2-trifluoroethyl)isophthalamide (I-8)

Compound VI-8 (400 mg, 0.69 mmol) and acryloyl chloride (65 mg, 0.72 mmol) were used as raw materials, the procedure was the same as that of compound I-1 to obtain 231 mg of white solid with a yield of 52.8%, m.p. at 174-176°C. $^1$H-NMR (300 MHz, DMSO-$d_6$) $\delta$ (ppm): 10.43 (s, 1H, NH), 9.30 (s, 1H, NH), 8.50 (s, 1H, ArH), 8.33-8.00 (m, 2H, ArH), 7.66 (s, 1H, ArH), 7.35-6.97 (m, 2H, ArH), 6.95-6.55 (m, 2H, ArH, $CH=CH_2$), 6.37-5.92 (m, 3H, $CH=CH_2$, $OCH_2O$), 5.83-5.49 (m, 1H, $CH=CH_2$), 4.86-4.46 (m, 1.5H, $NC\underline{H}_2C\underline{H}$), 4.37-3.91 (m, 3H, $NC\underline{H}_2C\underline{H}$, $NHC\underline{H}_2CF_3$), 3.79-3.58 (m, 0.5H, $NC\underline{H}_2CH$), 3.07-2.84 (m, 1H, $NC\underline{H}_2CH_2$), 2.36-2.03 (m, 2H, $NC\underline{H}_2CH_2$, $NCH_2CHC\underline{H}_2$), 2.02-1.80 (m, 1H, $NCH_2CHC\underline{H}_2$), 1.72-1.46 (m, 1H, $NCH_2C\underline{H}_2$), 1.36-1.10 (m, 1H, $NCH_2C\underline{H}_2$); HRMS (ESI): m/z $[M+H]^+$. Calcd for $C_{30}H_{28}F_3N_8O_5$: 637.2135; found: 637.2134.

Example 9

Synthesis of (*R*)-*N*-(7-(1-(1-acryloylpiperidin-3)-4-amino-1*H*-pyrazolo[3,4-*d*]pyrimidin-3)benzo[*d*][1,3]dioxo heterocyclopenten-4)-4-trifluoromethylbenzamide (I-9)

Synthesis of *N*-(7-(4,4,5,5-tetramethyl-1,3,2-dioxaborolan-2-)benzo[d][1,3]dioxo heterocyclopenten-4)-4-trifluoromethylbenzamide (IV-9)

Compound XI (0.50 g, 1.90 mmol) and 4-trifluoromethylbenzoyl chloride (0.60 g, 2.85 mmol) were used as raw materials, the operation process was the same as that of compound IV-1 to obtain 654 mg of white solid with a yield of 79.1%, m.p. at 240-242°C. $^1$H-NMR (300 MHz, $CDCl_3$) $\delta$ (ppm): 7.99 (d, *J* = 8.16 Hz, 2H, ArH), 7.88-7.81 (m, 2H, ArH, NH), 7.76 (d, *J* = 8.19 Hz, 2H, ArH), 7.29 (d, *J* = 8.52 Hz, 1H, ArH), 6.08 (s, 2H, $OCH_2O$), 1.37 (s, 12H, $4CH_3$).

Synthesis of (*R*)-3-(4-amino-3-(7-(4-trifluoromethylbenzamido) benzo[*d*][1,3]dioxo heterocyclopenten-4)-1*H*-pyrazolo [3,4-*d*]pyrimidin-1-)piperidine-1-tert-butyl formate (V-9)

Compound III-1 (0.50 g, 1.13 mmol) and IV-9 (0.59 g, 1.35 mmol) were used as raw materials, the operation process was the same as that of compound V-1 to obtain 0.62 g of a light-yellow solid with a yield of 88.1%, m.p. at 140-142°C. $^1$H-NMR (300 MHz, $CDCl_3$) $\delta$ (ppm): 8.38 (s, 1H, ArH), 8.05 (d, *J* = 8.13 Hz, 2H, ArH), 8.01-7.95 (m, 2H, ArH, NH), 7.81 (d, *J* = 8.28 Hz, 2H, ArH), 7.18 (d, *J* = 8.73 Hz, 1H, ArH), 6.16 (s, 2H, $OCH_2O$), 5.74 (s, 2H, $NH_2$), 4.934.81 (m, 1H, $CHC\underline{H}_2NBoc$), 4.42-4.27 (m, 1H, $C\underline{H}CH_2NBoc$), 4.21-4.10 (m, 1H, $CHC\underline{H}_2NBoc$), 3.54-3.39 (m, 1H, $NC\underline{H}_2CH_2$), 2.95-2.79 (m, 1H, $NC\underline{H}_2CH_2$), 2.35-2.19 (m, 2H, $NCH_2CHC\underline{H}_2$), 1.96-1.87 (m, 1H, $NCH_2C\underline{H}_2$), 1.78-1.68 (m, 1H, $NCH_2C\underline{H}_2$), 1.47 (s, 9H, NBoc).

Synthesis of (R)-N-(7-(4-amino-1-(piperidin-3)-1*H*-pyrazolo[3,4-*d*]pyrimidin-3-)benzo[*d*][1,3]dioxo heterocyclopenten-4)-4-trifluoromethylbenzamide (VI-9)

Compound V-9 (550 mg, 0.88 mmol) was used as the raw material, the operation process was the same as that of compound VI-1 to obtain 420 mg of light-yellow solid with a yield of 90.91%.

Synthesis of (*R*)-*N*-(7-(1-(1-acryloylpiperidin-3)-4-amino-1*H*-pyrazolo[3,4-*d*]pyrimidin-3)benzo[*d*][1,3]dioxo heterocyclopenten-4)-4-trifluoromethylbenzamide (I-9)

Compound VI-9 (500 mg, 0.95 mmol) and acryloyl chloride (103 mg, 1.14 mmol) were used as raw materials, the procedure was the same as that of compound I-1 to obtain 289 mg of white solid with a yield of 52.4%, m.p. at 148.5-150°C. $^1$H-NMR (300 MHz, $CDCl_3$) $\delta$ (ppm): 8.43-8.23 (m, 2H, ArH, NH), 8.05 (d, *J* = 7.62 Hz, 2H, ArH), 7.92-7.81 (m, 1H, ArH), 7.76 (d, *J* = 7.44 Hz, 2H, ArH), 7.20-7.04 (m, 1H, ArH), 6.70-6.48 (m, 1H, $CH=CH_2$), 6.35-6.19 (m, 1H, $CH=CH_2$), 6.11 (s, 2H, $OCH_2O$), 5.96 (s, 1H, $NH_2$), 5.75-5.59 (m, 1H, $CH=CH_2$), 4.98-4.75 (m, 1.5H, $NC\underline{H}_2C\underline{H}$), 4.67-4.48 (m, 0.5H, $NCH_2C\underline{H}$), 4.30-4.13 (m, 0.5H, $NC\underline{H}_2CH$), 4.09-3.92 (m, 0.5H, $NC\underline{H}_2CH$), 3.88-3.67 (m, 0.5H, $NC\underline{H}_2CH_2$), 3.45-3.28 (m, 0.5H, $NC\underline{H}_2CH_2$), 3.26-3.09 (m, 0.5H, $NC\underline{H}_2CH_2$), 2.96-2.80 (m, 0.5H, $NC\underline{H}_2CH_2$), 2.50-2.31 (m, 1H, $NCH_2CHC\underline{H}_2$), 2.29-2.18 (m, 2H, $NCH_2CHC\underline{H}_2$, $NH_2$), 2.07-1.89 (m, 1H, $NCH_2C\underline{H}_2$), 1.81-1.60 (m, 1H, $NCH_2C\underline{H}_2$); $^{13}$C-NMR (75 MHz, DMSO-$d_6$) $\delta$ (ppm): 164.55, 163.98, 158.03, 155.89, 155.66, 153.71, 145.90, 140.95, 138.29, 137.78, 128.73, 128.32, 127.29, 125.69, 125.40, 122.11, 120.95, 118.82, 111.82, 101.72, 98.64, 52.91(0.5C), 52.19(0.5C), 49.19(0.5C), 48.70(0.5C), 45.65(0.5C), 45.10(0.5C), 30.38(0.5C), 29.57(0.5C), 26.78(0.5C), 24.81(0.5C); HRMS (ESI): m/z $[M+H]^+$. Calcd for $C_{28}H_{25}F_3N_7O_4$: 580.1920; found: 580.1918.

Example 10

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-methoxybenzamide (I-10)

Synthesis of *N*-(7-(4,4,5,5-tetramethyl-1,3,2-dioxaborolan-2-yl)benzo[d][1,3]dioxo heterocyclopenten-4)-4-methoxybenzamide (IV-10)

Compound XI (1.50 g, 5.70 mmol) and 4-methoxybenzoyl chloride (1.46 g, 8.55 mmol) were used as raw materials, the operation process was the same as that of compound IV-1 to obtain 1.92 g of white solid with a yield of 84.1%, m.p. at 176-178°C. $^1$H-NMR (300 MHz, $CDCl_3$) $\delta$ (ppm): 7.93-7.81 (m, 3H, ArH), 7.78 (br, 1H, NH), 7.28 (d, J = 8.31 Hz, 1H, ArH), 6.97 (d, J = 8.58 Hz, 2H, ArH), 6.06 ( s, 2H, $OCH_2O$), 3.87 (s, 3H, $OCH_3$), 1.36 (s, 12H, $4CH_3$).

Synthesis of (*R*)-3-(4-amino-3-(7-(4-methoxybenzamido)benzo [*d*][1,3]dioxo heterocyclopenten-4)-1*H*-pyrazolo[3,4-*d*] pyrimidin-1-yl)piperidine-1-tert-butyl formate (V-10)

Compound III-1 (1.20 g, 2.70 mmol) and IV-10 (1.29 g, 3.24 mmol) were used as raw materials, the operation process was the same as that of compound V-1 to obtain 1.49 g of a light-yellow solid with a yield of 93.7%, m.p. at 104-106 °C. $^1$H-NMR (300 MHz, $CDCl_3$) $\delta$ (ppm): 8.38 (s, 1H, ArH), 8.01 (d, *J* = 8.70 Hz, 1H, ArH), 7.90 (d, *J* = 8.85 Hz, 2H, ArH), 7.87 (br, 1H, NH), 7.15 (d, *J* = 8.70 Hz, 1H, ArH), 7.02 (d, *J* = 8.82 Hz, 2H, ArH), 6.14 (s, 2H, $OCH_2O$), 5.75 (s, 2H, $NH_2$), 4.94-4.80 (m, 1H, $CHC\underline{H}_2NBoc$), 4.44-4.25 (m, 1H, $C\underline{H}CH_2NBoc$), 4.23-4.06 (m, 1H, $CHC\underline{H}_2NBoc$), 3.91 (s, 3H, $OCH_3$), 3.56-3.36 (m, 1H, $NC\underline{H}_2CH_2$), 2.93-2.78 (m, 1H, $NC\underline{H}_2CH_2$), 2.34-2.17 (m, 2H, $NCH_2CHC\underline{H}_2$), 1.98-1.83 (m, 2H, $NCH_2C\underline{H}_2$), 1.47 (s, 9H, NBoc).

Synthesis of (*R*)-*N*-(7-(4-amino-1-(piperidin-3)-1*H*-pyrazolo[3,4-*d*]pyrimidin-3-)benzo[d][1,3]dioxo heterocyclopenten-4)-4-methoxybenzamide (VI-10)

Compound V-10 (1.27 g, 2.17 mmol) was used as the raw material, the operation process was the same as that of compound VI-1 to obtain 1.04 g of a light-yellow solid with a yield of 98.01%.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-methoxybenzamide (I-10)

Compound VI-10 (100 mg, 0.21 mmol) and acryloyl chloride (19 mg, 0.22 mmol) were used as raw materials, the procedure was the same as compound I-1 to obtain 89 mg of white solid with a yield of 80.1%, m.p. at 216-218°C. $^1$H-NMR (300 MHz, $CDCl_3$) $\delta$ (ppm): 8.35 (s, 1H, ArH), 8.02-7.93 (m, 2H, ArH, NH), 7.90 (d, *J* = 7.68 Hz, 2H, ArH), 7.12 (d, *J* = 8.16

Hz, 1H, ArH), 7.00 (d, *J* = 7.74 Hz, 2H, ArH), 6.71-6.51 (m, 1H, $CH=CH_2$), 6.38-6.22 (m, 1H, $CH=CH_2$), 6.12 (s, 2H, $OCH_2O$), 5.90 (s, 1H, $NH_2$), 5.77-5.61 (m, 1H, $CH=CH_2$), 4.96-4.80 (m, 1.5H, $NC\underline{H}_2C\underline{H}$), 4.71-4.53 (m, 0.5H, $NCH_2C\underline{H}$), 4.29-4.16 (m, 0.5H, $NC\underline{H}_2CH$), 4.08-3.98 (m, 0.5H, $NC\underline{H}_2CH$), 3.90 (s, 3H, $OCH_3$), 3.82-3.69 (m, 0.5H, $NC\underline{H}_2CH_2$), 3.51-3.32 (m, 0.5H, $NC\underline{H}_2CH_2$), 3.29-3.10 (m, 0.5H, $NC\underline{H}_2CH_2$), 2.97-2.75 (m, 0.5H, $NC\underline{H}_2CH_2$), 2.51-2.35 (m, 1H, $NCH_2CHC\underline{H}_2$), 2.35-2.14 (m, 2H, $NCH_2CHC\underline{H}_2$, $NH_2$), 2.09-1.89 (m, 1H, $NCH_2C\underline{H}_2$), 1.83-1.64 (m, 1H, $NCH_2C\underline{H}_2$); HRMS (ESI): m/z $[M+H]^+$. Calcd for $C_{28}H_{28}N_7O_5$: 542.2152; found: 542.2156.

Example 11

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-tert-butylbenzamide (I-11)

Synthesis of 4-tert-butyl-*N*-(7-(4,4,5,5-tetramethyl-1,3,2-dioxaborolan-2-)benzo[d][1,3]dioxo heterocyclopenten-4-) benzamide (IV-11)

Compound XI (1.00 g, 3.80 mmol) and 4-tert-butylbenzoic acid (1.02 g, 5.70 mmol) were used as raw materials, the operation process was the same as that of compound IV-2 to obtain 982 mg of white solid with a yield of 61.0%, m.p. at 170-172°C. $^1$H-NMR (300 MHz, $CDCl_3$) $\delta$ (ppm): 7.91 (d, *J* = 8.52 Hz, 1H, ArH), 7.86 (s, 1H, NHCO), 7.82 (d, *J* = 8.46 Hz, 2H, ArH), 7.51 (d, *J* = 8.46 Hz, 2H, ArH), 7.28 (d, *J* = 8.52 Hz, 1H, ArH), 6.07 (s, 2H, $OCH_2O$), 1.36 (s, 12H, $4CH_3$), 1.35 (s, 9H, $C(CH_3)_3$).

Synthesis of (*R*)-3-(4-amino-3-(7-(4-tert-butylbenzamido)benzo [*d*][1,3]dioxo heterocyclopenten-4)-1*H*-pyrazolo[3,4-*d*] pyrimidin-1-)piperidine-1-tert-butyl formate (V-11)

Compound III-1 (0.75 g, 1.69 mmol) and IV-11 (0.86 g, 2.03 mmol) were used as raw materials, the operation process was the same as that of compound V-1 to obtain 969 mg of light-yellow solid with a yield of 93.5%, m.p. at 140-142°C. $^1$H-NMR (300 MHz, $CDCl_3$) $\delta$ (ppm): 8.39 (s, 1H, ArH), 8.07-7.99 (m, 2H, ArH, NH), 7.89 (d, *J* = 8.34 Hz, 2H, ArH), 7.56 (d, *J* = 8.31 Hz, 2H, ArH), 7.16 (d, *J* = 8.67 Hz, 1H, ArH), 6.16 (s, 2H, $OCH_2O$), 5.93 (s, 1H, $NH_2$), 4.96-4.82 (m, 1H, $CHC\underline{H}_2NBoc$), 4.49-4.10 (m, 2H, $C\underline{H}C\underline{H}_2NBoc$), 3.62-3.32 (m, 1H, $NC\underline{H}_2CH_2$), 2.97-2.79 (m, 1H, $NC\underline{H}_2CH_2$), 2.27-2.14 (m, 3H, $NCH_2CHC\underline{H}_2$, $NH_2$), 1.99-1.87 (m, 1H, $NCH_2C\underline{H}_2$), 1.80-1.68 (m, 1H, $NCH_2C\underline{H}_2$), 1.48 (s, 9H, $3CH_3$), 1.40 (s, 9H, $3CH_3$).

Synthesis of (*R*)-*N*-(7-(4-amino-1-(piperidin-3-yl)-1*H*-pyrazolo[3,4-*d*]pyrimidin-3-)benzo[*d*][1,3]dioxo heterocyclopenten-4)-4-tert-butylbenzamide (VI-11)

Compound V-11 (900 mg, 1.47 mmol) was used as the raw material, the operation process was the same as that of compound VI-1 to obtain 697 mg of a light-yellow solid with a yield of 92.5%, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-tert-butylbenzamide (I-11)

Compound VI-11 (600 mg, 1.17 mmol) and acryloyl chloride (111 mg, 1.23 mmol) were used as raw materials, the procedure was the same as compound I-1 to obtain 242 mg of white solid with a yield of 36.5%, m.p. at 136-138 °C. $^1$H-NMR (400 MHz, $CDCl_3$) $\delta$ (ppm): 8.32 (d, *J*= 11.08 Hz, 1H, ArH), 8.12 (s, 1H, NH), 7.98-7.89 (m, 1H, ArH), 7.86 (d, *J* = 6.92 Hz, 2H, ArH), 7.52 (d, *J* = 7.12 Hz, 2H, ArH), 7.10 (d, *J* = 6.04 Hz, 1H, ArH), 6.68-6.51 (m, 1H, $CH=CH_2$), 6.36-6.21 (m, 1H, $CH=CH_2$), 6.17-6.01 (m, 3H, $NH_2$, $OCH_2O$), 5.76-5.60 (m, 1H, $CH=CH_2$), 4.95-4.78 (m, 1.5H, $NC\underline{H}_2C\underline{H}$), 4.67-4.50 (m, 0.5H, $NCH_2C\underline{H}$), 4.29-4.14 (m, 0.5H, $NC\underline{H}_2CH$), 4.09-3.95 (m, 0.5H, $NC\underline{H}_2CH$), 3.84-3.69 (m, 0.5H, $NC\underline{H}_2CH_2$), 3.41-3.27 (m, 0.5H, $NC\underline{H}_2CH_2$), 3.24-3.10 (m, 0.5H, $NC\underline{H}_2CH_2$), 2.92-2.78 (m, 0.5H, $NC\underline{H}_2CH_2$), 2.65 (s, 1H, $NH_2$), 2.48-2.31 (m, 1H, $NCH_2CHC\underline{H}_2$), 2.30-2.16 (m, 1H, $NCH_2CHC\underline{H}_2$), 2.04-1.89 (m, 1H, $NCH_2C\underline{H}_2$), 1.79-1.61 (m, 1H, $NCH_2C\underline{H}_2$), 1.35 (s, 9H, $3CH_3$); HRMS (ESI): m/z $[M+H]^+$. Calcd for $C_{31}H_{34}N_7O_4$: 568.2672; found: 568.2674.

Example 12

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4)-3,4-dimethoxybenzamide (I-12)

Synthesis of 3,4-dimethoxy-N-(7-(4,4,5,5-tetramethyl-1,3,2-dioxaborolan-2-)benzo[d][1,3]dioxo heterocyclopenten-4-)benzamide (IV-12)

Compound XI (1.00 g, 3.80 mmol) and 3,4-dimethoxybenzoyl chloride (1.14 g, 5.70 mmol) were used as raw materials, the operation process was the same as that of compound IV-1 to obtain 926 mg of white solid with a yield of 57.2%, m.p. at 166-168°C. $^1$H-NMR(300 MHz, $CDCl_3$) $\delta$(ppm): 7.90-7.82 (m, 2H, ArH, NHCO), 7.51(s, 1H, ArH), 7.41(d, *J*=8.31 Hz, 1H, ArH), 7.28(d, *J*=8.04 Hz, 1H, ArH), 6.91(d, *J*=8.34 Hz, 1H, ArH), 6.07(s, 2H, $OCH_2O$), 3.95(s, 3H, $OCH_3$), 3.95(s, 3H, $OCH_3$), 1.36(s, 12H, $4CH_3$).

Synthesis of (*R*)-3-(4-amino-3-(7-(3,4-dimethoxybenzamido) benzo[*d*][1,3]dioxo heterocyclopenten-4)-1*H*-pyrazolo[3,4-*d*]pyrimidin-1-)piperidine-1-tert-butyl formate (V-12)

Compound III-1 (0.70 g, 1.58 mmol) and IV-12 (0.81 g, 1.89 mmol) were used as raw materials, the operation process was the same as that of compound V-1 to obtain 731 mg of light-yellow solid with a yield of 75.1%, m.p. at 120-122°C. $^1$H-NMR (300 MHz, $CDCl_3$) $\delta$ (ppm): 8.37(s, 1H, ArH), 8.00(d, *J*=8.94 Hz, 1H, ArH), 7.88(s, 1H, ArH), 7.54(s, 1H, NH), 7.44(d, *J* = 8.37 Hz, 1H, ArH), 7.15(d, *J* = 8.91 Hz, 1H, ArH), 6.94 (d, *J* = 8.01 Hz, 1H, ArH), 6.13(s, 2H, $OCH_2O$), 5.78(s, 2H, $NH_2$), 4.92-4.78 (m, 1H, $CHCH_2NBoc$), 4.38-4.24(m, 1H, $CHCH_2NBoc$), 4.20-4.08 (m, 1H, $CHCH_2NBoc$), 3.98 (s, 3H, $OCH_3$), 3.97(s, 3H, $OCH_3$), 3.52-3.37 (m, 1H, $NCH_2CH_2$), 2.91-2.77 (m, 1H, $NCH_2CH_2$), 2.33-2.24(m, 1H, $NCH_2CHCH_2$), 2.23-2.14 (m, 1H, $NCH_2CHCH_2$), 1.96-1.84 (m, 1H, $NCH_2CH_2$), 1.77-1.65(m, 1H, $NCH_2CH_2$), 1.45 (s, 9H, NBoc).

Synthesis of (*R*)-*N*-(7-(4-amino-1-(piperidin-3)-1*H*-pyrazolo[3,4-*d*]pyrimidin-3-yl)benzo[*d*] [1,3]dioxo heterocyclopenten-4)-3,4-dimethoxybenzamide (VI-12)

Compound V-12 (700 mg, 1.13 mmol) was used as the raw material, the operation process was the same as that of compound VI-1 to obtain 587 mg of light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4)-3,4-dimethoxybenzamide (I-12)

Compound VI-12 (537 mg, 1.04 mmol) and acryloyl chloride (99 mg, 1.09 mmol) were used as raw materials, the procedure was the same as that of compound I-1 to obtain 309 mg of white solid with a yield of 52.1%, m.p. at 164-165°C. $^1$H-NMR (400 MHz, $CDCl_3$) $\delta$ (ppm): 8.41-8.25(m, 1H, ArH), 8.05(s, 1H, NH), 7.94-7.87(m, 1H, ArH), 7.56-7.51(m, 1H, ArH), 7.49-7.44(m, 1H, ArH), 7.11(d, *J* = 8.64 Hz, 1H, ArH), 6.93(d, *J* =8.40 Hz, 1H, ArH), 6.68-6.52(m, 1H, $CH=CH_2$), 6.36-6.23(m, 1H, $CH=CH_2$), 6.12(s, 2H, $OCH_2O$), 5.95(br, 1H, $NH_2$), 5.78-5.60(m, 1H, $CH=CH_2$), 4.94-4.83(m, 1.5H, $NCH_2CH$), 4.66-4.55(m, 0.5H, $NCH_2CH$), 4.26-4.17(m, 0.5H, $NCH_2CH$), 4.09-4.00(m, 0.5H, $NCH_2CH$), 3.96(s, 3H, $OCH_3$), 3.96(s, 3H, $OCH_3$), 3.83-3.73(m, 0.5H, $NCH_2CH_2$), 3.41-3.31(m, 0.5H, $NCH_2CH_2$), 3.24-3.13(m, 0.5H, $NCH_2CH_2$), 2.94-2.81(m, 0.5H, $NCH_2CH_2$), 2.45-2.34(m, 1H, $NCH_2CHCH_2$), 2.29-2.23(m, 2H, $NCH_2CHCH_2$, $NH_2$), 2.02-1.94(m, 1H, $NCH_2CH_2$), 1.79-1.64 (m, 1H, $NCH_2CH_2$); HRMS (ESI): m/z $[M+H]^+$. Calcd for $C_{29}H_{30}N_7O_6$: 572.2258; found: 572.2252.

Example 13

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-(dimethylamino)benzamide (I-13)

Synthesis of 4-(dimethylamino)-*N*-(7-(4,4,5,5-tetramethyl-1,3,2-dioxaborolan-2-)benzo[*d*][1,3]dioxo heterocyclopenten-4-)benzamide (IV-13)

Compound XI (1.00 g, 3.80 mmol) and 4-dimethylamino benzoyl chloride (1.05 g, 5.70 mmol) were used as raw materials, the operation process was the same as that of compound IV-1 to obtain 950 mg of white solid with a yield of 60.9%. The solid was directly used in the next step without further purification.

Synthesis of (*R*)-3-(4-amino-3-(7-(4-(dimethylamino)benzamido) benzo[d][1,3]dioxo heterocyclopenten-4)-1*H*-pyrazolo[3,4-*d*]pyrimidin-1-yl)piperidine-1-tert-butyl formate (V-13)

Compound III-1 (0.60 g, 1.35 mmol) and IV-13 (0.67 g, 1.62 mmol) were used as raw materials, the operation process was the same as that of compound V-1 to obtain 678 mg of light-yellow solid with a yield of 83.5%, m.p. at

138-140°C. $^1$H-NMR (300 MHz, $CDCl_3$) δ (ppm): 8.36 (s, 1H, ArH), 8.04 (d, *J*= 9.03 Hz, 1H, ArH), 7.89-7.76(m, 3H, ArH, NH), 7.12 (d, *J* = 7.98 Hz, 1H, ArH), 6.73 (d, *J* = 8.61 Hz, 2H, ArH), 6.12 (s, 2H, $OCH_2O$), 5.75 (s, 2H, $NH_2$), 4.90-4.79 (m, 1H, $CHC\underline{H_2}NBoc$), 4.40-4.26 (m, 1H, $C\underline{H}CH_2NBoc$), 4.22-4.06 (m, 1H, $CHC\underline{H_2}NBoc$), 3.54-3.35 (m, 1H, $NC\underline{H_2}CH_2$), 3.07 (s, 6H, $N(C\underline{H_3})_2$), 2.90-2.74 (m, 1H, $NC\underline{H_2}CH_2$), 2.34-2.15 (m, 2H, $NCH_2CHC\underline{H_2}$), 1.99-1.82 (m, 2H, $NCH_2C\underline{H_2}$), 1.45 (s, 9H, NBoc).

Synthesis of (*R*)-*N*-(7-(4-amino-1-(piperidin-3)-1*H*-pyrazolo[3,4-*d*]pyrimidin-3-)benzo[d][1,3]dioxo heterocyclopenten-4)-4-(dimethylamino)benzamide (VI-13)

Compound V-13 (850 mg, 1.42 mmol) was used as the raw material, the operation process was the same as that of compound VI-1 to obtain 588 mg of a light-yellow solid with a yield of 83.0%, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-(dimethylamino)benzamide (I-13)

Compound VI-13 (540 mg, 1.08 mmol) and acryloyl chloride (103 mg, 1.13 mmol) were used as raw materials, the operation process was the same as that of the target compound I-1 to obtain 342 mg of white solid with a yield of 57.2%, m.p. at 160-162°C. $^1$H-NMR (300 MHz, $CDCl_3$) δ (ppm): 8.33 (s, 1H, ArH), 8.04-7.88 (m, 2H, ArH, NH), 7.81 (d, *J* = 6.66 Hz, 2H, ArH), 7.09 (d, *J* = 8.07 Hz, 1H, ArH), 6.71 (d, *J* = 6.96 Hz, 2H, ArH), 6.65-6.52 (m, 1H, $CH{=}CH_2$), 6.36-6.21 (m, 1H,$CH{=}CH_2$), 6.17-5.99 (m, 3H, $NH_2$, $OCH_2O$), 5.76-5.59 (m, 1H, $CH{=}CH_2$), 4.96-4.79 (m, 1.5H, $NC\underline{H_2}C\underline{H}$), 4.69-4.57 (m, 0.5H, $NCH_2C\underline{H}$), 4.28-4.15 (m, 0.5H, $NC\underline{H_2}CH$), 4.08-3.96 (m, 0.5H, $NC\underline{H_2}CH$), 3.85-3.70 (m, 0.5H, $NC\underline{H_2}CH_2$), 3.43-3.28 (m, 0.5H, $NC\underline{H_2}CH_2$), 3.24-3.14 (m, 0.5H, $NC\underline{H_2}CH_2$), 3.06 (s, 6H, $2CH_3$), 2.89-2.72 (m, 1.5H, $NC\underline{H_2}CH_2$, $NH_2$), 2.47-2.31 (m, 1H, $NCH_2CHC\underline{H_2}$), 2.30-2.16 (m, 1H, $NCH_2CHC\underline{H_2}$), 2.04-1.89 (m, 1H, $NCH_2C\underline{H_2}$), 1.80-1.61 (m, 1H, $NCH_2C\underline{H_2}$); HRMS (ESI): m/z $[M+H]^+$. Calcd for $C_{29}H_{31}N_8O_4$: 555.2468; found: 555.2466.

Example 14

Synthesis of *N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-(dimethylamino)benzamide (I-14)

Synthesis of tert-butyl 3-(4-amino-3-iodo-1*H*-pyrazolo[3,4-d]pyrimidin-1-)piperidine-1-tert-butyl formate (III-2)

Compound VII-1 (3.00 g, 11.49 mmol) and 1-tert-butoxycarbonyl-3-hydroxypiperidine (VIII-2) (4.63 g, 22.99 mmol) were used as raw materials, the operation process was the same as that of compound III-1 to obtain 3.91 g white solid with a yield of 76.5%, m.p. at 176-178°C. $^1$H-NMR (300 MHz, $CDCl_3$) δ (ppm): 8.31 (s, 1H, ArH), 6.17 (s, 2H, $NH_2$), 4.82-4.68 (m, 1H, $CHC\underline{H_2}N$), 4.22-4.05 (m, 1H, $C\underline{H}CH_2N$), 3.79-3.67 (m, 1H, $CHC\underline{H_2}N$), 3.22-3.02 (m, 1H, $CHCH_2CH_2C\underline{H_2}N$), 2.91-2.74 (m, 1H, $CHCH_2CH_2C\underline{H_2}N$), 2.19-2.08 (m, 2H, $CHC\underline{H_2}CH_2CH_2N$), 1.95-1.81 (m, 2H, $CHCH_2C\underline{H_2}CH_2N$), 1.45 (s, 9H, NBoc).

Synthesis of 3-(4-amino-3-(7-(4-(dimethylamino)benzamido) benzo[*d*][1,3]dioxo heterocyclopenten-4)-1*H* -tert-butyl-pyrazolo[3,4-*d*]pyrimidin-1-)piperidine-1-tert-butyl formate (V-14)

Compound III-2 (0.95 g, 2.14 mmol) and IV-13 (1.05 g, 2.57 mmol) were used as raw materials, the operation process was the same as that of compound V-1 to obtain 890 mg of light-yellow solid with a yield of 69.5%, m.p. at 140-142°C. $^1$H-NMR (300 MHz, $CDCl_3$) δ (ppm): 8.38 (s, 1H, ArH), 8.04 (d, *J* = 8.73 Hz, 1H, ArH), 7.89 (s, 1H, NHCO), 7.85 (d, *J* = 8.88 Hz, 2H, ArH), 7.15 (d, *J* = 8.70 Hz, 1H, ArH), 6.75 (d, *J* = 8.94 Hz, 2H, ArH), 6.15 (s, 2H, $OCH_2O$), 5.94 (s, 2H, $NH_2$), 4.94-4.81 (m, 1H, $NC\underline{H_2}CH$), 4.33-4.12 (m, 2H, $NC\underline{H_2}C\underline{H}$), 3.59-3.37 (m, 1H, $NC\underline{H_2}CH_2$), 3.10 (s, 6H, $N(CH_3)_2$), 2.93-2.81 (m, 1H, $NC\underline{H_2}CH_2$), 2.27-2.19 (m, 2H, $NCH_2CHC\underline{H_2}$), 1.98-1.87 (m, 1H, $NCH_2C\underline{H_2}$), 1.78-1.66 (m, 1H, $NCH_2C\underline{H_2}$), 1.48 (s, 9H, NBoc).

Synthesis of N-(7-(4-amino-1-(piperidin-3)-1*H*-pyrazolo[3,4-d]pyrimidin-3-)benzo[d][1,3]dioxo heterocyclopenten-4)-4-(dimethylamino)benzamide (VI-14)

Compound V-14 (850 mg, 1.42 mmol) was used as the raw material, the operation process was the same as that of compound VI-1 to obtain 436 mg of light-yellow solid with a yield of 61.6%, which was directly used in the next step without further purification.

Synthesis of *N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-(dimethylamino)benzamide (I-14)

Compound VI-14 (420 mg, 0.84 mmol) and acryloyl chloride (80 mg, 0.88 mmol) were used as raw materials, the operation process was the same as that of compound I-1 to obtain 213 mg of white solid with a yield of 45.7%, m.p. at 160-162°C. $^1$H-NMR (300 MHz, $CDCl_3$) $\delta$ (ppm): 8.33 (s, 1H, ArH), 8.03 (d, $J$ = 8.97 Hz, 1H, ArH), 7.86-7.76 (m, 3H, ArH, NH), 7.11 (d, $J$ = 8.82 Hz, 1H, ArH), 6.71 (d, $J$ = 7.32 Hz, 2H, ArH), 6.67-6.52 (m, 1H, $CH=CH_2$), 6.36-6.22 (m, 1H, $CH=CH_2$), 6.11 (s, 2H, $OCH_2O$), 5.97 (br, 1H, $NH_2$), 5.77-5.63 (m, 1H, $CH=CH_2$), 4.97-4.80 (m, 1.5H, $NC\underline{H}_2C\underline{H}$), 4.72-4.58 (m, 0.5H, $NCH_2C\underline{H}$), 4.30-4.17 (m, 0.5H, $NC\underline{H}_2CH$), 4.11-3.99 (m, 0.5H, $NC\underline{H}_2CH$), 3.85-3.65 (m, 0.5H, $NC\underline{H}_2CH_2$), 3.48-3.34 (m, 0.5H, $NC\underline{H}_2CH_2$), 3.26-3.13 (m, 0.5H, $NC\underline{H}_2CH_2$), 3.06 (s, 6H, $N(CH_3)_2$), 2.93-2.70 (m, 1.5H, $NC\underline{H}_2CH_2$, $NH_2$), 2.46-2.32 (m, 1H, $NCH_2CHC\underline{H}_2$), 2.31 -2.19 (m, 1H, $NCH_2CHC\underline{H}_2$), 2.06-1.92 (m, 1H, $NCH_2C\underline{H}_2$), 1.83-1.65 (m, 1H, $NCH_2C\underline{H}_2$); HRMS (ESI): m/z [M+H]$^+$. Calcd for $C_{29}H_{31}N_8O_4$: 555.2468; found: 555.2470.

Example 15

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-(diethylamino)benzamide (I-15)

Synthesis of 4-(diethylamino)-*N*-(7-(4,4,5,5-tetramethyl-1,3,2-dioxaborolane-2-)benzo[*d*][1,3]dioxo heterocyclopenten-4-)benzamide (IV-15)

Compound XI (1.00 g, 3.80 mmol) and 4-diethylaminobenzoic acid (1.10 g, 5.70 mmol) were used as raw materials, the operation process was the same as that of compound IV-2 to obtain 2.04 g of brown oil product, which was directly fed into next step without further purification.

Synthesis of (*R*)-3-(4-amino-3-(7-(4-(diethylamino)benzamido) benzo[d][1,3]dioxo heterocyclopenten-4)-1*H*-pyrazolo [3,4-*d*]pyrimidin-1-yl)piperidine-1-tert-butyl formate (V-15)

Compounds III-1 (0.80 g, 1.80 mmol) and IV-15 (0.95 g, 2.16 mmol) were used as raw materials, the operation process was the same as that of compound V-1 to obtain 657 mg of a light-yellow solid with a yield of 58.0%, m.p. at 116-118°C. $^1$H-NMR (300 MHz, $CDCl_3$) $\delta$ (ppm): 8.35 (s, 1H, ArH), 8.06 (d, $J$ = 8.25 Hz, 1H, ArH), 7.83-7.76 (m, 3H, ArH, NH), 7.12 (d, $J$ = 8.52 Hz, 1H, ArH), 6.69 (d, $J$ = 8.43 Hz, 2H, ArH), 6.12 (s, 2H, $OCH_2O$), 6.03 (s, 2H, $NH_2$), 4.89-4.78 (m, 1H, $CHC\underline{H}_2NBoc$), 4.37-4.26 (m, 1H, $C\underline{H}CH_2NBoc$), 4.17-4.10 (m, 1H, $CHC\underline{H}_2NBoc$), 3.47-3.39 (m, 5H, $NC\underline{H}_2CH_2$, $N(C\underline{H}_2CH_3)_2$), 2.87-2.80 (m, 1H, $NC\underline{H}_2CH_2$), 2.25-2.15 (m, 2H, $NCH_2CHC\underline{H}_2$), 1.91-1.84 (m, 1H, $NCH_2C\underline{H}_2$), 1.72-1.66 (m, 1H, $NCH_2C\underline{H}_2$), 1.45 (s, 9H, NBoc), 1.24-1.19 (m, 6H, $N(CH_2C\underline{H}_3)_2$).

Synthesis of (R)-N-(7-(4-amino-1-(piperidin-3)-1H-pyrazolo[3,4-d]pyrimidin-3-)benzo[d][1,3]dioxo heterocyclopenten-4)-4-(diethylamino)benzamide (VI-15)

Compound V-15 (657 mg, 1.05 mmol) was used as the raw material, the operation process was the same as that of compound VI-1 to obtain 328 mg of a light-yellow solid with a yield of 59.4%, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-(diethylamino)benzamide (I-15)

Compound VI-15 (300 mg, 0.57 mmol) and acryloyl chloride (54 mg, 0.60 mmol) were used as raw materials, the procedure was the same as that of compound I-1 to obtain 209 mg of a white solid with a yield of 63.2%, m.p. at 158-160°C. $^1$H-NMR (300 MHz, $CDCl_3$) $\delta$ (ppm): 8.34(s, 1H, ArH), 8.06-7.95(m, 1H, ArH), 7.92-7.85(m, 1H, ArH), 7.85-7.73(m, 2H, ArH, NH), 7.18-7.03(m, 1H, ArH), 6.77-6.51(m, 3H, ArH, $CH=CH_2$), 6.39-6.22(m, 1H, $CH=CH_2$), 6.11(s, 2H, $OCH_2O$), 6.00(s, 1H, $NH_2$), 5.79-5.61(m, 1H, $CH=CH_2$), 4.98-4.79(m, 1.5H, $NC\underline{H}_2C\underline{H}$), 4.72-4.55(m, 0.5H, $NCH_2C\underline{H}$), 4.31-4.16(m, 0.5H, $NC\underline{H}_2CH$), 4.12-3.96(m, 0.5H, $NC\underline{H}_2CH$), 3.87-3.71(m, 0.5H, $NC\underline{H}_2CH_2$), 3.53-3.30(m, 4H, $N(C\underline{H}_2CH_3)_2$), 3.28-3.12(m, 0.5H, $NC\underline{H}_2CH_2$), 2.94-2.77 (m, 0.5H, $NC\underline{H}_2CH_2$), 2.46-2.20 (m, 3.5H, $NC\underline{H}_2CH_2$, $NCH_2CHC\underline{H}_2$, $NH_2$), 2.08-1.91 (m, 1H, $NCH_2C\underline{H}_2$), 1.84-1.63(m, 1H, $NCH_2C\underline{H}_2$), 1.31-1.15(m, 6H, $N(CH_2C\underline{H}_3)_2$); HRMS (ESI): m/z [M+H]$^+$. Calcd for $C_{31}H_{35}N_8O_4$: 583.2781; found: 583.2777.

Example 16

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4-)-3-chlorobenzo[*b*]thiophene-2-formamide (I-16)

Synthesis of 3-chloro-*N*-(7-(4,4,5,5-tetramethyl-1,3,2-dioxaborolane-2)benzo[*d*][1,3]dioxo heterocyclopenten-4-)benzo[*b*]thiophene-2-carboxamide (IV-16)

Compound XI (800 g, 3.04 mmol) and 3-Chlorobenzo[*b*]thiophene-2-carboxylic acid (1.00 g, 4.70 mmol) were used as raw materials, the operation process was the same as that of compound IV-2 to obtain 1.02 g of a yellow solid with a yield of 73.4%, which was directly fed into next step without further purification.

Synthesis of (*R*)-3-(4-amino-3-(7-(3-Chlorobenzo[*b*]thiophene-2-formamide)benzo[*d*][1,3]dioxo heterocyclopenten-4)-1*H*-tert-butyl-pyrazolo[3,4-*d*]pyrimidin-1-)piperidine-1-tert-butyl formate (V-16)

Compounds III-1 (0.80 g, 1.80 mmol) and IV-16 (0.99 g, 2.16 mmol) were used as raw materials, the operation process was the same as that of compound V-1 to obtain 998 mg of a light-yellow solid with a yield of 85.3%, m.p. at 218-220°C. $^1$H-NMR (300 MHz, $CDCl_3$) $\delta$ (ppm): 9.13 (s, 1H, NH), 8.36 (s, 1H, ArH), 8.08 (d, *J* = 8.85 Hz, 1H, ArH), 7.97-7.85 (m, 2H, ArH), 7.58-7.50 (m, 2H, ArH), 7.16 (d, *J* = 8.73 Hz, 1H, ArH), 6.19 (s, 2H, $OCH_2O$), 5.94 (s, 2H, $NH_2$), 4.94-4.76 (m, 1H, $CHC\underline{H}_2NBoc$), 4.42-4.24 (m, 1H, $C\underline{H}CH_2NBoc$), 4.22-4.07 (m, 1H, $CHC\underline{H}_2NBoc$), 3.56-3.31 (m, 1H, $NC\underline{H}_2CH_2$), 2.93-2.74 (m, 1H, $NC\underline{H}_2CH_2$), 2.51-2.35 (m, 1H, $NCH_2CHC\underline{H}_2$), 2.24-2.12 (m, 1H, $NCH_2CHC\underline{H}_2$), 1.96-1.80 (m, 1H, $NCH_2C\underline{H}_2$), 1.78-1.64 (m, 1H, $NCH_2C\underline{H}_2$), 1.45 (s, 9H, $3CH_3$).

Synthesis of (R)-N-(7-(4-amino-1-(piperidin-3)-1H-pyrazolo[3,4-d]pyrimidin-3-)benzo[d][1,3]dioxo heterocyclopenten-4)-3-chlorobenzo[*b*]thiophene-2-formamide(VI-16)

Compound V-16 (1.77 g, 1.47 mmol) was used as the raw material, the operation process was the same as that of compound VI-1 to obtain 797 mg of a light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4-)-3-chlorobenzo[*b*]thiophene-2-formamide (I-16)

Compound VI-16 (500 mg, 0.91 mmol) and acryloyl chloride (87 mg, 0.96 mmol) were used as raw materials, the procedure was the same as that of compound I-1 to obtain 312 mg of a white solid with a yield of 56.8%, m.p. at 172-174°C. $^1$H-NMR (400 MHz, $CDCl_3$) $\delta$ (ppm): 9.13 (s, 1H, NH), 8.36 (s, 1H, ArH), 8.08 (d, *J* = 8.64 Hz, 1H, ArH), 7.97-7.91 (m, 1H, ArH), 7.91-7.85 (m, 1H, ArH), 7.59-7.48 (m, 2H, ArH), 7.15 (d, *J* = 8.68 Hz, 1H, ArH), 6.67-6.54 (m, 1H, $CH=CH_2$), 6.36-6.24 (m, 1H, $CH=CH_2$), 6.19 (s, 2H, $OCH_2O$), 5.89 (s, 1H, $NH_2$), 5.77-5.61 (m, 1H, $CH=CH_2$), 4.96-4.80 (m, 1.5H, $NC\underline{H}_2C\underline{H}$), 4.69-4.59 (m, 0.5H, $NCH_2C\underline{H}$), 4.28-4.16 (m, 0.5H, $NC\underline{H}_2CH$), 4.09-3.97 (m, 0.5H, $NC\underline{H}_2CH$), 3.86-3.73 (m, 0.5H, $NC\underline{H}_2CH_2$), 3.47-3.34 (m, 0.5H, $NC\underline{H}_2CH_2$), 3.26-3.15 (m, 0.5H, $NC\underline{H}_2CH_2$), 2.93-2.80 (m, 0.5H, $NC\underline{H}_2CH_2$), 2.50-2.34 (m, 1H, $NCH_2CHC\underline{H}_2$), 2.31-2.22 (m, 2H, $NCH_2CHC\underline{H}_2$, $NH_2$), 2.06-1.93 (m, 1H, $NCH_2C\underline{H}_2$), 1.81-1.66 (m, 1H, $NCH_2C\underline{H}_2$); $^{13}$C-NMR (101 MHz, $CDCl_3$) $\delta$ (ppm): 165.71, 158.68, 157.87, 155.78, 155.56, 154.14, 145.15, 138.71, 138.35, 137.56, 136.90, 132.84, 127.96, 127.70, 125.71, 123.42, 123.27, 122.93, 122.22, 119.39, 115.63, 111.52, 102.26, 99.65, 53.75(0.5C), 52.65(0.5C), 49.97(0.5C), 46.02(0.5C), 45.87(0.5C), 42.12(0.5C), 30.31(0.5C), 30.05(0.5C), 25.25(0.5C), 23.99(0.5C); HRMS (ESI): m/z $[M+H]^+$. Calcd for $C_{29}H_{25}ClN_7O_4S$: 602.1377; found: 602.1378.

Example 17

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)-1-naphthalenecarboxamide(I-17)

Synthesis of *N*-(7-(4,4,5,5-tetramethyl-1,3,2-dioxaborolane-2-yl)benzo[d][1,3]dioxo heterocyclopenten-4-)-1-naphthalenecarboxamide (IV-17)

Compound XI (1.00 g, 3.80 mmol) and 1-naphthoyl chloride (1.09 g, 5.70 mmol) were used as raw materials, the operation process was the same as that of compound IV-1 to obtain 1.47 g of red-brown solid with a yield of 93.0%, m.p. at 174-176°C. $^1$H-NMR (300 MHz, $CDCl_3$) $\delta$ (ppm): 8.39 (d, *J* = 7.65 Hz, 1H, ArH), 7.95 (d, *J* = 7.92 Hz, 2H, ArH), 7.88 (d, *J* = 6.99 Hz, 1H, ArH), 7.78 (s, 1H, NHCO), 7.74 (d, *J* = 6.87 Hz, 1H, ArH), 7.60-7.52 (m, 2H, ArH), 7.52-7.42 (m, 1H, ArH), 7.31 (d, *J* = 8.31 Hz, 1H, ArH), 6.01 (s, 2H, $OCH_2O$), 1.37 (s, 12H, $4CH_3$).

Synthesis of (*R*)-3-(3-(7-(1-naphthalenecarboxamido)benzo[*d*][1,3]dioxo heterocyclopenten-4)-4-amino- 1*H*-pyrazolo [3,4-d]pyrimidin-1-)piperidine-1-tert-butyl formate (V-17)

Compounds III-1 (0.80 g, 1.80 mmol) and IV-17 (0.90 g, 2.16 mmol) were used as raw materials, the operation process was the same as that of compound V-1 to obtain 751 mg of a light-yellow solid with a yield of 68.6%, m.p. at 144-146°C. $^1$H-NMR (300 MHz, $CDCl_3$) $\delta$(ppm): 8.45-8.40(m, 1H, ArH), 8.36(s, 1H, ArH), 8.14-8.07(m, 1H, ArH), 8.00(d, J=8.22 Hz, 1H, ArH), 7.95-7.90(m, 1H, ArH), 7.87(s, 1H, NH), 7.83-7.77(m, 1H, ArH), 7.62-7.56(m, 2H, ArH), 7.56-7.52(m, 1H, ArH), 7.18(d, *J* = 8.67 Hz, 1H, ArH), 6.10(s, 2H, $OCH_2O$), 5.91(s, 2H, $NH_2$), 4.92-4.78(m, 1H, $CHC\underline{H_2}NBoc$), 4.39-4.23(m, 1H, $C\underline{H}CH_2NBoc$), 4.21-4.07(m, 1H, $CHC\underline{H_2}NBoc$), 3.56-3.32(m, 1H, $NC\underline{H_2}CH_2$), 2.92-2.75(m, 1H, $NC\underline{H_2}CH_2$), 2.23-2.14(m, 2H, $NCH_2CHC\underline{H_2}$), 1.97-1.82(m, 1H, $NCH_2C\underline{H_2}$), 1.77-1.63(m, 1H, $NCH_2C\underline{H_2}$), 1.45(s, 9H, NBoc).

Synthesis of (*R*)-*N*-(7-(4-amino-1-(piperidin-3)-1*H*-pyrazolo[3,4-*d*]pyrimidin-3-)benzo[*d*][1,3]dioxo heterocyclopenten-4-)-1-naphthalenecarboxamide (VI-17)

Compound V-17 (700 mg, 1.15 mmol) was used as the raw material, the operation process was the same as that of compound VI-1 to obtain 498 mg of a light-yellow solid with a yield of 85.2%, which was directly used in the next step without further purification.

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)-1-naphthalenecarboxamide(I-17)

Compound VI-17 (441 mg, 0.87 mmol) and acryloyl chloride (83 mg, 0.91 mmol) were used as raw materials, the procedure was the same as that of compound I-1 to obtain 214 mg of a white solid with a yield of 43.8%, m.p. at 172.0-174°C. $^1$H-NMR (300 MHz, $CDCl_3$) $\delta$ (ppm): 8.42(d, *J*=7.98 Hz, 1H, ArH), 8.35(s, 1H, ArH), 8.09(d, *J*= 7.38 Hz, 1H, ArH), 8.00(d, *J*=8.16 Hz, 1H, ArH), 7.96-7.87(m, 2H, ArH, NH), 7.80 (d, *J*=6.72 Hz, 1H, ArH), 7.68-7.47(m, 3H, ArH), 7.17(d, *J*=8.55 Hz, 1H, ArH), 6.68-6.51 (m, 1H, $CH=CH_2$), 6.34-6.21(m, 1H, $CH=CH_2$), 6.10(s, 2H, $OCH_2O$), 5.88(s, 1H, $NH_2$), 5.75-5.61(m, 1H, $CH=CH_2$), 4.98-4.80(m, 1.5H, $NC\underline{H_2}C\underline{H}$), 4.67-4.54(m, 0.5H, $NCH_2C\underline{H}$), 4.28-4.14(m, 0.5H, $NC\underline{H_2}CH$), 4.07-3.94(m, 0.5H, $NC\underline{H_2}CH$), 3.84-3.68(m, 0.5H, $NC\underline{H_2}CH_2$), 3.42-3.29(m, 0.5H, $NC\underline{H_2}CH_2$), 3.24-3.09(m, 0.5H, $NC\underline{H_2}CH_2$), 2.94-2.78(m, 0.5H, $NC\underline{H_2}CH_2$), 2.47-2.33(m, 1H, $NCH_2CHC\underline{H_2}$), 2.30-2.19(m, 1H, $NCH_2CHC\underline{H_2}$), 2.06(s, 1H, $NH_2$), 2.02-1.92(m, 1H, $NCH_2C\underline{H_2}$), 1.82-1.64(m, 1H, $NCH_2C\underline{H_2}$); HRMS (ESI): m/z $[M+H]^+$. Calcd for $C_{31}H_{28}N_7O_4$: 562.2203; found: 562.2199.

Example 18

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-2-naphthalenecarboxamide (I-18)

Synthesis of N-(7-(4,4,5,5-tetramethyl-1,3,2-dioxaborolane-2-yl)benzo[*d*][1,3]dioxo heterocyclopenten-4-)-2-naphthalenecarboxamide (*IV*-18)

Compound XI (1.00 g, 3.80 mmol) and 2-naphthoyl chloride (1.09 g, 5.70 mmol) were used as raw materials, the operation process was the same as that of compound IV-1 to obtain 1.51 g of a white solid with a yield of 95.2%, m.p. at 152-154°C. $^1$H-NMR (300 MHz, $CDCl_3$) $\delta$ (ppm): 8.40(s, 1H, NH), 8.02-7.88(m, 6H, ArH), 7.64-7.55(m, 2H, ArH), 7.31(d, *J*= 8.58 Hz, 1H, ArH), 6.11(s, 2H, $OCH_2O$), 1.37(s, 12H, $4CH_3$).

Synthesis of (*R*)-3-(3-(7-(2-naphthalenecarboxamido)benzo[*d*][1,3]dioxo heterocyclopenten-4)-4-amino- 1*H*-pyrazolo [3,4-*d*]pyrimidin-1-)piperidine-1-tert-butyl formate (V-18)

Compounds III-1 (0.75 g, 1.69 mmol) and IV-18 (0.85 g, 2.03 mmol) were used as raw materials, the operation process was the same as that of compound V-1 to obtain 757 mg of a light-yellow solid with a yield of 73.8%, m.p. at 118-120°C. $^1$H-NMR (300 MHz, $CDCl_3$) $\delta$ (ppm): 8.44 (s, 1H, ArH), 8.37 (s, 1H, ArH), 8.12-8.04 (m, 2H, ArH, NH), 8.02-7.89 (m, 4H, ArH), 7.67-7.55 (m, 2H, ArH), 7.17 (d, *J* = 8.76 Hz, 1H, ArH), 6.16 (s, 2H, $OCH_2O$), 5.76 (s, 2H, $NH_2$), 4.94-4.78 (m, 1H, $CHC\underline{H_2}NBoc$), 4.42-4.24 (m, 1H, $C\underline{H}CH_2NBoc$), 4.21-4.04 (m, 1H, $CHC\underline{H_2}NBoc$), 3.57-3.37 (m, 1H, $NC\underline{H_2}CH_2$), 2.92-2.75 (m, 1H, $NC\underline{H_2}CH_2$), 2.34-2.14 (m, 2H, $NCH_2CHC\underline{H_2}$), 1.95-1.82 (m, 1H, $NCH_2C\underline{H_2}$), 1.77-1.62 (m, 1H, $NCH_2C\underline{H_2}$), 1.45 (s, 9H, NBoc).

Synthesis of (*R*)-N-(7-(4-amino-1-(piperidin-3)-1*H*-pyrazolo[3,4-*d*]pyrimidin-3)benzo[*d*][1,3]dioxo heterocyclopenten-4)-2-naphthalenecarboxamide (VI-18)

Compound V-18 (700 mg, 1.15 mmol) was used as the raw material, the operation process was the same as that of compound VI-1 to obtain 448 mg of a light-yellow solid with a yield of 76.6%.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-2-naphthalenecarboxamide (I-18)

Compound VI-18 (420 mg, 0.83 mmol) and acryloyl chloride (79 mg, 0.87 mmol) were used as raw materials, the procedure was the same as that of compound I-1 to obtain 247 mg of a white solid with a yield of 53.1%, m.p. at 172.5-174°C. $^1H$-NMR (300 MHz, $CDCl_3$) $\delta$ (ppm): 8.44 (s, 1H, ArH), 8.39-8.30 (m, 1H, ArH), 8.30-8.22 (m, 1H, ArH), 8.05-7.86 (m, 4H, ArH), 7.68-7.53 (m, 2H, ArH), 7.14 (d, *J* = 8.25 Hz, 1H, ArH), 6.68-6.51 (m, 1H, $CH=CH_2$), 6.37-6.22 (m, 1H, $CH=CH_2$), 6.14 (s, 2H, $OCH_2O$), 5.97 (br, 1H, $NH_2$), 5.77-5.61 (m, 1H, $CH=CH_2$), 4.97-4.77 (m, 1.5H, $N\underline{CH_2}\underline{CH}$), 4.68-4.55 (m, 0.5H, $NCH_2\underline{CH}$), 4.27-4.15 (m, 0.5H, $NC\underline{H_2}CH$), 4.09-3.95 (m, 0.5H, $NC\underline{H_2}CH$), 3.86-3.69 (m, 0.5H, $NC\underline{H_2}CH_2$), 3.44-3.29 (m, 0.5H, $NC\underline{H_2}CH_2$), 3.26-3.09 (m, 0.5H, $NC\underline{H_2}CH_2$), 2.93-2.76 (m, 0.5H, $NC\underline{H_2}CH_2$), 2.49-2.31 (m, 1H, $NCH_2CHC\underline{H_2}$), 2.30-2.11 (m, 2H, $NCH_2CHC\underline{H_2}$, $NH_2$), 2.03-1.91 (m, 1H, $NCH_2C\underline{H_2}$), 1.79-1.62 (m, 1H, $NCH_2C\underline{H_2}$); RMS (ESI): m/z $[M+H]^+$. Calcd for $C_{31}H_{28}N_7O_4$: 562.2203; found: 562.2205.

Example 19

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-methyl-1-naphthalenecarboxamide (I-19)

Synthesis of 4-methyl-*N*-(7-(4,4,5,5-tetramethyl-1,3,2-dioxaborolane-2-yl)benzo[*d*][1,3]dioxo heterocyclopenten-4-)-1-naphthalenecarboxamide (IV-19)

Compound *XI* (1.00 g, 3.80 mmol) and 4-methyl-1-naphthalenecarboxylic acid (1.06 g, 5.70 mmol) were used as raw materials, the operation process was the same as that of compound *IV*-2 to obtain 1.52 g of a light-yellow solid with a yield of 92.7%, m.p. at 142-144°C. $^1H$-NMR (300 MHz, $CDCl_3$) $\delta$ (ppm): 8.48-8.39 (m, 1H, ArH), 8.09-8.01 (m, 1H, ArH), 8.00-7.92 (m, 1H, ArH), 7.71 (s, 1H, NHCO), 7.65 (d, *J* = 6.87 Hz, 1H, ArH), 7.61-7.53 (m, 2H, ArH), 7.38-7.27 (m, 2H, ArH), 6.02 (s, 2H, $OCH_2O$), 2.74 (s, 3H, $CH_3$), 1.37 (s, 12H, $4CH_3$).

Synthesis of (*R*)-3-(4-amino-3-(7-(4-methyl-1-naphthalenecarboxamido)benzo [*d*][1,3]dioxo heterocyclopenten-4)-1*H*-pyrazolo[3,4-*d*]pyrimidin-1-yl)piperidine-1-tert-butyl formate (V-19)

Compounds III-1 (0.73 g, 1.65 mmol) and IV-19 (1.07 g, 2.48 mmol) were used as raw materials, the operation process was the same as that of compound V-1 to obtain 719 mg of a light-yellow solid with a yield of 70.0%, m.p. at 140-142°C. $^1H$-NMR (300 MHz, $CDCl_3$) $\delta$ (ppm): 8.48-8.38 (m, 1H, ArH), 8.29 (s, 1H, ArH), 8.22 (s, 1H, NH), 8.07-7.98 (m, 1H, ArH), 7.97-7.88 (m, 1H, ArH), 7.64 (d, *J= 7.11* Hz, 1H, ArH), 7.60-7.49 (m, 2H, ArH), 7.28 *(d, J* = 7.35 Hz, 1H, ArH), 7.10 (d, *J* = 8.49 Hz, 1H, ArH), 6.24 (br, 2H, $NH_2$), 6.03 (s, 2H, $OCH_2O$), 4.86-4.73 (m, 1H, $CHC\underline{H_2}NBoc$), 4.41-4.22 (m, 1H, $\underline{CH}CH_2NBoc$), 4.17-3.99 (m, 1H, $CHC\underline{H_2}NBoc$), 3.52-3.24 (m, 1H, $NC\underline{H_2}CH_2$), 2.82-2.61 (m, 4H, $NC\underline{H_2}CH_2$, $CH_3$), 2.31-2.07 (m, 2H, $NCH_2CHC\underline{H_2}$), 1.92-1.76 (m, 1H, $NCH_2C\underline{H_2}$), 1.74-1.57 (m, 1H, $NCH_2C\underline{H_2}$), 1.44 (s, 9H, NBoc).

Synthesis of (*R*)-*N*-(7-(4-amino-1-(piperidin-3)-1H-pyrazolo[3,4-d]pyrimidin-3-)benzo[*d*][1,3]dioxo heterocyclopenten-4)-4-methyl-1-naphthalenecarboxamido (VI-19)

Compound V-19 (700 mg, 1.13 mmol) was used as the raw material, the operation process was the same as that of compound VI-1 to obtain 548 mg of a light-yellow solid with a yield of 93.4%, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-methyl-1-naphthalenecarboxamide (I-19)

Compound VI-19 (500 mg, 0.96 mmol) and acryloyl chloride (91 mg, 1.01 mmol) were used as raw materials, the procedure was the same as that of compound I-1 to obtain 289 mg of a white solid with a yield of 53.3%, m.p. at 178-179°C. $^1H$-NMR (300 MHz, $CDCl_3$) $\delta$ (ppm): 8.44 (s, 1H, ArH), 8.31 (d, *J* = 7.62 Hz, 1H, ArH), 8.21-7.92 (m, 3H, ArH, NH), 7.68 (d, *J* = 6.48 Hz, 1H, ArH), 7.64-7.49 (m, 2H, ArH), 7.33 (d, *J* = 6.09 Hz, 1H, ArH), 7.13 (d, *J* = 7.17 Hz, 1H, ArH), 6.66-6.48 (m, 1H,

$CH=CH_2$), 6.31-6.20 (m, 1H, $CH=CH_2$), 6.17-5.98 (m, 3H, $OCH_2O$, $NH_2$), 5.75-5.57 (m, 1H, $CH=CH_2$), 4.95-4.76 (m, 1.5H, $NC\underline{H}_2C\underline{H}$), 4.67-4.50 (m, 0.5H, $NCH_2C\underline{H}$), 4.29-4.09 (m, 0.5H, $NC\underline{H}_2CH$), 4.08-3.89 (m, 0.5H, $NC\underline{H}_2CH$), 3.83-3.64 (m, 0.5H, $NC\underline{H}_2CH_2$), 3.42-3.26 (m, 0.5H, $NC\underline{H}_2CH_2$), 3.22-3.08 (m, 0.5H, $NC\underline{H}_2CH_2$), 2.90-2.79 (m, 0.5H, $NC\underline{H}_2CH_2$), 2.73 (s, 3H, $CH_3$), 2.56-2.17 (m, 3H, $NCH_2CHC\underline{H}_2$, $NH_2$), 2.03-1.87 (m, 1H, $NCH_2C\underline{H}_2$), 1.78-1.58 (m, 1H, $NCH_2C\underline{H}_2$); HRMS (ESI): m/z $[M+H]^+$. Calcd for $C_{32}H_{30}N_7O_4$: 576.2359; found: 576.2357.

Example 20

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)-5,6,7,8-tetrahydronaphthalene-1-carboxamide (I-20)

Synthesis of *N*-(7-(4,4,5,5-tetramethyl-1,3,2-dioxaborolane-2-yl)benzo[d][1,3]dioxo heterocyclopenten-4)-5,6,7,8-tetrahydronaphthalene-1-carboxamide(IV-20)

Compound *XI* (1.00 g, 3.80 mmol) and 5,6,7,8-tetrahydronaphthalene-1-carboxamid (1.00 g, 5.70 mmol) were used as raw materials, the operation process was the same as that of compound *IV*-2 to obtain 1.51 g of a white solid with a yield of 94.4%, m.p. at 168-170°C. $^1$H-NMR(300 MHz, $CDCl_3$) $\delta$(ppm): 7.92(d, *J*=8.37 Hz, 1H, ArH), 7.47(s, 1H, NHCO), 7.33-7.27(m, 2H, ArH), 7.21-7.14(m, 2H, ArH), 6.04(s, 2H, $OCH_2O$), 3.02-2.77(m, 4H, $C\underline{H}_2CH_2CH_2C\underline{H}_2$), 1.88-1.73(m, 4H, $CH_2C\underline{H}_2C\underline{H}_2CH_2$), 1.36(s, 12H, $4CH_3$).

Synthesis of (*R*)-3-(4-amino-3-(7-(5,6,7,8-tetrahydronaphthalene-1-carboxamide)benzo[d][1,3]dioxo heterocyclopenten-4)-1*H*-pyrazolo[3,4-*d*]pyrimidin-1-)piperidine-1-tert-butyl formate (V-20)

Compound III-1 (0.75 g, 1.69 mmol) and IV-20 (0.85 g, 2.03 mmol) were used as raw materials, the operation process was the same as that of compound V-1 to obtain 784 mg of a light-yellow solid with a yield of 75.9%, m.p. at 150-152°C. $^1$H-NMR (300 MHz, $CDCl_3$) $\delta$ (ppm): 8.36 (s, 1H, ArH), 8.05 (d, *J*= 8.76 Hz, 1H, ArH), 7.56 (s, 1H, NH), 7.37-7.30 (m, 1H, ArH), 7.25-7.10 (m, 3H, ArH), 6.10 (s, 2H, $OCH_2O$), 5.80 (s, 2H, $NH_2$), 4.91-4.78 (m, 1H, $CHC\underline{H}_2NBoc$), 4.41-4.22 (m, 1H, $C\underline{H}CH_2NBoc$), 4.21-4.06 (m, 1H, $CHC\underline{H}_2NBoc$), 3.53-3.33 (m, 1H, $NC\underline{H}_2CH_2$), 3.05-2.91 (m, 2H, $CH_2C$), 2.90-2.76 (m, 3H, $CH_2C$, $NC\underline{H}_2CH_2$), 2.33-2.15 (m, 2H, $NCH_2CHC\underline{H}_2$), 1.96-1.86 (m, 1H, $NCH_2C\underline{H}_2$), 1.86-1.76 (m, 4H, $CCH_2C\underline{H}_2C\underline{H}_2CH_2C$), 1.74-1.63 (m, 1H, $NCH_2C\underline{H}_2$), 1.45 (s, 9H, NBoc).

Synthesis of (*R*)-*N*-(7-(4-amino-1-(piperidin-3)-1*H*-pyrazolo[3,4-*d*]pyrimidin-3-)benzo[*d*][1,3]dioxo heterocyclopenten-4)-5,6,7,8-tetrahydronaphthalene-1-carboxamide (VI-20)

Compound V-20 (700 mg, 1.14 mmol) was used as the raw material, the operation process was the same as that of compound VI-1 to obtain 472 mg of a light-yellow solid with a yield of 80.62%.

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)-5,6,7,8-tetrahydronaphthalene-1-carboxamide (I-20)

Compound VI-20 (420 mg, 0.82 mmol) and acryloyl chloride (78 mg, 0.86 mmol) were used as raw materials, the procedure was the same as that of compound I-1 to obtain 316 mg of a white solid with a yield of 68.1%, m.p. at 158-160°C. $^1$H-NMR (400 MHz, $CDCl_3$) $\delta$(ppm): 8.33(s, 1H, ArH), 8.11-7.92 (m, 1H, ArH), 7.63 (s, 1H, NH), 7.38-7.29 (m, 1H, ArH), 7.24-7.15 (m, 2H, ArH), 7.12(d, *J*=8.40 Hz, 1H, ArH), 6.67-6.50 (m, 1H, $CH=CH_2$), 6.34-6.20 (m, 1H, $CH=CH_2$), 6.09 (s, 2H, $OCH_2O$), 5.88 (br, 1H, $NH_2$), 5.75-5.58 (m, 1H, $CH=CH_2$), 4.97-4.75 (m, 1.5H, $NC\underline{H}_2C\underline{H}$), 4.69-4.52 (m, 0.5H, $NCH_2C\underline{H}$), 4.27-4.13 (m, 0.5H, $NC\underline{H}_2CH$), 4.10-3.94 (m, 0.5H, $NC\underline{H}_2CH$), 3.86-3.67 (m, 0.5H, $NC\underline{H}_2CH_2$), 3.45-3.29 (m, 0.5H, $NC\underline{H}_2CH_2$), 3.26-3.11 (m, 0.5H, $NC\underline{H}_2CH_2$), 3.03-2.90 (m, 2H, $C\underline{H}_2CH_2CH_2C\underline{H}_2$), 2.89-2.75 (m, 2.5H, $NC\underline{H}_2CH_2$, $C\underline{H}_2CH_2CH_2C\underline{H}_2$), 2.46-2.31 (m, 1H, $NCH_2CHC\underline{H}_2$), 2.30-2.20 (m, 1H, $NCH_2CHC\underline{H}_2$), 2.10(br, 1H, $NH_2$), 2.03-1.91(m, 1H, $NCH_2C\underline{H}_2$), 1.86-1.65 (m, 5H, $CH_2C\underline{H}_2C\underline{H}_2CH_2$, $NCH_2C\underline{H}_2$); HRMS (ESI): m/z $[M+H]^+$. Calcd for $C_{31}H_{32}N_7O_4$: 566.2516; found: 566.2512.

Example 21

Synthesis of (*R*)-*N*-(7-(7-(1-acryloylpiperidin-3-yl)-4-amino-7*H*-pyrrolo[2,3-*d*]pyrimidin-5-yl)benzo[*d*][1,3]dioxo heterocyclopenten-4-)-1-naphthalenecarboxamide(I-21)

Synthesis of (*R*)-3-(4-amino-5-iodo-7*H*-pyrrolo[2,3-d]pyrimidin-7-yl)piperidine-1-tert-butyl formate (III-3)

5-iodo-7*H*-pyrrolo[2,3-*d*]pyrimidine-4-amine(VII-2) (1.00 g, 3.85 mmol), (*S*)-1-tert-butoxycarbonyl-3-hydroxy piperidine (VIII-1) (1.55 g, 7.69 mmol), $PPh_3$ (2.02 g, 7.69 mmol), anhydrous THF (100 mL) were added to a 250 mL three-necked flask under nitrogen protection, the mixture was stirred for 30 min under an ice bath; then, DIAD (1.56 g, 7.69 mmol) was added. Next, the mixture was reacted at a room temperature for 18 h. The THF was distilled off, the residue was dissolved in ethyl acetate (100 mL), and washed successively with saturated sodium carbonate (30 mL × 3), water (30 mL × 3) and saturated sodium chloride (30 mL×3), and was dried by anhydrous $Na_2SO_4$. After suction filtration, the filtrate was evaporated to remove the solvent under reduced pressure to obtain a residue, which was separated by column chromatography (eluent: DCM:MeOH=150-60:1), 1.71 g of a white solid was obtained with a yield of 70.6%, m.p. at 126-128°C. $^1$H-NMR (300 MHz, $CDCl_3$) $\delta$ (ppm): 8.30 (s, 1H, ArH), 7.16 (s, 1H, ArH), 5.92 (s, 2H, $NH_2$), 4.82-4.66 (m, 1H, $NC\underline{H_2}CH$), 4.30-4.11 (m, 1H, $NCH_2C\underline{H}$), 4.07-3.94 (m, 1H, $NC\underline{H_2}CH$), 3.35-3.19 (m, 1H, $NC\underline{H_2}CH_2$), 3.10-2.93 (m, 1H, $NC\underline{H_2}CH_2$), 2.24-2.12 (m, 1H, $CHC\underline{H_2}CH_2$), 2.09-1.97 (m, 1H, $CHC\underline{H_2}CH_2$), 1.91-1.79 (m, 1H, $CHCH_2C\underline{H_2}$), 1.78-1.68 (m, 1H, $CHCH_2C\underline{H_2}$), 1.49 (s, 9H, $C(CH_3)_3$).

Synthesis of (R)-3-(5-(7-(1-naphthylamide)benzo[d][1,3]dioxol-4-yl)-4-amino-7H-pyrrolo[ 2,3-d]pyrimidin-7-yl) piperidine-1-carboxylate tert-butyl ester (V-21)

Compounds III-3 (0.48 g, 1.08 mmol) and IV-17 (0.54 g, 1.30 mmol) were used as raw materials, the operation process was the same as that of compound V-1 to obtain 353 mg of a light-yellow solid with a yield of 53.7%, m.p. at 124-126°C.

Synthesis of (*R*)-N-(7-(4-amino-7-(piperidin-3)-7*H*-pyrrolo[2,3-d]pyrimidin-5-)benzo[*d*][1,3]dioxo heterocyclopenten-4)-1-naphthylformamide (VI-21)

Compound V-21 (580 mg, 0.96 mmol) was used as the raw material, the operation process was the same as that of compound VI-1 to obtain 340 mg of a light-yellow solid with a yield of 70.2%, which was directly used in the next step without further purification.

Synthesis of (*R*)-*N*-(7-(7-(1-acryloylpiperidin-3-yl)-4-amino-7*H*-pyrrolo[2,3-*d*]pyrimidin-5-yl)benzo[*d*][1,3]dioxo heterocyclopenten-4-)-1-naphthalenecarboxamide(I-21)

Compound VI-21 (337 mg, 0.67 mmol) and acryloyl chloride (65 mg, 0.70 mmol) were used as raw materials, the procedure was the same as that of compound I-1 to obtain 189 mg of a white solid with a yield of 50.7%, m.p. at 166-167.5°C. $^1$H-NMR (300 MHz, $CDCl_3$) $\delta$ (ppm): 8.43 (d, *J* = 7.41 Hz, 1H, ArH), 8.31 (s, 1H, ArH), 8.06-7.96 (m, 2H, ArH), 7.94-7.84 (m, 2H, ArH), 7.79 (d, *J* = 6.51 Hz, 1H, ArH), 7.65-7.46 (m, 3H, ArH, NH), 7.16 (s, 1H, ArH), 7.01 (d, *J* = 8.49 Hz, 1H, ArH), 6.70-6.54 (m, 1H, $CH{=}CH_2$), 6.37-6.25 (m, 1H, $CH{=}CH_2$), 6.05 (s, 2H, $OCH_2O$), 5.78-5.65 (m, 1H, $CH{=}CH_2$), 5.40 (s, 1H, $NH_2$), 4.89-4.53 (m, 2H, $NC\underline{H_2}C\underline{H}$), 4.39-4.23 (m, 0.5H, $NC\underline{H_2}CH$), 4.09-3.89 (m, 0.5H, $NC\underline{H_2}CH$), 3.44-3.14 (m, 1.5H, $NC\underline{H_2}CH_2$), 2.96-2.77 (m, 0.5H, $NC\underline{H_2}CH_2$), 2.34-2.24 (m, 1H, $NCH_2CHC\underline{H_2}$), 2.22-2.09 (m, 2H, $NCH_2CHC\underline{H_2}$, $NH_2$), 2.02-1.88 (m, 1H, $NCH_2C\underline{H_2}$), 1.82-1.65 (m, 1H, $NCH_2C\underline{H_2}$); HRMS (ESI): m/z $[M+H]^+$. Calcd for $C_{32}H_{29}N_6O_4$: 561.2250; found: 561.2253.

Example 22

Synthesis of (*R*)-*N*-(7-(7-(1-acryloylpiperidin-3-yl)-4-amino-7*H*-pyrrolo[2,3-*d*]pyrimidin-5-yl)benzo[*d*][1,3]dioxo heterocyclopenten-4-)-2-naphthalenecarboxamide(I-22)

Synthesis of (R)-3-(5-(7-(1-naphthylamide)benzo[*d*][1,3]dioxol-4-yl)-4-amino-7H-pyrrolo[ 2,3-*d*]pyrimidin-7-yl) piperidine-1-carboxylate tert-butyl ester (V-22)

Compounds III-3 (1.00 g, 2.26 mmol) and IV-18 (1.13 g, 2.71 mmol) were used as raw materials, the operation process was the same as that of compound V-1 to obtain 988 mg of a light-yellow solid with a yield of 72.2%, m.p. at 124-126°C. $^1$H-NMR (300 MHz, $CDCl_3$) $\delta$ (ppm): 8.43 (s, 1H, ArH), 8.33 (s, 1H, ArH), 8.09 (s, 1H, NH), 8.03-7.86 (m, 5H, ArH), 7.66-7.55 (m, 2H, ArH), 7.19 (s, 1H, ArH), 7.00 (d, *J* = 8.91 Hz, 1H, ArH), 6.10 (s, 2H, $OCH_2O$), 5.36 (s, 2H, $NH_2$), 4.84-4.67 (m, 1H, $CHC\underline{H_2}N$), 4.33-4.19 (m, 1H, $C\underline{H}CH_2N$), 4.11-3.96 (m, 1H, $CHC\underline{H_2}N$), 3.31-3.16 (m, 1H, $NC\underline{H_2}CH_2$), 3.02-2.85 (m, 1H, $NC\underline{H_2}CH_2$), 2.33-2.15 (m, 2H, $CHC\underline{H_2}CH_2$), 2.08-1.99 (m, 1H, $CHCH_2C\underline{H_2}$), 1.87-1.78 (m, 1H, $CHCH_2C\underline{H_2}$), 1.46 (s, 9H, $C(CH_3)_3$).

Synthesis of (R)-*N*-(7-(4-Amino-7-(piperidin-3-yl)-7*H*-pyrrolo[2,3-d]pyrimidin-5-yl)benzo[*d*][1, 3] Dioxol-4-yl)-2-naphthylcarboxamide (VI-22)

Compound V-22 (988 mg, 1.63 mmol) was used as the raw material, the procedure was the same as that of compound VI-1 to obtain 825 mg of a light-yellow solid, which was directly used in the next step without further purification.

Synthesis of (*R*)-*N*-(7-(7-(1-acryloylpiperidin-3-yl)-4-amino-7*H*-pyrrolo[2,3-*d*]pyrimidin-5-yl)benzo[*d*][1,3]dioxo heterocyclopenten-4-)-2-naphthalenecarboxamide(I-22)

Compound VI-22 (825 mg, 1.63 mmol) and acryloyl chloride (155 mg, 1.71 mmol) were used as raw materials, the procedure was the same as that of compound I-1 to obtain 356 mg of a white solid with a yield of 38.9%, m.p. at 149.5-151°C. $^1$H-NMR (400 MHz, $CDCl_3$) $\delta$ (ppm): 8.44 (s, 1H, ArH), 8.32 (s, 1H, ArH), 8.23 (s, 1H, NH), 8.01-7.88 (m, 5H, ArH), 7.68-7.53 (m, 2H, ArH), 7.23-7.12 (m, 1H, ArH), 6.99 (s, 1H, ArH), 6.70-6.57 (m, 1H, $CH{=}CH_2$), 6.38-6.27 (m, 1H, $CH{=}CH_2$), 6.10 (s, 2H, $OCH_2O$), 5.78-5.66 (m, 1H, $CH{=}CH_2$), 5.47 (s, 1H, $NH_2$), 4.91-4.67 (m, 1.5H, $NC\underline{H_2}C\underline{H}$), 4.67-4.55 (m, 0.5H, $NCH_2C\underline{H}$), 4.39-4.27 (m, 0.5H, $NC\underline{H_2}CH$), 4.08-3.94 (m, 0.5H, $NC\underline{H_2}CH$), 3.45-3.33(m, 0.5H, $NC\underline{H_2}CH_2$), 3.31-3.15(m, 1H, $NC\underline{H_2}CH_2$), 2.92-2.79(m, 0.5H, $NC\underline{H_2}CH_2$), 2.42-2.26(m, 2H, $NCH_2CHC\underline{H_2}$, $NH_2$), 2.23-2.09(m, 1H, $NCH_2CHC\underline{H_2}$), 2.02-1.88(m, 1H, $NCH_2C\underline{H_2}$), 1.83-1.66(m, 1H, $NCH_2C\underline{H_2}$); HRMS (ESI): m/z $[M+H]^+$. Calcd for $C_{32}H_{29}N_6O_4$: 561.2250; found: 561.2254.

Example 23

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-2-phenylacetamide (I-23)

Synthesis of 2-phenyl-*N*-(7-(4,4,5,5-tetramethyl-1,3,2-dioxaborolane-2-yl)benzo[*d*][1,3]dioxo heterocyclopenten-4-) acetamide (IV-23)

Compound XI (500 mg, 1.90 mmol), phenylacetic acid (388 mg, 2.85 mmol) and 1*H*-benzotriazol-1-oxytripyrrolidinylphosphonium hexafluorophosphate (PyBOP) (1.48 g, 2.84 mmol ) were dissolved in DMF (6 mL), DIEA (491 mg, 3.80 mmol) was added, and then reaction was conducted at a room temperature for 10 h. The post-processing method was the same as that of compound IV-2 to obtain 215 mg of a white solid with a yield of 29.7%, m.p. at 152-154°C. $^1$H-NMR(300 MHz, $CDCl_3$)$\delta$(ppm): 7.72(d, *J*=8.10 Hz, 1H, ArH), 7.45-7.29(m, 5H, ArH), 7.20(d, *J*=8.46 Hz, 1H, ArH), 7.14(s, 1H, CONH), 5.96(s, 2H, $OCH_2O$), 3.76(s, 2H, $COCH_2Ph$), 1.34(s, 12H, $4CH_3$).

Synthesis of (*R*)-3-(4-amino-3-(7-(2-phenylacetamide)benzo[*d*][1,3]dioxo heterocyclopenten-4)-1*H*-tert-butyl-pyrazolo [3,4-*d*]pyrimidin-1-)piperidine-1-tert-butyl formate (V-23)

Compounds III-1 (0.20 g, 0.45 mmol) and IV-23 (0.21 g, 0.54 mmol) were used as the raw materials, the operation process was the same as that of compound V-1 to obtain 125 mg of a light-yellow solid with a yield of 48.5%, m.p. at 130-132°C. $^1$H-NMR (300 MHz, $CDCl_3+D_2O$) $\delta$ (ppm): 8.33 (s, 1H, ArH), 7.83 (d, *J* = 8.67 Hz, 1H, ArH), 7.46-7.30 (m, 5H, ArH), 7.05 (d, *J* = 8.70 Hz, 1H, ArH), 6.00 (s, 2H, $OCH_2O$), 4.90-4.75 (m, 1H, $CHC\underline{H_2}NBoc$), 4.38-4.21 (m, 1H, $C\underline{H}CH_2NBoc$), 4.18-4.03 (m, 1H, $CHC\underline{H_2}NBoc$), 3.78 (s, 2H, $COC\underline{H_2}Ph$), 3.49-3.29 (m, 1H, $NC\underline{H_2}CH_2$), 2.89-2.74 (m, 1H, $NC\underline{H_2}CH_2$), 2.31-2.10 (m, 2H, $NCH_2CHC\underline{H_2}$), 1.91-1.77 (m, 1H, $NCH_2C\underline{H_2}$), 1.74-1.57 (m, 1H, $NCH_2C\underline{H_2}$), 1.43 (s, 9H, NBoc).

Synthesis of (*R*)-*N*-(7-(4-amino-1-(piperidin-3)-1*H*-pyrazolo[3,4-d]pyrimidin-3)benzo[d][1,3]dioxo heterocyclopenten-4)-2-phenylacetamide(VI-23)

Compound V-23 (0.21 g, 0.37 mmol) was used as the raw material, the operation process was the same as that of compound VI-1 to obtain 136 mg of a light-yellow solid with a yield of 78.51%.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-2-phenylacetamide (I-23)

Compound VI-23 (136 mg, 0.29 mmol) and acryloyl chloride (31 mg, 0.34 mmol) were used as raw materials, the procedure was the same as compound I-1 to obtain 61 mg of a white solid with a yield of 40.4%, m.p. at 118-119.5°C. $^1$H-NMR (300 MHz, CDCl3) $\delta$ (ppm): 8.36 (s, 1H, ArH), 7.87 (d, *J* = 7.20 Hz, 1H, ArH), 7.51-7.33 (m, 5H, ArH, NH), 7.23 (s, 1H, NH), 7.08 (d, *J* = 8.46 Hz, 1H, ArH), 6.70-6.52 (m, 1H, $CH{=}CH_2$), 6.37-6.23 (m, 1H, $CH{=}CH_2$), 6.03 (s, 2H, $OCH_2O$),

5.78-5.60 (m, 3H, $CH=CH_2$, $NH_2$), 4.94-4.82 (m, 1H, $NC\underline{H}_2CH$), 4.70-4.56 (m, 0.5H, $NCH_2C\underline{H}$), 4.29-4.13 (m, 0.5H, $NCH_2C\underline{H}$), 4.10-3.96 (m, 0.5H, $NC\underline{H}_2CH$), 3.88-3.73 (m, 2.5H, $NC\underline{H}_2CH$, $COC\underline{H}_2Ph$), 3.46-3.30 (m, 0.5H, $NC\underline{H}_2CH_2$), 2.96-2.81 (m, 0.5H, $NC\underline{H}_2CH_2$), 2.50-2.35 (m, 1H, $NC\underline{H}_2CH_2$), 2.32-2.25 (m, 1H, $NCH_2CHC\underline{H}_2$), 2.08-1.94 (m, 1H, $NCH_2CHC\underline{H}_2$), 1.93-1.87 (m, 1H, $NCH_2C\underline{H}_2$), 1.76-1.66 (m, 1H, $NCH_2C\underline{H}_2$); HRMS (ESI): m/z $[M+H]^+$. Calcd for $C_{28}H_{28}N_7O_4$: 526.2203; found: 526.2199.

Example 24

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)-[1,1'-Biphenyl]-4-carboxamide (I-24)

Synthesis of *N*-(7-(4,4,5,5-tetramethyl-1,3,2-dioxaborolan-2-yl)benzo[*d*][1,3]dioxo heterocyclopenten-4-)-[1,1'-Biphenyl]-4-carboxamide (IV-24)

Compound *XI* (1.00 g, 3.80 mmol) and 4-phenylbenzoic acid (1.13 g, 5.70 mmol) were used as raw materials, the operation process was the same as that of compound *IV*-2 to obtain 1.48 g of a white solid with a yield of 88.1%, m.p. at 120-122°C. $^1$H-NMR (300 MHz, $CDCl_3$) $\delta$ (ppm): 8.00-7.88 (m, 4H, ArH), 7.72 (d, $J$ = 8.01 Hz, 2H, ArH), 7.63 (d, $J$ = 6.96 Hz, 2H, ArH), 7.52-7.45 (m, 2H, ArH, NH), 7.43-7.40 (m, 1H, ArH), 7.30 (d, $J$ = 8.64 Hz, 1H, ArH), 6.09 (s, 2H, $OCH_2O$), 1.37 (s, 12H, $4CH_3$).

Synthesis of (*R*)-3-(3-(7-([1,1'-Biphenyl]-4-ylcarboxamido)benzo[*d*][1,3]dioxo heterocyclopenten-4)-4-amino- 1*H*-pyrazolo[3,4-*d*]pyrimidin-1-)piperidine-1-tert-butyl formate (V-24)

Compounds III-1 (0.80 g, 1.80 mmol) and IV-24 (0.96 g, 2.16 mmol) were used as raw materials, the operation process was the same as that of compound V-1 to obtain 821 mg of a light-yellow solid with a yield of 71.9%, m.p. at 151-152°C. $^1$H-NMR (300 MHz, $CDCl_3+D_2O$) $\delta$ (ppm): 8.36 (s, 1H, ArH), 8.09-7.95 (m, 3H, ArH), 7.79-7.72 (m, 2H, ArH), 7.69-7.61 (m, 2H, ArH), 7.54-7.38 (m, 3H, ArH), 7.16 (d, $J$ = 8.16 Hz, 1H, ArH), 6.15 (s, 2H, $OCH_2O$), 4.96-4.76 (m, 1H, $CHC\underline{H}_2NBoc$), 4.40-4.23 (m, 1H, $C\underline{H}CH_2NBoc$), 4.19-4.08 (m, 1H, $CHC\underline{H}_2NBoc$), 3.59-3.25 (m, 1H, $NC\underline{H}_2CH_2$), 2.96-2.74 (m, 1H, $NC\underline{H}_2CH_2$), 2.38-2.15 (m, 2H, $NCH_2CHC\underline{H}_2$), 1.94-1.86 (m, 1H, $NCH_2C\underline{H}_2$), 1.79-1.62 (m, 1H, $NCH_2C\underline{H}_2$), 1.45 (s, 9H, NBoc).

Synthesis of (*R*)-N-(7-(4-amino-1-(piperidin-3)-1*H*-pyrazolo[3,4-*d*]pyrimidin-3-)benzo[*d*][1,3]dioxo heterocyclopenten-4-)[1,1'-Biphenyl]-4-carboxamide (VI-24)

Compound V-24 (750 mg, 1.18 mmol) was used as the raw material, the operation process was the same as that of compound VI-1 to obtain 468 mg of a light-yellow solid with a yield of 74.1%, which was directly used in the next step without further purification.

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)-[1,1'-Biphenyl]-4-carboxamide (I-24)

Compound VI-24 (416 mg, 0.78 mmol) and acryloyl chloride (74 mg, 0.82 mmol) were used as raw materials, the procedure was the same as that of compound I-1 to obtain 212 mg of a white solid with a yield of 46.3%, m.p. at 216-218°C. $^1$H-NMR (300 MHz, $CDCl_3$) $\delta$ (ppm): 8.37 (s, 1H, ArH), 8.14-7.94 (m, 4H, ArH, NH), 7.74 (d, $J$ = 7.65 Hz, 2H, ArH), 7.65 (d, $J$ = 7.65 Hz, 2H, ArH), 7.56-7.37 (m, 3H, ArH), 7.15 (d, $J$ = 8.67 Hz, 1H, ArH), 6.69-6.51 (m, 1H, $CH=CH_2$), 6.35-6.25 (m, 1H, $CH=CH_2$), 6.15 (s, 2H, $OCH_2O$), 5.77 (s, 1H, $NH_2$), 5.73-5.63 (m, 1H, $CH=CH_2$), 4.98-4.82 (m, 1.5H, $NC\underline{H}_2C\underline{H}$), 4.70-4.58 (m, 0.5H, $NCH_2C\underline{H}$), 4.27-4.16 (m, 0.5H, $NC\underline{H}_2CH$), 4.11-3.99 (m, 0.5H, $NC\underline{H}_2CH$), 3.85-3.73 (m, 0.5H, $NC\underline{H}_2CH_2$), 3.47-3.35 (m, 0.5H, $NC\underline{H}_2CH_2$), 3.26-3.14 (m, 0.5H, $NC\underline{H}_2CH_2$), 2.94-2.80 (m, 0.5H, $NC\underline{H}_2CH_2$), 2.49-2.34 (m, 1H, $NCH_2CHC\underline{H}_2$), 2.31-2.21 (m, 1H, $NCH_2CHC\underline{H}_2$), 2.07-1.94 (m, 1H, $NCH_2C\underline{H}_2$), 1.88 (s, 1H, $NH_2$), 1.78-1.65 (m, 1H, $NCH_2C\underline{H}_2$); HRMS (ESI): m/z $[M+H]^+$. Calcd for $C_{33}H_{30}N_7O_4$: 588.2359; found: 588.2359.

Example 25

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-methoxybenzenesulfonamide (I-25)

Synthesis of 4-methoxy-*N*-(7-(4,4,5,5-tetramethyl-1,3,2-dioxaborolan-2-)benzo[*d*][1,3]dioxo heterocyclopenten-4-) benzene sulfonamide(IV-25)

Compound XI (1.00 g, 3.80 mmol), pyridine (1.50 g, 19.00 mmol) and dichloromethane (20 mL) were mixed, and 4-methoxybenzenesulfonyl chloride (0.94 g, 4.56 mmol) in dichloromethane solution was added dropwise. Next, the mixture was reacted at room temperature for 8.5 h. The post-processing method was the same as that of compound IV-1 to obtain 985 mg of a light-yellow solid with a yield of 59.7%, m.p. at 186-188°C. $^1$H-NMR(300 MHz, $CDCl_3$) $\delta$(ppm): 7.74(d, *J*=8.67 Hz, 2H, ArH), 7.15(d, *J*=8.37 Hz, 1H, ArH), 7.02(d, *J*=8.28 Hz, 1H, ArH), 6.88(d, *J*=8.55 Hz, 2H, ArH), 6.56(s, 1H, NH), 5.88(s, 2H, $OCH_2O$), 3.83(s, 3H, $OCH_3$), 1.33(s, 12H, $4CH_3$).

Synthesis of (*R*)-3-(4-amino-3-(7-(4-methoxybenzenesulfonamide)benzo [*d*][1,3]dioxo heterocyclopenten-4)-1*H*-pyrazolo[3,4-*d*]pyrimidin-1-yl)piperidine-1-tert-butyl formate (V-25)

Compounds III-1 (1.00 g, 1.04 mmol) and IV-25 (0.54 g, 1.24 mmol) were used as raw materials, the operation process was the same as that of compound V-1 to obtain 592 mg of a light-yellow solid with a yield of 91.5%, m.p. at 182-184°C. $^1$H-NMR (300 MHz, $CDCl_3$) $\delta$ (ppm): 8.34 (s, 1H, ArH), 7.80 (d, *J* = 8.85 Hz, 2H, ArH), 7.26 (s, 1H, NH), 7.19 (d, *J* = 8.70 Hz, 1H, ArH), 7.03 (d, *J* = 8.70 Hz, 1H, ArH), 6.94 (d, *J*= 8.88 Hz, 2H, ArH), 5.95 (s, 2H, $OCH_2O$), 5.69 (s, 2H, $NH_2$), 4.89-4.77 (m, 1H, $CHC\underline{H_2}NBoc$), 4.37-4.23 (m, 1H, $C\underline{H}CH_2NBoc$), 4.19-4.08 (m, 1H, $CHC\underline{H_2}NBoc$), 3.86 (s, 3H, OMe), 3.47-3.33 (m, 1H, $NC\underline{H_2}CH_2$), 2.89-2.77 (m, 1H, $NC\underline{H_2}CH_2$), 2.27-2.11 (m, 2H, $NCH_2CHC\underline{H_2}$), 1.94-1.81 (m, 2H, $NCH_2C\underline{H_2}$), 1.44 (s, 9H, NBoc).

Synthesis of (*R*)-*N*-(7-(4-amino-1-(piperidin-3)-1*H*-pyrazolo[3,4-*d*]pyrimidin-3-)benzo[*d*][1,3]dioxo heterocyclopenten-4)-4-methoxybenzenesulfonamide (VI-25)

Compound V-25 (500 mg, 0.80 mmol) was used as the raw material, the operation process was the same as that of compound VI-1 to obtain 741 mg of a light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-methoxybenzenesulfonamide(I-25)

Compound VI-25 (500 mg, 0.96 mmol) and acrylic acid (91 mg, 1.00 mmol) were used as raw materials, the procedure was the same as that of compound I-4 to obtain 287 mg of a white solid with a yield of 52.0%, m.p. at 157.5-159.0°C. $^1$H-NMR (300 MHz, $CDCl_3$) $\delta$ (ppm): 8.32 (s, 1H, ArH), 7.80 (d, *J*= 8.52 Hz, 2H, ArH), 7.18 (d, *J* = 8.88 Hz, 1H, ArH), 7.02 (d, *J*= 8.61 Hz, 1H, ArH), 6.94 (d, *J* = 8.67 Hz, 2H, ArH), 6.68-6.49 (m, 1H, $CH=CH_2$), 6.34-6.21 (m, 1H, $CH=CH_2$), 5.95 (s, 2H, $OCH_2O$), 5.86 (s, 1H, $NH_2$), 5.75-5.60 (m, 1H, $CH=CH_2$), 4.96-4.77 (m, 1.5H, $NC\underline{H_2}C\underline{H}$), 4.66-4.53 (m, 0.5H, $NCH_2C\underline{H}$), 4.23-4.12 (m, 0.5H, $NC\underline{H_2}CH$), 4.08-3.97 (m, 0.5H, $NC\underline{H_2}CH$), 3.86 (s, 3H, $OCH_3$), 3.53-3.43 (m, 0.5H, $NC\underline{H_2}CH_2$), 3.39-3.27 (m, 0.5H, $NC\underline{H_2}CH_2$), 3.24-3.10 (m, 0.5H, $NC\underline{H_2}CH_2$), 2.97-2.78 (m, 0.5H, $NC\underline{H_2}CH_2$), 2.46-2.19 (m, 2H, $NCH_2CHC\underline{H_2}$, $NH_2$), 2.11-1.91 (m, 2H, $NCH_2CHC\underline{H_2}C\underline{H_2}$), 1.81-1.64 (m, 1H, $NCH_2CHCH_2C\underline{H_2}$); HRMS (ESI): m/z $[M+H]^+$. Calcd for $C_{27}H_{28}N_7O_6S$: 578.1822; found: 578.1819.

Example 26

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-2-naphthalenesulfonamide (I-26)

Synthesis of *N*-(7-(4,4,5,5-tetramethyl-1,3,2-dioxaborolan-2-yl)benzo[*d*][1,3]dioxo heterocyclopenten-4)naphthalene-2-sulfonamide (IV-26)

Compound XI (1.00 g, 3.80 mmol) and 2-naphthalenesulfonyl chloride (1.29 g, 5.70 mmol) were used as raw materials, the operation process was the same as that of compound IV-25 to obtain 1.02 g of a white solid with a yield of 59.2%, m.p. at 180-182°C. $^1$H-NMR (300 MHz, $CDCl_3$) $\delta$ (ppm): 8.41 (s, 1H, ArH), 7.94-7.84 (m, 3H, ArH), 7.81 (d, *J*= 8.76 Hz, 1H, ArH), 7.66-7.54 (m, 2H, ArH), 7.16-7.07 (m, 2H, ArH), 6.76 (s, 1H, NHCO), 5.80 (s, 2H, $OCH_2O$), 1.30 (s, 12H, $4CH_3$).

Synthesis of (R)-3-(4-amino-3-(7-(2-naphthalenesulfonamido)benzo[*d*][1,3]dioxo heterocyclopenten-4)-1*H*-tert-butyl-pyrazolo[3,4-*d*]pyrimidin-1-)piperidine-1-tert-butyl formate (V-26)

Compounds III-1 (0.80 g, 1.80 mmol) and IV-26 (0.98 g, 2.16 mmol) were used as raw materials, the operation process was the same as that of compound V-1 to obtain 718 mg of a light-yellow solid with a yield of 61.9 %, m.p. at 172-174°C. $^1$H-NMR (300 MHz, $CDCl_3$) $\delta$(ppm): 8.44 (s, 1H, ArH), 8.32(s, 1H, ArH), 7.99-7.80(m, 4H, ArH), 7.70-7.57 (m, 2H, ArH), 7.50(s, 1H, NH), 7.30-7.23 (m, 1H, ArH), 7.03 (d, *J*=8.46Hz, 1H, ArH), 5.85 (s, 2H, $OCH_2O$), 5.66 (s, 2H, $NH_2$), 4.88-4.73 (m, 1H, $CHCH_2NBoc$), 4.36-4.19 (m, 1H, $CHCH_2NBoc$), 4.17-4.03 (m, 1H, $CHCH_2NBoc$), 3.43-3.28 (m, 1H, $NCH_2CH_2$), 2.88-2.73 (m, 1H, $NCH_2CH_2$), 2.25-2.08 (m, 2H, $NCH_2CHCH_2$), 1.91-1.79 (m, 1H, $NCH_2CH_2$), 1.73-1.60 (m, 1H, $NCH_2CH_2$), 1.42 (s, 9H, NBoc).

Synthesis of (*R*)-*N*-(7-(4-amino-1-(piperidin-3)-1*H*-pyrazolo[3,4-d]pyrimidin-3)benzo[*d*][1,3]dioxo heterocyclopenten-4)-2-naphthalenesulfonamide(VI-26)

Compound V-26 (907 mg, 1.41 mmol) was used as the raw material, the operation process was the same as that of compound VI-1 to obtain 588 mg of a light-yellow solid with a yield of 76.7%, which was directly used in the next step without further purification.

Synthesis of (*R*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-2-naphthalenesulfonamide (I-26)

Compound VI-26 (500 mg, 0.92 mmol) and acryloyl chloride (87 mg, 0.80 mmol) were used as raw materials, the procedure was the same as that of target compound I-1 to obtain 134 mg of a white solid with a yield of 24.3%, m.p. at 166.5-167°C. $^1$H-NMR (300 MHz, $CDCl_3$) $\delta$ (ppm): 8.44(s, 1H, ArH), 8.30(d, *J*=8.85 Hz, 1H, ArH), 8.02-7.80(m, 4H, ArH), 7.71-7.54(m, 2H, ArH), 7.21(d, *J*=8.13 Hz, 1H, ArH), 7.00(d, *J*=7.95 Hz, 1H, ArH), 6.70-6.46(m, 1H, $CH=CH_2$), 6.38-6.19(m, 1H, $CH=CH_2$), 6.03-5.77(m, 3H, $NH_2$, $OCH_2O$), 5.75-5.59(m, 1H, $CH=CH_2$), 4.92-4.74(m, 1.5H, $NCH_2CH$), 4.67-4.52(m, 0.5H, $NCH_2CH$), 4.23-4.09(m, 0.5H, $NCH_2CH$), 4.08-3.95(m, 0.5H, $NCH_2CH$), 3.78-3.63(m, 0.5H, $NCH_2CH_2$), 3.38-3.23(m, 0.5H, $NCH_2CH_2$), 3.21-3.09 (m, 0.5H, $NCH_2CH_2$), 2.91-2.76(m, 0.5H, $NCH_2CH_2$), 2.45-2.08(m, 3H, $NCH_2CHCH_2$, $NH_2$), 2.03-1.88(m, 1H, $NCH_2CH_2$), 1.78-1.52(m, 1H, $NCH_2CH_2$); HRMS (ESI): m/z $[M+H]^+$. Calcd for $C_{30}H_{28}N_7O_5S$: 598.1873; found: 598.1873.

Example 27

Synthesis of (*R*)-*N*-(7-(4-amino-1-(1-(2-cyano-3-cyclopropylacryloyl)piperidin-3-)-1*H*-pyrazolo[3,4-*d*]pyrimidin-3-)benzo[*d*][1,3]dioxo heterocyclopenten-4-)benzamide (I-27)

Synthesis of (*R*)-*N*-(7-(4-amino-1-(1-(2-cyanoacetyl)piperidin-3)-1*H*-pyrazolo[3,4-*d*]pyrimidine-3-)benzo[*d*][1,3] dioxo heterocyclopenten-4-)benzamide (XII-27)

Compound VI-1 (300 mg, 0.66 mmol), cyanoacetic acid (67 mg, 0.79 mmol), HOBt (133 mg, 0.98 mmol), EDCI (189 mg, 0.98 mmol) and TEA (133 mg, 1.31 mmol) were dissolved in 6 mL DMF, and stirred at a room temperature for 12 h. The post-processing method was the same as that of compound I-4 to obtain 210 mg of a white solid with a yield of 61.1%, m.p. at 167.5-169.5°C. $^1$H-NMR (300 MHz, DMSO-$d_6$) $\delta$ (ppm): 10.25 (s, 1H, NH), 8.24 (d, *J* = 6.09 Hz, 1H, ArH), 7.99(d, *J*=7.95 Hz, 2H, ArH), 7.68-7.49(m, 3H, ArH), 7.20 (d, *J*=7.95 Hz, 1H, ArH), 7.10-6.99(m, 1H, ArH), 6.15(s, 2H, $OCH_2O$), 4.92-4.80(m, 0.5H, $NCH_2CH$), 4.77-4.64(m, 0.5H, $NCH_2CH$), 4.48-4.38(m, 0.5H, $NCH_2CH$), 4.16-4.06(m, 2H, $CNCH_2CO$), 4.05-3.99(m, 0.5H, $NCH_2CH$), 3.97-3.85(m, 0.5H, $NCH_2CH$), 3.80-3.64(m, 1H, $NCH_2CH$, $NCH_2CH_2$), 3.27-3.13(m, 1H, $NCH_2CH_2$), 3.06-2.95 (m, 0.5H, $NCH_2CH_2$), 2.25-2.03(m, 2H, $NCH_2CHCH_2$), 1.94-1.54(m, 2H, $NCH_2CH_2$).

Synthesis of (*R*)-*N*-(7-(4-amino-1-(1-(2-cyano-3-cyclopropylacryloyl)piperidin-3-)-1*H*-pyrazolo[3,4-*d*]pyrimidin-3-)benzo[*d*][1,3]dioxo heterocyclopenten-4-)benzamide (I-27)

XII-27 (100 mg, 0.19 mmol), methanol (3 mL), piperidine (24 mg, 0.29 mmol) and cyclopropanaldehyde (20 mg, 0.29 mmol) were mixed and conducted reaction at a room temperature for 2 h. The mixture was concentrated under reduced pressure, dissolved in ethyl acetate, washed with water (10 mL×3) and saturated sodium chloride (10 mL×3) successively, and concentrated under reduced pressure to obtain a crude product, which was subjected to column chromatography (eluent: dichloromethane: methanol=100-60:1) to obtain 68 mg of a white solid with a yield of 61.8%, m.p. at 155.5-157°C. $^1$H-NMR (300 MHz, $CDCl_3$) $\delta$ (ppm): 8.37 (s, 1H, ArH), 8.07 (s, 1H, NH), 8.03-7.92 (m, 3H, ArH), 7.67-7.50

(m, 3H, ArH), 7.16 (d, *J*= 8.61 Hz, 1H, ArH), 6.70-6.49 (m, 1H, $CH=CH_2$), 6.15 (s, 2H, $OCH_2O$), 5.97 (s, 2H, $NH_2$), 5.04-4.89 (m, 1H, $NCH_2CH$), 4.69-4.47 (m, 0.5H, $NCH_2CH$), 4.37-4.18 (m, 1H, $NCH_2CH$), 3.74-3.48 (m, 0.5H, $NCH_2CH$), 3.41-3.06 (m, 1H, $NCH_2CH_2$), 2.52-2.38 (m, 1H, $NCH_2CH_2$), 2.35-2.24 (m, 1H, $CHCH_2CH_2$), 2.23-2.12 (m, 2H, $CHCH_2CH_2$), 2.11-1.99 (m, 2H, $CHCH_2CH_2$, C=CCH), 1.93-1.74 (m, 1H, $C=CCHCH_2CH_2$), 1.39-1.13 (m, 3H, $C=CCHCH_2CH_2$); HRMS (ESI): m/z $[M+H]^+$. Calcd for $C_{31}H_{29}N_8O_4$: 577.2312; found: 577.2318.

Example 28

Synthesis of (*S*)-*N*-(7-(1-(1-acryloylpiperidin-3)-4-amino-1*H*-pyrazolo[3,4-*d*]pyrimidin-3-)benzo[*d*][1,3]dioxo heterocyclopenten-4)-4-(dimethylamino)benzamide (I-28)

Compound VIII-1 was replaced with (R)-1-tert-butoxycarbonyl-3-hydroxypiperidine (VIII-3), and the experimental operation refers to the preparations of compound I-1 or I-13 to obtain a white solid I-28, m.p. at 160-162°C. $^1$H-NMR (300 MHz, $CDCl_3$) $\delta$ (ppm): 8.32 (s, 1H, ArH), 8.00 (d, *J* = 8.88 Hz, 1H, ArH), 7.86 (s, 1H, NH), 7.81 (d, *J*= 8.04 Hz, 2H, ArH), 7.10 (d, *J*= 8.73 Hz, 1H, ArH), 6.71 (d, *J* = 8.49 Hz, 2H, ArH), 6.66-6.52 (m, 1H, $CH=CH_2$), 6.35-6.22 (m, 2H, $CH=CH_2$, $NH_2$), 6.11 (s, 2H, $OCH_2O$), 5.75-5.61 (m, 1H, $CH=CH_2$), 4.97-4.80 (m, 1.5H, $NCH_2CH$), 4.70-4.54 (m, 0.5H, $NCH_2CH$), 4.30-4.14 (m, 0.5H, $NCH_2CH$), 4.09-3.95 (m, 0.5H, $NCH_2CH$), 3.86-3.69 (m, 0.5H, $NCH_2CH_2$), 3.46-3.31 (m, 0.5H, $NCH_2CH_2$), 3.25-3.13 (m, 0.5H, $NCH_2CH_2$), 3.06 (s, 6H, $N(CH_3)_2$), 2.92-2.79 (m, 1.5H, $NCH_2CH_2$, $NH_2$), 2.47-2.35 (m, 1H, $NCH_2CHCH_2$), 2.30-2.17 (m, 1H, $NCH_2CHCH_2$), 2.08-1.91 (m, 1H, $NCH_2CH_2$), 1.82-1.64 (m, 1H, $NCH_2CH_2$); HRMS (ESI): m/z $[M+H]^+$. Calcd for $C_{29}H_{31}N_8O_4$: 555.2468; found: 555.2471.

Example 29

Synthesis of (S)-N-(7-(1-((1-acryloylpyrrolidin-2-yl)methyl)-4-amino-1*H*-pyrazolo[3,4-*d*]pyrimidin-3-yl )Benzo[ *d*][1,3]dioxol-4-yl)-4-dimethylaminobenzamide (I-29)

Synthesis of (S)-2-((4-amino-3-iodo-1H-pyrazolo[3,4-*d*]pyrimidin-1-yl)methyl)pyrrolidine-1-carboxylic acid tert-butyl ester (III-5 )

Compound VII-1 (1.50 g, 5.75 mmol), Boc-*L*-prolinol (VIII-4) (2.31 g, 11.49 mmol), $PPh_3$ (3.01 g, 11.49 mmol) and anhydrous THF (100 mL) were mixed under nitrogen protection, and stirred for 30 min, under an ice bath, DIAD (2.32 g, 11.49 mmol) was added, after the addition was completed, reacted at a room temperature for 18 h. The post-processing method was the same as that of compound III-1 to obtain 1.48 g of a light-yellow solid with a yield of 58.0%, m.p. at 176-178°C. $^1$H-NMR (300 MHz, $CDCl_3$) $\delta$ (ppm): 8.36 (s, 1H, ArH), 6.24 (s, 2H, $NH_2$), 4.65-4.27 (m, 3H, $NCH_2CHNBoc$), 3.51-3.26 (m, 2H, $CH_2CH_2CH_2NBoc$), 2.00-1.77 (m, 4H, $CH_2CH_2CH_2NBoc$), 1.48 (s, 9H, $3CH_3$).

Synthesis of (S)-2-((4-amino-3-(7-(4-(dimethylamino)benzamido)benzo[*d*][1,3]dioxole-4-Base)-1*H*-pyrazolo[3,4-*d*]pyrimidin-1-yl)methyl)pyrrolidine-1-carboxylic acid tert-butyl ester (V-29)

Compounds III-5 (0.80 g, 1.80 mmol) and IV-13 (0.89 g, 2.16 mmol) were used as raw materials, the operation process was the same as that of compound V-1 to obtain 820 mg of a light-yellow solid with a yield of 75.8%, m.p. at 138-140°C. $^1$H-NMR (300 MHz, $CDCl_3$) $\delta$ (ppm): 8.38 (s, 1H, ArH), 8.06 (d, *J*= 8.64 Hz, 1H, ArH), 7.91-7.82 (m, 3H, ArH, NHCO), 7.17 (d, *J*= 8.73 Hz, 1H, ArH), 6.76 (d, *J* = 8.70 Hz, 2H, ArH), 6.15 (s, 2H, $OCH_2O$), 5.95 (br, 2H, $NH_2$), 4.72-4.59 (m, 1H, $NCH_2CHNBoc$), 4.46-4.35 (m, 1H, $NCH_2CHNBoc$), 3.50-3.38 (m, 1H, $NCH_2CHNBoc$), 3.35-3.24 (m, 1H, $CH_2CH_2CH_2NBoc$), 3.11 (s, 6H, $N(CH_3)_2$), 3.07-3.03 (m, 1H, $CH_2CH_2CH_2NBoc$), 2.01-1.78 (m, 4H, $CH_2CH_2CH_2NBoc$), 1.48 (s, 9H, $3CH_3$).

Synthesis of (*S*)-*N*-(7-(4-amino-1-(pyrrolidin-2-ylmethyl)-1*H*-pyrazolo[3,4-*d*]pyrimidin-3-yl)benzo[*d*] [1,3]dioxol-4-yl)-4-dimethylaminobenzamide (VI-29)

Compound V-29 (650 mg, 1.08 mmol) was used as the raw material, the operation process was the same as that of compound VI-1 to obtain 443 mg of a light-yellow solid with a yield of 81.8%, which was directly used in the next step without further purification.

Synthesis of (*S*)-*N*-(7-(1-((1-acryloylpyrrolidin-2-yl)methyl)-4-amino-1*H*-pyrazolo[3,4-*d*]pyrimidin-3-yl)Benzo[*d*] [1,3]dioxol-4-yl)-4-dimethylaminobenzamide (I-29)

Compound VI-29 (360 mg, 0.72 mmol) and acryloyl chloride (68 mg, 0.76 mmol) were used as raw materials, the

procedure was the same as that of compound I-1 to obtain 168 mg of a white solid with a yield of 42.1%, m.p. at 98-100°C. $^1$H-NMR (300 MHz, $CDCl_3$+$D_2O$) $\delta$ (ppm): 8.28 (d, *J* = 8.19 Hz, 1H, ArH), 7.89-7.72 (m, 3H, ArH), 7.01 (d, *J* = 8.52 Hz, 1H, ArH), 6.64 (d, *J* = 8.52 Hz, 2H, ArH), 6.42-6.31 (m, 1H, CH=$CH_2$), 6.28-6.16 (m, 1H, CH=$CH_2$), 6.09-5.97 (m, 2H, $OCH_2O$), 5.71-5.45 (m, 1H, CH=$CH_2$), 4.85-4.26 (m, 5H, $NC\underline{H_2}C\underline{H}N$, $NH_2$), 3.70-3.52 (m, 1H, $NC\underline{H_2}CH_2$), 3.48-3.37 (m, 1H, $NC\underline{H_2}CH_2$), 3.00 (s, 6H, 2$CH_3$), 2.11-1.53 (m, 4H, $NCH_2C\underline{H_2}C\underline{H_2}$); HRMS (ESI): m/z [M+H]$^+$. Calcd for $C_{29}H_{31}N_8O_4$: 555.2468; found: 555.2471.

Example 30

Synthesis of (*R*)-*N*-(7-(1-((1-acryloylpyrrolidin-2-yl)methyl)-4-amino-1*H*-pyrazolo[3,4-*d*]pyrimidin-3-yl )Benzo[ *d*][1,3]dioxol-4-yl)-4-dimethylaminobenzamide (I-30)

Compound VIII-4 was replaced with Boc-D-prolinol (VIII-5), and the experimental operation refers to the preparations of compound I-29 to obtain 157 mg of a white solid with a yield of 30.7%, m.p. at 98-100°C. $^1$H-NMR (300 MHz, $CDCl_3$) $\delta$ (ppm): 8.37 (d, *J*= 7.65 Hz, 1H, ArH), 8.05-7.94 (m, 2H, ArH, NHCO), 7.85 (d, *J*= 8.70 Hz, 2H, ArH), 7.11 (d, *J*= 8.61 Hz, 1H, ArH), 6.74 (d, *J* = 8.79 Hz, 2H, ArH), 6.48-6.41 (m, 1H, CH=$CH_2$), 6.38-6.27 (m, 1H, CH=$CH_2$), 6.18-6.10 (m, 3H, $NH_2$, $OCH_2O$), 5.76-5.58 (m, 1H, CH=$CH_2$), 4.81-4.36 (m, 3H, $NC\underline{H_2}C\underline{H}N$), 3.82-3.61 (m, 1H, $NC\underline{H_2}CH_2$), 3.56-3.45 (m, 1H, $NC\underline{H_2}CH_2$), 3.09 (s, 6H, 2$CH_3$), 2.73 (s, 1H, $NH_2$), 2.19-1.79 (m, 4H, $NCH_2C\underline{H_2}C\underline{H_2}$); HRMS (ESI): m/z [M+H]$^+$. Calcd for $C_{20}H_{31}N_8O_4$: 555.2468; found: 555.2470.

Example 31

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4-)-5-fluorobenzofuran-2-carboxamide (I-31)

Synthesis of 4-methoxy-*N*-(7-(4,4,5,5-tetramethyl-1,3,2-dioxaborolan-2-)benzo[*d*][1,3]dioxo heterocyclopenten-4-)5-fluorobenzo[*b*]furan-2-carboxamide(IV-31)

XI (321m g, 0.12 mmol) and 5-Fluorobenzo[*b*]furan-2-carboxylic acid were used as the raw materials, the operation process was the same as that of compound IV-2 to obtain 407 mg of a yellow solid with a yield of 87.01%, m.p. at 92.0-94.0°C. $^1$H-NMR (300 MHz, $CDCl_3$) $\delta$ (ppm): 8.39 (s , 1H, NHCO), 7.94 (d, *J*=8.49 Hz, 1H, ArH), 7.62 (s, 1H, ArH), 7.56 (dd, *J*=9.06 Hz, 1H, ArH), 7.41 (dd, *J*=8.16 Hz ,1H, ArH), 7.34 (d, *J*=8.49Hz, 1H, ArH), 7.24 (td, *J*= 9.00Hz, 1H, ArH), 6.18 (s, 2H, $OCH_2O$), 1.42 (s, 12H, 4$CH_3$).

Synthesis of (*R*)-3-(4-amino-3-(7-(5-fluorobenzo[b]furan-2-carboxamido)benzo[d][1,3]dioxo heterocyclopenten-4)-1*H*-pyrazolo[3,4-*d*]pyrimidin-1-)piperidine-1-tert-butyl formate (V-31)

Compounds III-1 (345 mg, 1.78mmol) and IV-31 (300 mg, 0.7 mmol) were used as raw materials, the operation process was the same as that of compound V-1 to obtain 314 mg of a light-yellow solid with a yield of 72.86%, m.p. at 204-206°C. $^1$H-NMR (300 MHz, $CDCl_3$) $\delta$ (ppm): 8.40 (s , 1H, NHCO), 8.39 (s , 1H,ArH), 8.05 (d, *J*=8.7 Hz, 1H, ArH), 7.63(s, 1H, ArH), 7.56 (dd, *J*=9.56 Hz, 1H, ArH), 7.40(dd, *J*=8.13 Hz ,1H, ArH), 7.23 (td, *J*=9.00Hz, 1H, ArH), 7.17 (d, *J*=8.73Hz, 1H, ArH), 6.20 (s, 2H, $OCH_2O$), 5.76 (s, 2H, $NH_2$), 4.98-4.80 (m, 1H, $CHC\underline{H_2}NBoc$), 4.50-4.25 (m, 1H, $C\underline{H}CH_2NBoc$), 4.25-4.10 (m, 1H, $CHC\underline{H_2}NBoc$), 3.50-3.32 (m, 1H, $NC\underline{H_2}CH_2$), 2.95-2.80 (m, 1H, $NC\underline{H_2}CH_2$), 2.38-2.26 (m, 1H, $NCH_2CHC\underline{H_2}$), 2.25-2.17 (m, 1.0H, $NCH_2CHC\underline{H_2}$, $NCH_2C\underline{H_2}$), 1.81-1.72 (m, 1.0H, $NCH_2C\underline{H_2}$), 1.47 (s, 9H, NBoc).

Synthesis of (*R*)-*N*-(7-(4-amino-1-(piperidin-3)-1H-pyrazolo[3,4-*d*]pyrimidin-3-)benzo[*d*][1,3]dioxo heterocyclopenten-4-)-5-fluorobenzo[*b*]furan-2-carboxamide (VI-31)

Compound V-31 (300 mg, 0.49 mmol) was used as the raw material, the operation process was the same as that of compound VI-1 to obtain 206 mg of a light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4)-5-fluorobenzo[*b*]furan-2-carboxamide(I-31)

Compound *VI*-31 (200 mg, 0.39 mmol) and acryloyl chloride (37 mg, 0.4 mmol) were used as raw materials, the procedure was the same as that of compound I-1 to obtain 72 mg of a white solid with a yield of 32.3%, m.p. at 184.0-186.0°C. $^1$H-NMR (300 MHz, $CDCl_3$) $\delta$ (ppm): 10.55 (s, 1H, NHCO), 8.26 (s, 1H, ArH), 7.84 (s, 1H, ArH), 7.80 (q, *J* =

9.27 Hz, 1H, ArH), 7.69 (q, *J* = 8.64 Hz, 1H, ArH), 7.40 (td, *J*=9.21 Hz, 1H, ArH), 7.25 (d, *J*=8.64Hz, 1H, ArH), 7.08 (d, *J*=8.49Hz, 1H, ArH), 6.95-6.70 (m, 1H, C$\underline{H}$=$CH_2$), 6.20 (s, 2H, $OCH_2O$), 6.15-6.04 (m, 1H, CH=C$\underline{H_2}$), 5.86 (s, 2H, $NH_2$), 5.78-5.60 (m, 1H, CH=C$\underline{H_2}$), 4.79-4.65 (m, 1H, CHC$\underline{H_2}$NBoc), 4.63-4.51 (m, 0.5H, C$\underline{H}$C$H_2$NBoc), 4.35-4.20 (m, 1H, CHC$\underline{H_2}$NBoc), 4.18-4.04 (m, 1H, NC$\underline{H_2}$CH), 3.81-3.65 (m, 0.5H, C$\underline{H}$C$H_2$NBoc), 3.29-3.22 (m, 1H, NC$\underline{H_2}$C$H_2$), 2.37-2.23 (m, 1 H, N$CH_2$CHC$\underline{H_2}$), 2.20-2.10 (m, 1H, N$CH_2$CHC$\underline{H_2}$), 2.00-1.90 (m, 1H, N$CH_2$C$\underline{H_2}$), 1.72-1.53 (m, 1H, N$CH_2$C$\underline{H_2}$).

Example 32

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4-)-benzo[b]thiophene-2-carboxamide (I-32)

Synthesis of 4-methoxy-*N*-(7-(4,4,5,5-tetramethyl-1,3,2-dioxaborolane-2)benzo[*d*][1,3]dioxo heterocyclopenten-4-)-benzo[b]thiophene-2-carboxamide (IV-32)

XI (2.28 g, 0.018mol) and benzo[*b*]thiophene-2-formic acid (1.63g, 0.0091 mol) were used as raw materials, the operation process was the same as that of compound IV-2 to obtain 2.78 g of a yellow solid with a yield of 72.09%, m.p. at 124-125°C. 1H-NMR (300 MHz, CDCl3): δ(ppm):10.56 (s, 1H, NHCO), 8.39 (s, 1H, ArH), 8.08 (d, *J*=7.08Hz, 1H, ArH), 8.03 (d, 1H, *J*=6.66Hz), 7.58-7.46 (m, 2H, ArH), 7.09 (m, 2H, ArH), 6.12 (s, 2H, $OCH_2O$), 1.32 (s, 12H, $CH_3$). Synthesis of (*R*)-3-(4-amino-3-(7-benzo[b]thiophene-2-carboxamide)benzo[d][1,3]dioxo heterocyclopenten-4)-1*H*-tert-butyl-pyrazolo[3,4-d]pyrimidin-1-)piperidine-1-tert-butyl formate (V-32)

Compounds III-1 (1.4 g, 3.1 mmol) and IV-32 (1.2 g, 2.8 mmol) were used as raw materials, the operation process was the same as that of compound V-1 to obtain 834 mg of a light-yellow solid with a yield of 48.53%, m.p. at 126.0-128.0°C. $^1$H-NMR (300MHz, CDCl3), δ(ppm): 8.42 (s, 1H, NHCO), 8.03 (d, 1H, *J*=8.82Hz, ArH), 8.00 (s, 1H, ArH), 7.99-7.92 (m, 3H, ArH), 7.58-7.44 (m, 2H, ArH), 7.20 (d, 1H, *J*=8.70Hz, ArH), 6.20 (s, 2H, $OCH_2O$), 5.80 (s, 2H, $NH_2$), 4.90 (m, 1H, CHC$\underline{H_2}$NBoc), 4.52-4.28 (m, 1H, C$\underline{H}$C$H_2$NBoc), 4.27-4.13 (m, 1H, CHC$\underline{H_2}$NBoc), 3.62-3.33 (m, 1H, NC$\underline{H_2}$C$H_2$), 2.88 (m, 1H, NC$\underline{H_2}$C$H_2$), 2.40-2.28 (m, 1H, N$CH_2$CHC$\underline{H_2}$), 2.28-2.22 (m, 1H, N$CH_2$CHC$\underline{H_2}$), 1.83-1.74 (m, 1H, N$CH_2$C$\underline{H_2}$), 1.74-1.65 (1H, N$CH_2$C$\underline{H_2}$), 1.51 (s, 9H, $CH_3$).

Synthesis of (R)-N-(7-(4-amino-1-(piperidin-3)-1H-pyrazolo[3,4-*d*]pyrimidin-3)benzo[*d*][1,3]dioxo heterocyclopenten-4)-benzo[b]thiophene-2-carboxamide(VI-32)

Compound V-32 (0.8 mg, 1.3 mmol) was used as the raw material, the operation process was the same as that of compound VI-1 to obtain 301 mg of a light-yellow solid, which was directly used in the next step without further purification.

Synthesis of (*R*)-*N*-(7-(1-(1-acryloylpiperidin-3)-4-amino-1*H*-pyrazolo[3,4-*d*]pyrimidin-3-yl)benzo[*d*][1,3]dioxo heterocyclopenten-4)-benzo[b]thiophene-2-carboxamide (I-32)

Compound VI-32 (300 mg, 0.58 mmol) and acryloyl chloride (63 mg, 0.696 mmol) were used as raw materials, the procedure was the same as that of compound I-4 to obtain 79 mg of a white solid with a yield of 24.01%, m.p. at 162.0-164.0°C.

$^1$H-NMR (300MHz, CDCl3): δ(ppm): $^1$H-NMR (300MHz, CDCl3), δ (ppm): 8.42 (s, 1H, NHCO), 8.03 (d, 1H, *J*=8.91Hz, ArH), 8.01 (s, 1H, ArH), 7.98 (d, 2H, *J*=3.57Hz, ArH), 7.95 (s, 1H, ArH), 7.52 (m, 2H, ArH), 7.19 (d, *J*=8.64Hz,1H, ArH), 6.75-6.60 (m, 1H, C$\underline{H}$=$CH_2$), 6.42-6.30 (m, 1H, CH=C$\underline{H_2}$), 6.21 (s, 2H, $OCH_2O$), 5.85-5.7 (m, 3H, CH=C$\underline{H_2}$, $NH_2$), 5.01-4.87 (m, 1.5H, CHC$\underline{H_2}$NBoc, C$\underline{H}$C$H_2$NBoc), 4.76-4.63 (m, 0.5H, C$\underline{H}$C$H_2$NBoc), 4.32-4.22 (m, 0.5H, CHC$\underline{H_2}$NBoc), 4.15-4.04 (m, 0.5H, CHC$\underline{H_2}$NBoc), 3.89-3.77 (m, 0.5H, NC$\underline{H_2}$C$H_2$), 3.75-3.52 (m, 0.5H, NC$\underline{H_2}$C$H_2$), 3.49-3.37 (m, 0.5H, NC$\underline{H_2}$C$H_2$), 3.32-3.18 (m, 0.5H, NC$\underline{H_2}$C$H_2$), 2.99-2.83 (m, 1H, N$CH_2$CHC$\underline{H_2}$), 2.51-2.38 (m, 1H, N$CH_2$CHC$\underline{H_2}$), 2.37-2.21 (m, 1H, N$CH_2$C$\underline{H_2}$), 2.11-1.99 (m, 1H, N$CH_2$C$\underline{H_2}$).

Example 33

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4)-3-(4-methylpiperazin-1-yl)benzamide(I-33) (Reference Example)

Synthesis of 3-(4-methylpiperazin-1-yl)-N-(7-(4,4,5,5-tetramethyl-1,3,2-dioxaborolan-2-yl )Benzo[*d*][1,3]dioxol-4-yl)-benzamide (IV-33)

XI (1.43 g, 0.0055 mol) and 3-(4-methylpiperazin-1-)benzoic acid (1.00 g, 0.0045 mol) were used as raw materials, the operation process was the same as compound IV-2 to obtain 1.80 g of a white solid with a yield of 85.98%,

m.p. at 142.0-144.0°C. $^1$H-NMR (300MHz, DMSO): δ(ppm): 10.20 (s, 1H, NHCO), 8.41 (s, 1H, ArH), 7.67 (d, *J*=7.95Hz, 1H, ArH), 7.52 (d, *J*=8.46Hz, 1H, ArH), 7.36-7.30 (m, 1H, ArH), 7.24 (s, 1H, ArH), 7.12 (t, *J*=7.34Hz, 1H, ArH), 6.12 (s, 2H, $OCH_2O$), 3.99 (s, 3H, $NCH_3$), 1.32 (s, 12H, $CH_3$).

Synthesis of (*R*)-3-(4-amino-3-(7-(3-(4-methylpiperazin-1-yl)benzamido)benzo[*d*][1,3]dioxo heterocyclopenten-4)-1*H*-pyrazolo[3,4-*d*]pyrimidin-1-)piperidine-1-tert-butyl formate (V-33)

Compounds III-1 (0.178 mg, 4 mmol) and IV-33 (0.17 mg, 37 mmol) were used as raw materials, the operation process was the same as that of compound V-1 to obtain 98 mg of a light-yellow solid with a yield of 40.42%, m.p. at 182-184°C. $^1$H-NMR (300MHz, DMSO), δ(ppm): 9.56 (s, 1H, NHCO), 8.41 (s, 1H, ArH), 8.02 (d, *J*= 8.7Hz, 1H, ArH), 7.93 (s, 1H, ArH), 7.52-7.47 (m, 1H, ArH), 7.35 (s, 1H, ArH), 7.18 (d, *J*=8.64, 1H, ArH), 7.13-7.17 (m, 1H, ArH), 6.16 (s, 2H, $OCH_2O$), 5.78 (s, 2H, $NH_2$), 4.96-4.83 (m, 1H, $C\underline{H_2}CHNBoc$), 4.47-4.27 (m, 1H, $CH_2C\underline{H}NBoc$), 4.26-4.17 (m, 1H, $C\underline{H_2}CHNBoc$), 3.41-3.35 (m, 4H, $CH_2NCH_2$), 3.25-3.19 (m, 4H, $C\underline{H_2}N(CH_3)C\underline{H_2}$), 2.95-2.81 (m, 1H, $NC\underline{H_2}CH_2$ ), 2.49 (s, 3H, $NC\underline{H_3}$), 2.40-2.29 (m, 1H, $NC\underline{H_2}CH_2$), 2.28-2.19 (m, 1.5H, $NCH_2CHC\underline{H_2}$, $NCH_2C\underline{H_2}$), 1.79-1.69 (m, 1.5H, $NCH_2C\underline{H_2}$), 1.49 (s, 9H, Boc). Synthesis of (*R*)-*N*-(7-(4-amino-1-(piperidin-3)-1*H*-pyrazolo[3,4-*d*]pyrimidin-3-)benzo[*d*][1,3]dioxo heterocyclopenten-4-)-3-(4-methylpiperazin-1-yl)benzamide(VI-33)

Compound V-33 (98 mg, 17 mmol) was used as the raw material, the operation process was the same as that of compound VI-1 to obtain 80 mg of a light-yellow solid, which was directly used in the next step without further purification.

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4)-3-(4-methylpiperazin-1-yl)benzamide(I-33)

Compound VI-33 (80 mg, 0.14 mmol) and acryloyl chloride (15 mg, 0.16 mmol) were used as raw materials, the procedure was the same as that of compound I-4 to obtain 49 mg of a white solid with a yield of 55.85%, m.p. at 152.0-154.0°C. $^1$H-NMR (300MHz, DMSO), δ(ppm): 10.23 (s, 1H, NHCO), 8.41 (s, 1H, ArH), 8.04 (d, *J*=8.7Hz, 1H, ArH), 7.52-7.47(m, 1H, ArH), 7.33 (d, *J*=7.08Hz, 1H, ArH), 7.18 (d, 1H, *J*=8.7Hz, ArH), 7.16 (s, 1H, ArH), 7.12-7.07 (m, 1H, ArH), 6.71-6.58 (m, 1H, $C\underline{H}=CH_2$), 6.39-6.29 (m, 1H, $CH=C\underline{H_2}$), 6.16 (s, 2H, $OCH_2O$), 5.80-5.66 (m, 3H, $NH_2$, $CH=C\underline{H_2}$), 4.99-4.85 (m, 1.5H, $C\underline{H_2}CHNBoc$, $CH_2C\underline{H}NBoc$), 4.72-4.62 (m, 0.5H, $CH_2C\underline{H}NBoc$), 4.30-4.20 (m, 0.5H, $C\underline{H_2}CHNBoc$ ), 4.15-4.02 (m, 0.5H, $C\underline{H_2}CHNBoc$), 3.86-3.73 (m, 0.5H, $NC\underline{H_2}CH_2$), 3.41-3.36 (m, 4H, $CH_2NCH_2$), 3.25-3.19 (m, 4H, $CH_2N(CH_3)CH_2$), 2.97-2.84 (m, 0.5H, $NC\underline{H_2}CH_2$), 2.49 (s, 3H, $CH_3$), 2.45-2.36 (m, 1H, $NC\underline{H_2}CH_2$), 2.35-2.26 (m, 1H, $C\underline{H_2}CHNBoc$), 2.08-1.99 (m, 1H, $NC\underline{H_2}CHCH_2$), 1.79-1.70 (m, 2H, $NC\underline{H_2}CHCH_2$).

Example 34

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)- 3-(morpholinomethyl)benzamide (I-34) (Reference Example)

Synthesis of 3-(morpholinomethyl)-*N*-(7-(4,4,5,5-tetramethyl-1,3,2-dioxaborolan-2-yl)benzo[*d*][1,3]dioxo heterocyclopenten-4-)benzamide (IV-34)

Compound XI (0.65 g, 0.0025 mol) and 3-(morpholinomethyl)benzoic acid (0.5 g, 0.0023 mol) were used as raw materials, the operation process was the same as that of compound IV-2 to obtain 732 mg of a white solid with a yield of 73.32%, m.p. at 192-194°C. $^1$H-NMR (300MHz, DMSO): δ(ppm):10.21 (s, 1H, NHCO), 7.92 (s, 1H, ArH), 7.89 (d, J=8.31Hz, 1H, ArH), 7.56 (d, *J*=6.69Hz, 1H, ArH), 7.50 (t, *J*=7.77Hz, 1H, ArH), 7.09(d, *J*=7.86Hz, 1H, ArH), 7.04 (d, *J*=8.31Hz, 1H, ArH), 6.09 (s, 2H, $OCH_2O$), 5.73 (s, 2H, $NH_2$), 3.66-3.60 (m, 4H, $C\underline{H_2}NC\underline{H_2}$), 3.56 (s, 2H, $CH_2$), 2.46-2.36 (m, 4H, $C\underline{H_2}OC\underline{H_2}$), 1.32 (s, 12H, $4CH_3$).

Synthesis of (*R*)-3-(4-amino-3-(7-(3-(morpholinomethyl)benzamido) benzo[*d*][1,3]dioxo heterocyclopenten-4)-1*H*-pyrazolo[3,4-*d*]pyrimidin-1-)piperidine-1-tert-butyl carboxylate(V-34)

Compounds III-1 (0.77 mg, 0.0017 mol) and IV-34 (0.7 mg, 0.0016 mol) were used as raw materials, the operation process was the same as that of compound V-1 to obtain 546 mg of a light-yellow solid with a yield of 51.99%, m.p. at 182-184°C. $^1$H-NMR (300MHz, DMSO), δ(ppm): 10.28 (s, 1H, NHCO), 8.27 (s, 1H, ArH), 8.03-7.86 (m, 2H, ArH), 7.69-7.46 (m, 2H, ArH), 7.22 (d, *J*=7.95Hz, 1H, ArH), 7.07 (d, *J*=7.62Hz, 1H, ArH), 6.98-6.73 (m, 1H, $C\underline{H}=CH_2$), 6.28-6.02 (m, 3H, $OCH_2O$, $CH=C\underline{H_2}$), 5.75 (s, 2H, $NH_2$)5.85- 5.58(m, 1H, $CH=C\underline{H_2}$), 4.83-4.66 (m, 1H, $C\underline{H_2}CHNBoc$), 4.54-4.65 (m, 0.5H, $CH_2C\underline{H}NBoc$), 4.38-4.20 (m, 1H, $C\underline{H_2}CHNBoc$), 4.20-4.02 (m, 0.5H, $CH_2C\underline{H}NBoc$), 3.82-3.70 (m, 0.5H, $NC\underline{H_2}CH_2$), 3.71-3.53 (m, 4H, $CH_2NCH_2$), 3.59 (s, 2H, $CH_2$), 3.31-3.16 (m, 1H, $NC\underline{H_2}CH_2$), 3.09-2.92 (m, 0.5H, $NC\underline{H_2}CH_2$) 2.50-2.42 (m, 4H, $CH_2OCH_2$), 2.35-2.23 (m, 1H, $NCH_2CHC\underline{H_2}$) 2.22-2.19 (m, 1H, $NCH_2CHC\underline{H_2}$), 2.06-1.90 (m, 1H, $NCH_2C\underline{H_2}$),

1.75-1.54 (m, 1H, $NCH_2C\underline{H}_2$ ), 1.42 (s, 9H, Boc).

Synthesis of (*R*)-N-(7-(4-amino-1-(piperidin-3-yl)-1*H*-pyrazol[3,4-*d*]pyrimidin-3-yl)benzo[*d*][1,3] Dioxol-4-yl)-3-(morpholinemethyl)benzamide (VI-34)

Compound V-34 (0.54 mg, 082 mmol) was used as the raw material, the operation process was the same as that of compound VI-1 to obtain 472 mg of a light-yellow solid, which was directly used in the next step without further purification.

Synthesis of (*R*)-*N*-(7-(1-(1-acryloylpiperidin-3-yl)-4-amino-1*H*-pyrazolo[3,4-*d*]pyrimidin-3-)benzo[*d*][1,3]dioxo heterocyclopenten-4-)- 3-(morpholinomethyl)benzamide (I-34)

Compound VI-34 (400 mg, 0.72 mmol) and acryloyl chloride (72 mg, 0.79 mmol) were used as raw materials, the procedure was the same as that of compound I-4 to obtain 225 mg of a white solid with a yield of 46.67%, m.p. at 148.0-150.0°C. $^1$H-NMR (300MHz, DMSO), δ(ppm): 10.25 (s, 1H, NHCO), 8.24 (s, 1H, ArH), 7.93 (s, 1H, ArH), 7.92-7.87 (m, 1H, ArH)7.57-7.53 (m, 1H, ArH), 7.50 (t, *J*=7.44Hz, 1H, ArH), 7.18 (d, *J*=8.58Hz, 1H, ArH), 7.05 (d, *J*=8.55Hz, 1H, ArH), 6.93-6.71 (m, 1H, $C\underline{H}=CH_2$), 6.14 (s, 2H, $OCH_2O$), 6.13-6.03 (m, 1H, $CH=C\underline{H}_2$), 5.78 (s, 2H, $NH_2$), 5.75-5.59 (m, 1H, $CH=C\underline{H}_2$), 4.78-4.65 (m, 1H, $C\underline{H}_2CHNBoc$), 4.62-4.53 (m, 0.5H, $CH_2C\underline{H}NBoc$), 4.31-4.18 (m, 1H, $C\underline{H}_2CHNBoc$ ), 4.15-4.08 (m, 0.5H, $CH_2C\underline{H}NBoc$), 3.74-3.64 (m, 1H, $NC\underline{H}_2CH_2$), 3.63-3.58 (m, 4H, $C\underline{H}_2NC\underline{H}_2$), 3.57-3.54 (s, 2H, $CH_2$), 3.27-3.20 (m, 1H, $NC\underline{H}_2CH_2$), 2.45-2.35 (m, 4H, $C\underline{H}_2OC\underline{H}_2$), 2.31-2.21 (m, 1H, $NCH_2CHC\underline{H}_2$), 2.17-2.08(m, 1H, $NCH_2CHC\underline{H}_2$), 1.98-1.88 (m, 1H, $NCH_2C\underline{H}_2$), 1.69-1.52 (m, 1H, $NCH_2C\underline{H}_2$)

Example 35

The pharmacological trials and results of the compounds as prepared as above-mentioned are as follows:

1. BTK, JAK3 Kinase Inhibitory Activity Experiment

Experimental method: The 384-well plate was divided into compound wells (starting at 10 μM, diluted three times to produce 10 concentrations), positive control wells and negative control wells; kinase solution was added to compound wells and positive control wells, and 1×kinase buffer was added to negative control wells; the solution is next centrifuged for 30 seconds and incubated at room temperature for 10 min. Add 15 μL of the mixed solution of ATP and substrate, shake and mix well after centrifugation and incubate at room temperature (30 min for JAK3, 20 min for BTK). Add stop detection solution to stop the reaction, and read the conversion rate with Caliper EZ reader, Data analysis: The formula is as follows:

$$\%\ \text{Inhibition} = \frac{Conversion\ \%_\max - Conversion\ \%_sample}{Conversion\ \%_\max - Conversion\ \%_min} \times 100$$

where Conversion %_sample is the conversion rate reading of the sample; Conversion %_min is the average value of the negative control wells representing the conversion rate reading of the wells without enzyme addition; and Conversion % _max is the average value of the positive control wells representing the conversion rate reading of the wells without adding the compound.

Fit the dose-response curve: Taking the log value of the concentration as the X-axis, and the percentage inhibition rate as the Y-axis, the dose-response curve was fitted using the log (inhibitor) vs. response-Variable slope of the software GraphPad Prism 5, so as to obtain the $IC_{50}$ value of each compound on the enzyme activity.

Experimental results: Some compounds of the invention were screened for in vitro BTK and JAK3 kinase inhibitory activity; the results are shown in Table 1.

Table 1. Inhibitory activity of some compounds on BTK and JAK3 kinases

| Serial number | BTK ($IC_{50}$:nM) | JAK3 ($IC_{50}$:nM) | Serial number | BTK ($IC_{50}$:nM) | JAK3 ($IC_{50}$:nM) |
|---|---|---|---|---|---|
| I-1 | A | B | I-17 | A | A |
| I-2 | A | B | I-18 | A | A |
| I-3 | A | B | I-19 | A | A |
| I-4 | A | B | I-20 | A | A |
| I-5 | A | B | I-21 | A | A |
| I-6 | A | A | I-22 | A | A |

(continued)

| Serial number | BTK ($IC_{50}$:nM) | JAK3 ($IC_{50}$:nM) | Serial number | BTK ($IC_{50}$:nM) | JAK3 ($IC_{50}$:nM) |
|---|---|---|---|---|---|
| I-7 | A | A | I-23 | A | B |
| I-8 | A | B | I-24 | A | B |
| I-9 | A | B | I-25 | A | B |
| I-10 | A | A | I-26 | A | B |
| I-11 | A | A | I-28 | A | B |
| I-12 | A | B | I-31 | A | B |
| I-13 | A | A | I-32 | A | A |
| I-14 | A | A | I-33 | A | C |
| I-15 | A | A | I-34 | A | C |
| I-16 | A | A | Ibrutinib | A | B |
| Note: A: $IC_{50}$:0.001-0.05 μM; B: $IC_{50}$:0.05-0.5 μM; C: $IC_{50}$:>0.5 μM. | | | | | |

The results in Table 1 showed that the compounds of the invention had good inhibitory activity to BTK and JAK3 kinases, where compounds I-6, I-7, I-10, I-11, I-13-I-22 and I-32 showed excellent inhibitory activity on BTK and JAK3.

2. Proliferation inhibitory effect of compounds on BaF3-TEL-JAK3 and Daudi cells

Experimental procedure: A certain number of cells in the logarithmic growth phase were inoculated in a 96-well plate and cultured at 37°C before adding drugs. After 72 h of drug action, after the 96-well cell plate was equilibrated to room temperature, 40 μL of Cell Titer-Glo® reagent was added to each well; each plate was then shaken for 2 min and allowed to stand for 10 min; the chemiluminescent signal was detected with an MD SpectraMax Paradigm microplate reader.

Data analysis: The formula is as follows:

$$\%\ \text{Inhibition} = \frac{(Max\ \ signal - Compound\ \ signal)}{(Max\ \ signal - Min\ \ signal)} \times 100$$

Max signal: DMSO signal; Min signal: medium signal.

$IC_{50}$ was calculated using GraphPad Prism 5.

Table 2. Proliferation inhibitory activity of some compounds on BaF3-JAK3 and Daudi cells

| Compound number | $IC_{50}$ (nM) | |
|---|---|---|
| | BaF3-JAK3 | Daudi |
| I-1 | B | B |
| I-2 | B | B |
| I-3 | B | B |
| I-4 | B | B |
| I-6 | B | B |
| I-7 | B | B |
| I-11 | A | A |
| I-13 | A | B |
| I-15 | A | B |
| I-16 | A | A |
| I-17 | B | B |
| I-18 | B | B |
| I-20 | B | B |

(continued)

| Compound number | $IC_{50}$ (nM) | |
|---|---|---|
| | BaF3-JAK3 | Daudi |
| I-21 | B | B |
| I-24 | A | A |
| Ibrutinib | C | B |
| Note: A: $IC_{50}$: 0.01-0.5μM, B: $IC_{50}$: 0.5-1μM, C: $IC_{50}$:>1μM. | | |

The experimental results showed that the tested compounds had significantly better inhibitory activity on BaF3-JAK3 cell proliferation than ibrutinib; the test compounds showed better proliferation inhibitory ability to Daudi cells with high expression of BTK, and some test compounds had better proliferation inhibitory effect on Daudi cells than ibrutinib.

## Claims

1. A compound of general formula (I) or a pharmaceutically acceptable salt thereof:

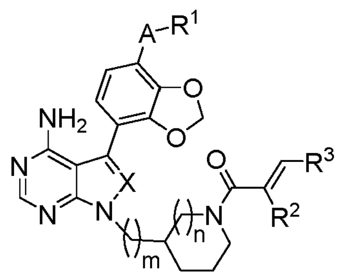

(I)

wherein X represents N or CH; A represents -NHCO-, or -$NHSO_2$-; *m* represents 0, and *n* represents 1; $R^1$ represents:

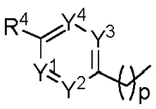 , 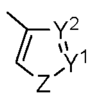 , 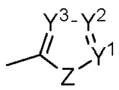 , 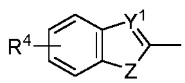 , 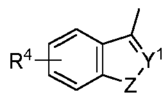 , 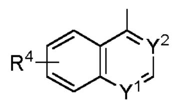

or

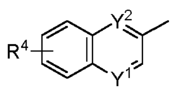 ,

wherein p represents 0 or 1; $R^4$ represents H, F, Cl, Br, I, C1-C6 alkyl, $CF_3$, OH, C1-C6 alkoxy, $OCF_3$, CN, $NO_2$, $NH_2$, C1-C6 alkylamine, $N(CH_3)_2$, $N(C_2H_5)_2$, $NHCOCH_3$, $CONH_2$, $CONHCH_3$, $CONHCH_2CF_3$, $OCH_2CH_2OCH_3$ or $C_6H_5$, and $R^4$ can be mono-, double-or triple-substituted; $Y^1$, $Y^2$, $Y^3$, $Y^4$ represent N or C-$R^5$, $R^5$ represents H, F, Cl, Br, I, $CH_3$, $CF_3$, OH, $OCH_3$, $OCF_3$ or CN; Z represents O, S or N-$R^6$, $R^6$ represents H, $CH_3$, $C_2H_5$ or cyclopropyl; $R^2$ and $R^3$ represent H.

2. The compound or the pharmaceutically acceptable salt thereof according to claim 1, wherein the compound has the following general structural formula (II):

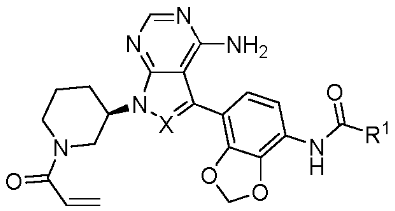

(II)

wherein definitions of X and $R^1$ are the same as those in claim 1.

**3.** The compound or the pharmaceutically acceptable salt thereof according to claim 2, wherein $R^1$ represents:

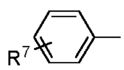 , 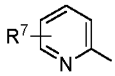 , 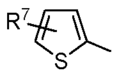 , 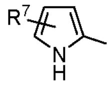 , 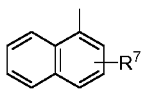 , 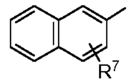 , 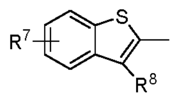 ,

or

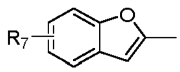 ,

wherein $R^7$ represents H, F, Cl, Br, $CH_3$, t-Bu, $CF_3$, CN, OH, $OCH_3$, $OCF_3$, $NH_2$, $N(CH_3)_2$, $N(C_2H_5)_2$, $NHCOCH_3$, $CONH_2$, $CONHCH_3$, $CONHCH_2CF_3$, or $C_6H_5$, $R^7$ can be mono-, double- or triple-substituted, and $R^8$ represents H, Cl or $CH_3$.

**4.** The compound or the pharmaceutically acceptable salt thereof according to claim 3, wherein $R^7$ represents H, Cl, CH3, t-Bu, $CF_3$, $OCH_3$, $N(CH_3)_2$, $N(C_2H_5)_2$ or $CONHCH_2CF_3$, $R^7$ can be mono-, double- or triple-substituted, and $R^8$ represents Cl.

**5.** The compound or the pharmaceutically acceptable salt thereof according to claim 1, wherein the compound is

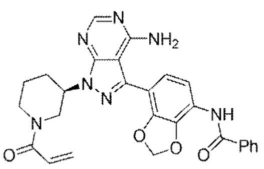

I-1

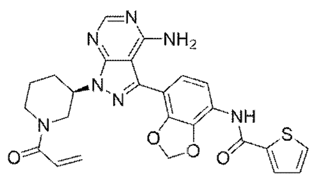

I-2

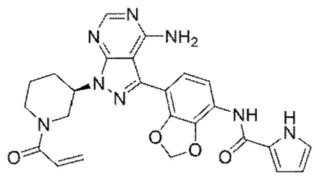

I-3

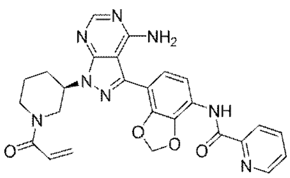

I-4

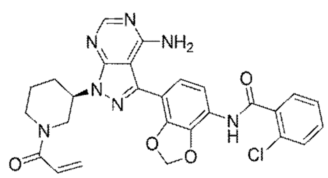

I-5

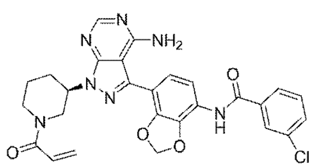

I-6

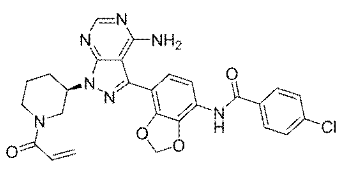

I-7

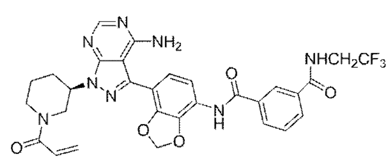

I-8

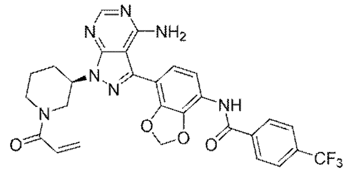

I-9

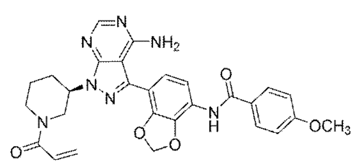

I-10

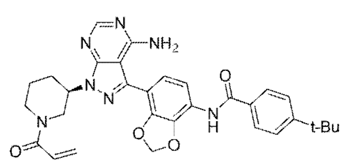

I-11

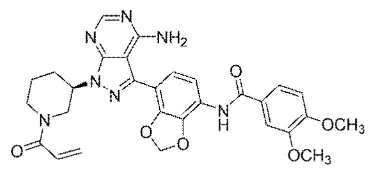

I-12

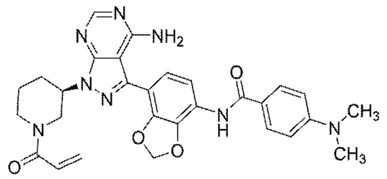

I-13

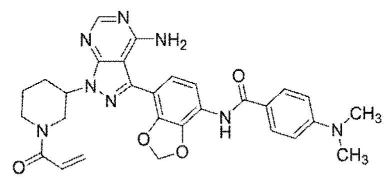

I-14

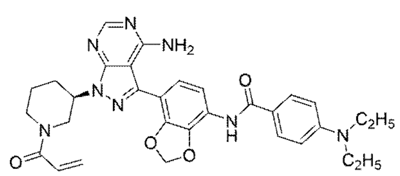

I-15

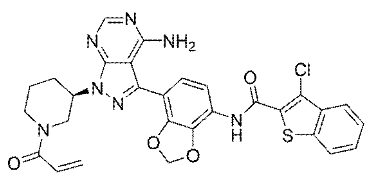

I-16

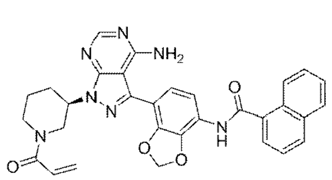

I-17

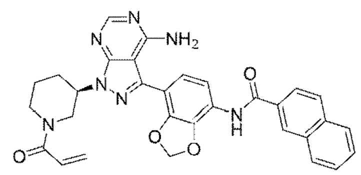

I-18

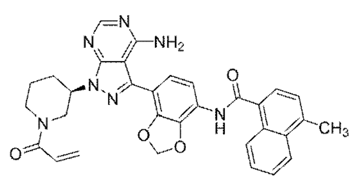

I-19

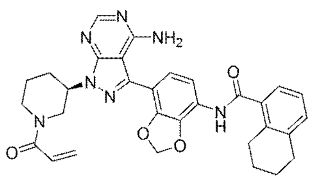

I-20

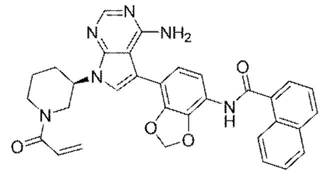

I-21

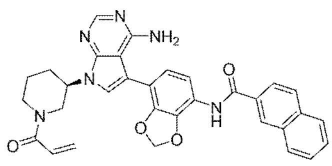

I-22

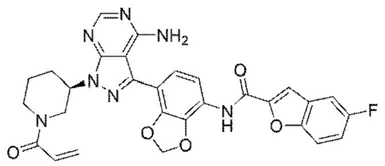

I-31

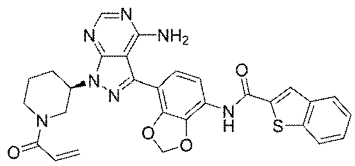

I-32.

**6.** The compound or the pharmaceutically acceptable salt thereof according to claim 1, wherein the pharmaceutically acceptable salt is an acid addition salt formed by the compound of general formula (I) according to claim 1 and the following acids: hydrogen chloride, hydrogen bromide, sulfuric acid, carbonic acid, oxalic acid, citric acid, succinic acid, tartaric acid, phosphoric acid, lactic acid, pyruvic acid, acetic acid, maleic acid, methanesulfonic acid, benzenesulfonic acid, p-toluenesulfonic acid, or ferulic acid.

**7.** A pharmaceutical composition, comprising the compound or the pharmaceutically acceptable salt thereof according to any one of claims 1 to 6 and a pharmaceutically acceptable carrier.

**8.** A preparation method for the compound according to claim 1, wherein a chemical reaction route is as follows:

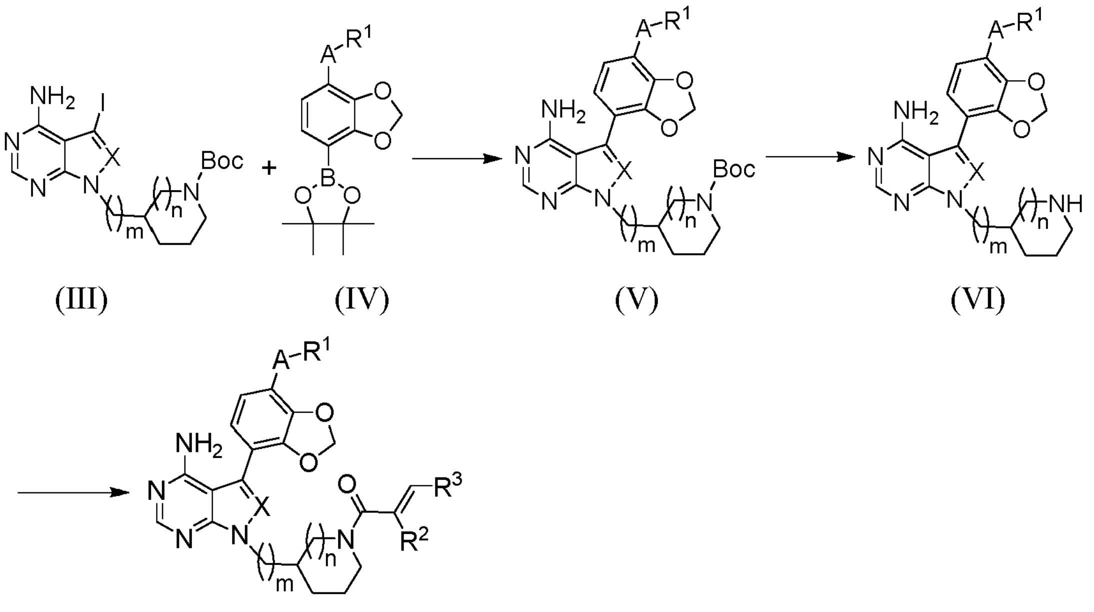

(III) (IV) (V) (VI)

(I)

where definitions of $R^1$, $R^2$, $R^3$, X, A, *m* and *n* are the same as those in claim 1.

**9.** The preparation method according to claim 8, further comprising a preparation step of a compound of general formula (III), wherein a chemical reaction route of the step is as follows:

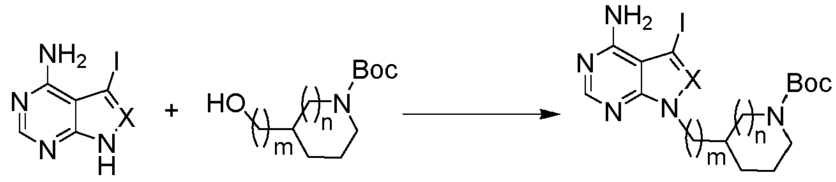

(VII) (VIII) (III)

wherein definitions of X, *m* and *n* are the same as those in claim 1.

**10.** The preparation method according to claim 9, further comprising a preparation step of a compound of general formula (IV), wherein a chemical reaction route of the step is as follows:

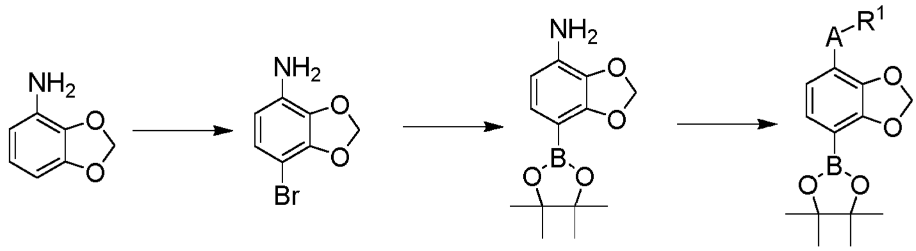

(IX) (X) (XI) (IV)

wherein A is selected from -NHCO-, $-NHCOCH_2-$ or $-NHSO_2-$, and a definition of $R^1$ is the same as that in claim 1.

**11.** A use of the compound or the pharmaceutically acceptable salt thereof according to any one of claims 1 to 6 in preparation of drugs such as a BTK and/or JAK3 inhibitor.

**12.** The use according to claim 11, being a use of the compound or the pharmaceutically acceptable salt thereof according to any one of claims 1 to 6 in the preparation of BTK and JAK3 dual-target inhibitor drugs.

**13.** The use according to claim 12, wherein the BTK and JAK3 dual-target inhibitor drugs are for treating rheumatoid arthritis or B-cell lymphoma.

**Patentansprüche**

**1.** Eine Verbindung der allgemeinen Formel (I) oder ein pharmazeutisch akzeptables Salz davon:

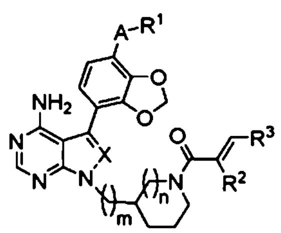

(I),

wobei X N oder CH darstellt; A -NHCO- oder $-NHSO_2-$ darstellt; *m* 0 darstellt und *n* 1 darstellt; wobei $R^1$ Folgendes darstellt:

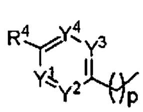 , 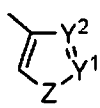 , 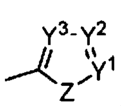 , 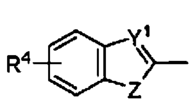 , 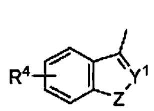 , 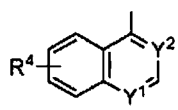

oder

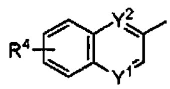

,

wobei *p* 0 oder 1 darstellt; $R^4$ H, F, Cl, Br, I, C1-C6-Alkyl, $CF_3$, OH, C1-C6-Alkoxy, $OCF_3$, CN, $NO_2$, $NH_2$, C1-C6-Alkylamin, $N(CH_3)_2$, $N(C_2H_5)_2$, $NHCOCH_3$, $CONH_2$, $CONHCH_3$, $CONHCH_2CF_3$, $OCH_2CH_2OCH_3$ oder $C_6H_5$ darstellt und $R^4$ mono-, doppelt oder dreifach substituiert sein kann; $Y^1$, $Y^2$, $Y^3$, $Y^4$ N oder C-$R^5$ darstellen, $R^5$ H, F, Cl, Br, I, $CH_3$, $CF_3$, OH, $OCH_3$, $OCF_3$ oder CN darstellt; Z O, S oder N-$R^6$ darstellt, $R^6$ H, $CH_3$, $C_2H_5$ oder Cyclopropyl darstellt; $R^2$ und $R^3$ H darstellen.

2. Verbindung oder pharmazeutisch akzeptables Salz davon gemäß Anspruch 1, wobei die Verbindung die folgende allgemeine Strukturformel (II) aufweist:

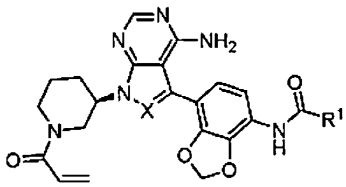

(II),

wobei die Definitionen von X und $R^1$ die gleichen sind wie die in Anspruch 1.

3. Verbindung oder pharmazeutisch akzeptables Salz davon gemäß Anspruch 2, wobei $R^1$ Folgendes darstellt:

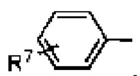 , 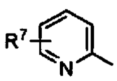 , 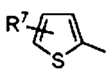 , 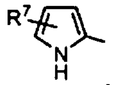 , 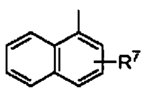 , 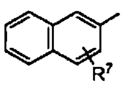 , 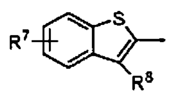

oder

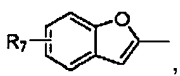

,

wobei $R^7$ **H,** F, Cl, Br, $CH_3$, t-Bu, $CF_3$, CN, OH, $OCH_3$, $OCF_3$, $NH_2$, $N(CH_3)_2$, $N(C_2H_5)_2$, $NHCOCH_3$, $CONH_2$, $CONHCH_3$, $CONHCH_2CF_3$ oder $C_6H_5$ darstellt, $R^7$ mono-, doppelt oder dreifach substituiert sein kann und $R^8$ H, Cl oder $CH_3$ darstellt.

4. Verbindung oder pharmazeutisch akzeptables Salz davon gemäß Anspruch 3, wobei $R^7$ H, Cl, $CH_3$, t-Bu, $CF_3$, $OCH_3$, $N(CH_3)_2$, $N(C_2H_5)_2$ oder $CONHCH_2CF_3$ darstellt, $R^7$ mono-, doppelt oder dreifach substituiert sein kann und $R^8$ Cl darstellt.

5. Verbindung oder pharmazeutisch akzeptables Salz davon gemäß Anspruch 1, wobei die Verbindung Folgendes ist:

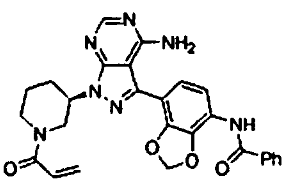

I-1

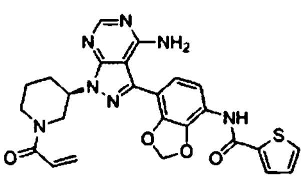

I-2

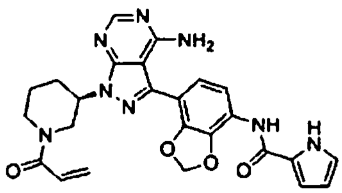

I-3

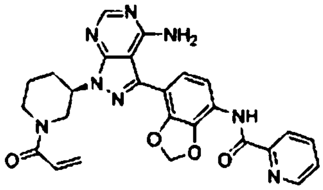
I-4

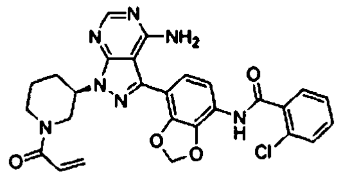
I-5

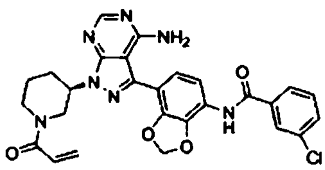
I-6

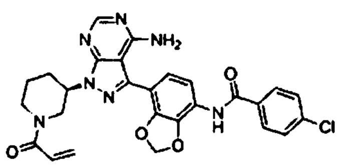
I-7

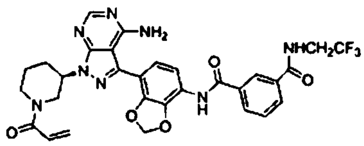
I-8

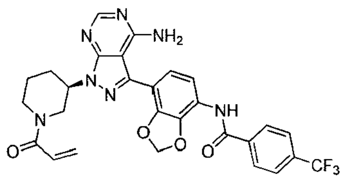
I-9

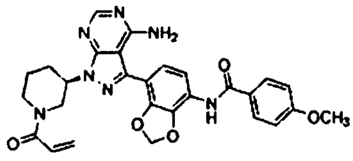
I-10

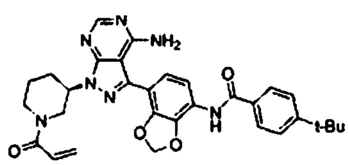
I-11

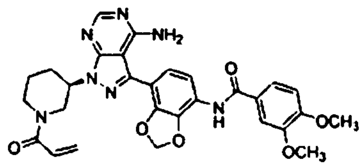
I-12

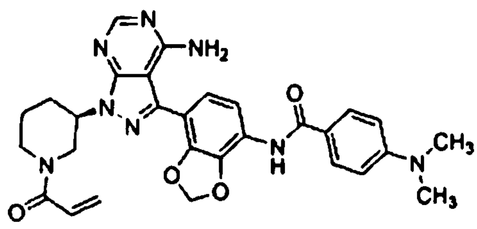
I-13

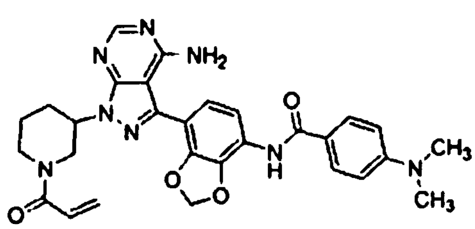
I-14

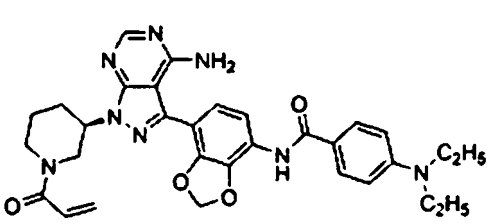
I-15

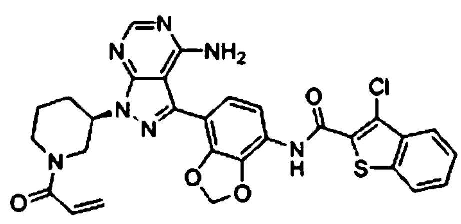
I-16

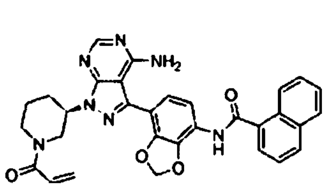
I-17

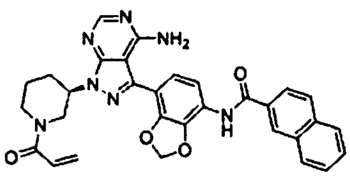
I-18

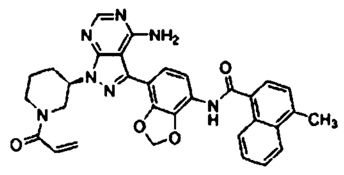
I-19

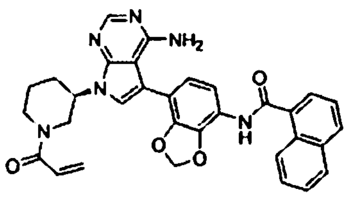 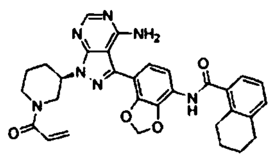 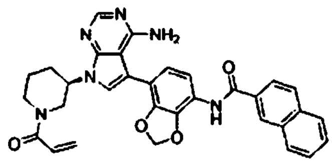

I-20 I-21 I-22

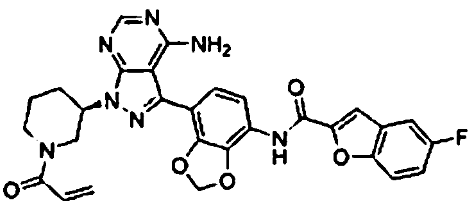 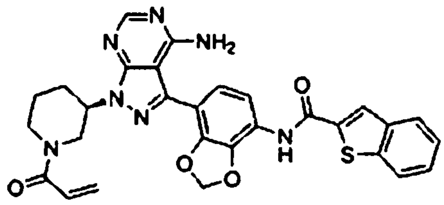

I-31 I-32.

6. Verbindung oder pharmazeutisch akzeptables Salz davon gemäß Anspruch 1, wobei das pharmazeutisch akzeptable Salz ein Säureadditionssalz ist, das durch die Verbindung der allgemeinen Formel (I) gemäß Anspruch 1 und die folgenden Säuren gebildet wird: Chlorwasserstoff, Bromwasserstoff, Schwefelsäure, Kohlensäure, Oxalsäure, Zitronensäure, Bernsteinsäure, Weinsäure, Phosphorsäure, Milchsäure, Brenztraubensäure, Essigsäure, Maleinsäure, Methansulfonsäure, Benzolsulfonsäure, p-Toluolsulfonsäure oder Ferulasäure.

7. Eine pharmazeutische Zusammensetzung, die die Verbindung oder das pharmazeutisch akzeptable Salz davon gemäß einem der Ansprüche 1 bis 6 und einen pharmazeutisch akzeptablen Träger beinhaltet.

8. Ein Herstellungsverfahren für die Verbindung gemäß Anspruch 1, wobei ein chemischer Reaktionsweg wie folgt ist:

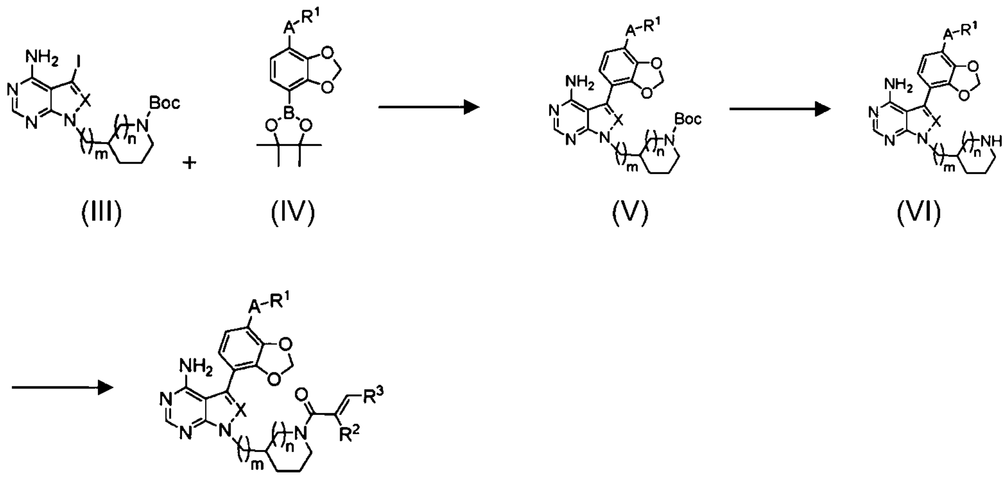

(III) + (IV) (V) (VI)

(I),

wobei die Definitionen von $R^1$, $R^2$, $R^3$, X, A, *m* und *n* die gleichen sind wie die in Anspruch 1.

9. Herstellungsverfahren gemäß Anspruch 8, das ferner einen Herstellungsschritt einer Verbindung der allgemeinen Formel (III) beinhaltet, wobei ein chemischer Reaktionsweg des Schritts wie folgt ist:

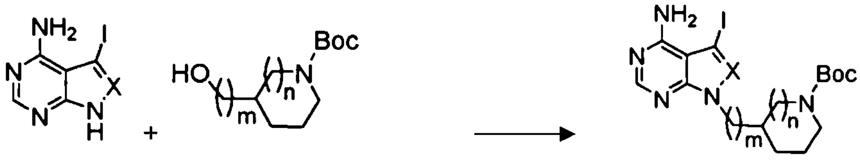

(VII) (VIII) (III),

wobei die Definitionen von X, m und n die gleichen sind wie die in Anspruch 1.

**10.** Herstellungsverfahren gemäß Anspruch 9, das ferner einen Herstellungsschritt einer Verbindung der allgemeinen Formel (IV) beinhaltet, wobei ein chemischer Reaktionsweg des Schritts wie folgt ist:

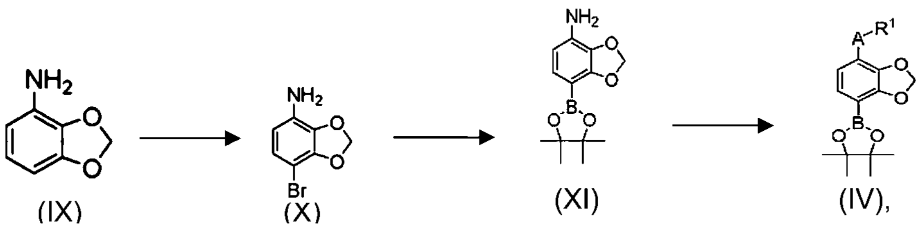

(IX) (X) (XI) (IV),

wobei A ausgewählt ist aus -NHCO-, $-NHCOCH_2-$ oder $-NHSO_2-$ und eine Definition von $R^1$ die gleiche ist wie die in Anspruch 1.

**11.** Eine Verwendung der Verbindung oder des pharmazeutisch akzeptablen Salzes davon gemäß einem der Ansprüche 1 bis 6 bei der Herstellung von Arzneimitteln wie einem BTK- und/oder JAK3-Inhibitor.

**12.** Verwendung gemäß Anspruch 11, die eine Verwendung der Verbindung oder des pharmazeutisch akzeptablen Salzes davon gemäß einem der Ansprüche 1 bis 6 bei der Herstellung von BTK- und JAK3-Dual-Target-Inhibitor-Arzneimitteln ist.

**13.** Verwendung gemäß Anspruch 12, wobei die BTK- und JAK3-Dual-Target-Inhibitor-Arzneimittel zur Behandlung von rheumatoider Arthritis oder B-Zell-Lymphom sind.

**Revendications**

**1.** Un composé de formule générale (I) ou un sel pharmaceutiquement acceptable de celui-ci :

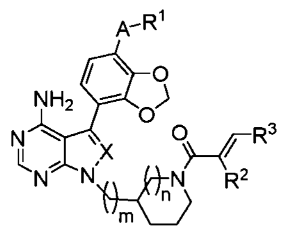

(I)

où X représente N ou CH ; A représente -NHCO-, ou $-NHSO_2-$ ; *m* représente 0, et *n* représente 1 ; $R^1$ représente :

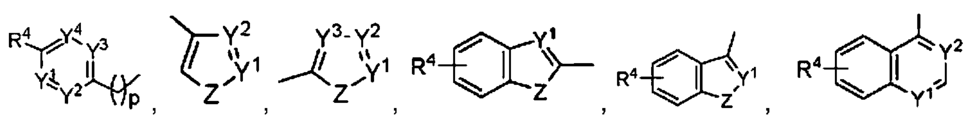

ou

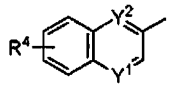

,

où p représente 0 ou 1 ; $R^4$ représente H, F, Cl, Br, I, alkyle en C1-C6, $CF_3$, OH, alcoxy en C1-C6, $OCF_3$, CN, $NO_2$, $NH_2$, alkylamine en C1-C6, $N(CH_3)_2$, $N(C_2H_5)_2$, $NHCOCH_3$, $CONH_2$, $CONHCH_3$, $CONHCH_2CF_3$, $OCH_2CH_2OCH_3$ ou $C_6Hs$, et $R^4$ peut être mono-, double- ou triple-substitué ; $Y^1$, $Y^2$, $Y^3$, $Y^4$ représentent N ou C-$R^5$, $R^5$ représente H, F, Cl, Br, I, $CH_3$, $CF_3$, OH, $OCH_3$, $OCF_3$ ou CN ; Z représente O, S ou N-$R^6$, $R^6$ représente H, $CH_3$, $C_2H_5$ ou cyclopropyle ; $R^2$ et $R^3$ représentent H.

**2.** Le composé ou le sel pharmaceutiquement acceptable de celui-ci selon la revendication 1, où le composé a la formule structurale générale (II) suivante :

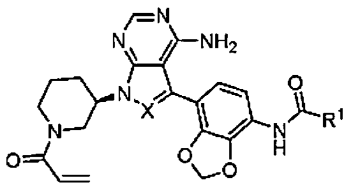

(II)

où les définitions de X et $R^1$ sont les mêmes que celles dans la revendication 1.

**3.** Le composé ou le sel pharmaceutiquement acceptable de celui-ci selon la revendication 2, où $R^1$ représente :

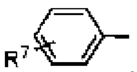 , 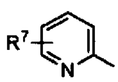 , 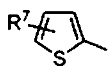 , 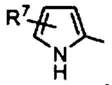 , 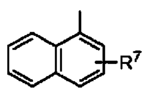 , 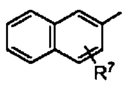 , 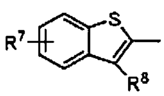 ,

ou

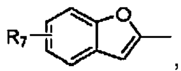

,

où $R^7$ représente H, F, Cl, Br, $CH_3$, t-Bu, $CF_3$, CN, OH, $OCH_3$, $OCF_3$, $NH_2$, $N(CH_3)_2$, $N(C_2H_5)_2$, $NHCOCH_3$, $CONH_2$, $CONHCH_3$, $CONHCH_2CF_3$, ou $C_6H_5$, $R^7$ peut être mono-, double- ou triple-substitué, et $R^8$ représente H, Cl ou $CH_3$.

**4.** Le composé ou le sel pharmaceutiquement acceptable de celui-ci selon la revendication 3, où $R^7$ représente H, Cl, $CH_3$, t-Bu, $CF_3$, $OCH_3$, $N(CH_3)_2$, $N(C_2H_5)_2$ ou $CONHCH_2CF_3$, $R^7$ peut être mono-, double- ou triple-substitué, et $R^8$ représente Cl.

**5.** Le composé ou le sel pharmaceutiquement acceptable de celui-ci selon la revendication 1, où le composé est

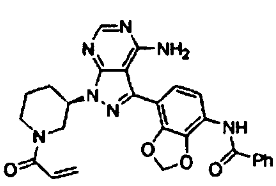 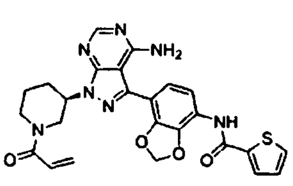 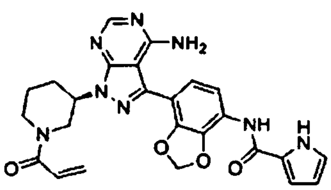

I-1 I-2 I-3

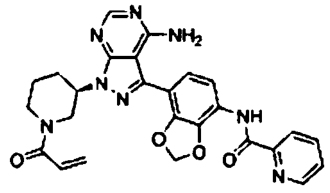

I-4

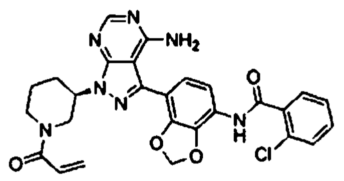

I-5

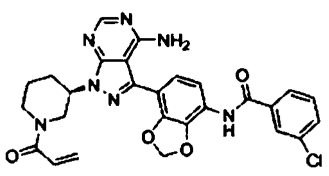

I-6

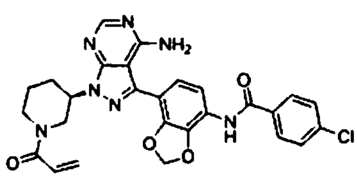

I-7

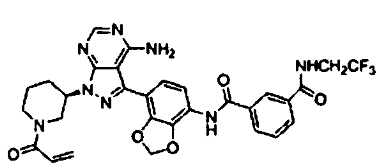

I-8

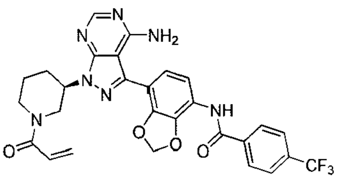

I-9

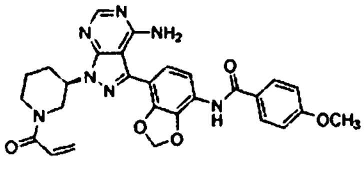

I-10

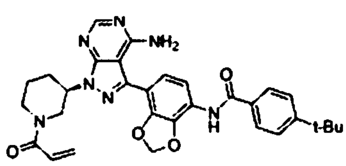

I-11

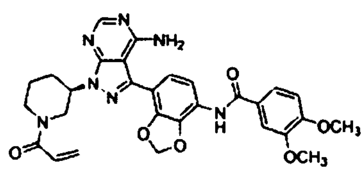

I-12

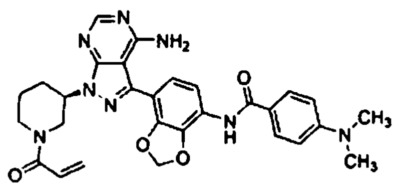

I-13

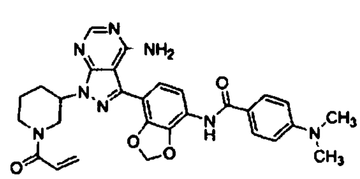

I-14

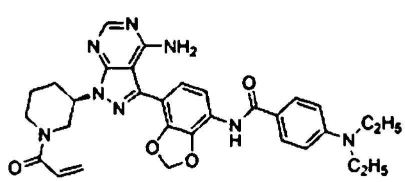

I-15

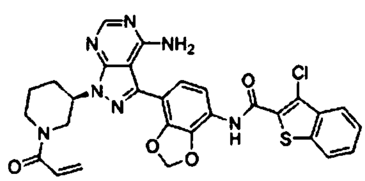

I-16

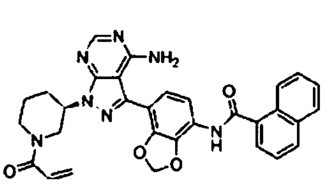

I-17

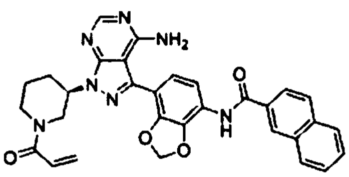

I-18

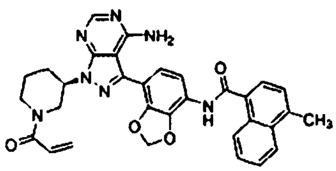

I-19

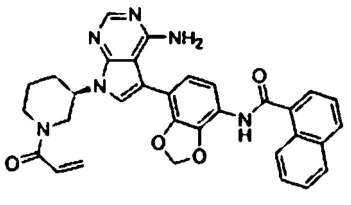

I-20

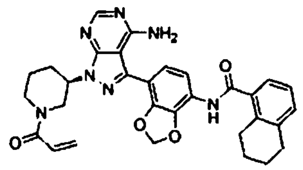

I-21

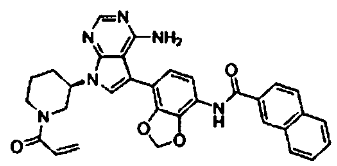

I-22

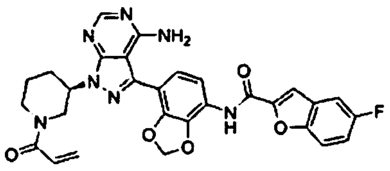

I-31

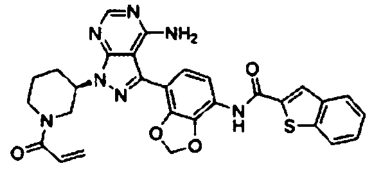

I-32.

**6.** Le composé ou le sel pharmaceutiquement acceptable de celui-ci selon la revendication 1, où le sel pharmaceutiquement acceptable est un sel d'addition acide formé par le composé de formule générale (I) selon la revendication 1 et les acides suivants : chlorure d'hydrogène, bromure d'hydrogène, acide sulfurique, acide carbonique, acide oxalique, acide citrique, acide succinique, acide tartrique, acide phosphorique, acide lactique, acide pyruvique, acide acétique, acide maléique, acide méthanesulfonique, acide benzènesulfonique, acide p-toluènesulfonique, ou acide férulique.

**7.** Une composition pharmaceutique, comprenant le composé ou le sel pharmaceutiquement acceptable de celui-ci selon l'une quelconque des revendications 1 à 6 et un support pharmaceutiquement acceptable.

**8.** Un procédé de préparation pour le composé selon la revendication 1, où une trajectoire de réaction chimique est comme suit :

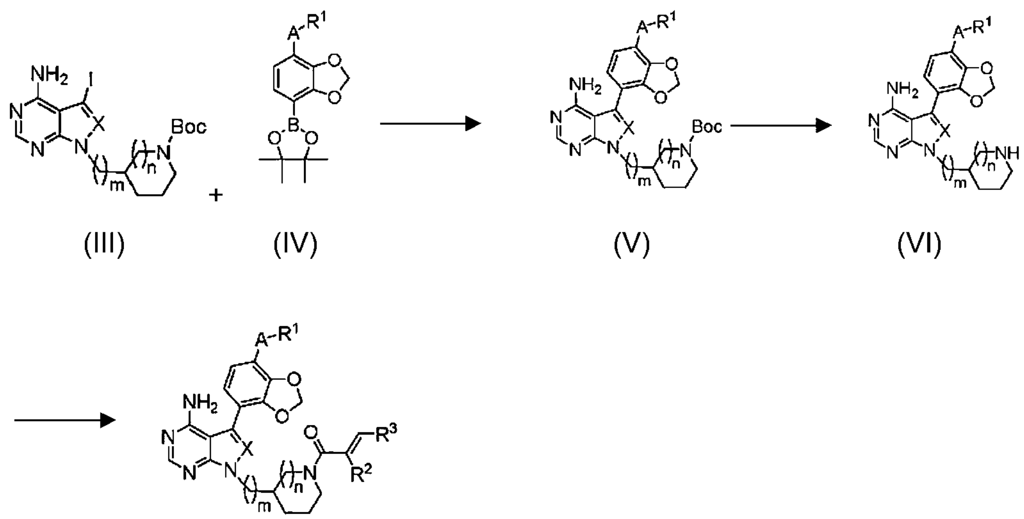

(III) (IV) (V) (VI)

(I)

où les définitions de $R^1$, $R^2$, $R^3$, X, A, m et n sont les mêmes que celles dans la revendication 1.

**9.** Le procédé de préparation selon la revendication 8, comprenant en outre une étape de préparation d'un composé de formule générale (III), où une trajectoire de réaction chimique de l'étape est comme suit :

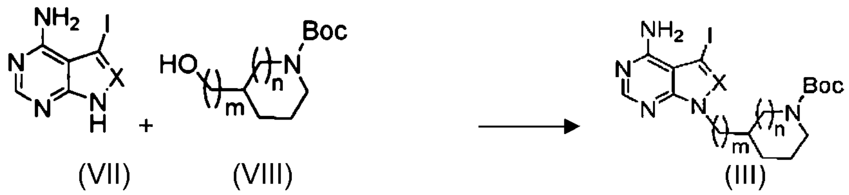

(VII) (VIII) (III)

où les définitions de X, m et n sont les mêmes que celles dans la revendication 1.

**10.** Le procédé de préparation selon la revendication 9, comprenant en outre une étape de préparation d'un composé de formule générale (IV), où une trajectoire de réaction chimique de l'étape est comme suit :

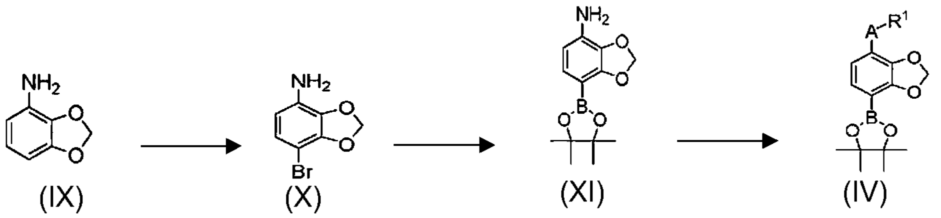

(IX) (X) (XI) (IV)

où A est choisi parmi -NHCO-, $-NHCOCH_2-$ ou $-NHSO_2-$, et une définition de R' est la même que celle dans la revendication 1.

**11.** Une utilisation du composé ou du sel pharmaceutiquement acceptable de celui-ci selon l'une quelconque des revendications 1 à 6 dans la préparation de médicaments tels qu'un inhibiteur de BTK et/ou de JAK3.

**12.** L'utilisation selon la revendication 11, étant une utilisation du composé ou du sel pharmaceutiquement acceptable de celui-ci selon l'une quelconque des revendications 1 à 6 dans la préparation de médicaments inhibiteurs à double cible de BTK et de JAK3.

**13.** L'utilisation selon la revendication 12, où les médicaments inhibiteurs à double cible de BTK et de JAK3 sont destinés au traitement de la polyarthrite rhumatoïde ou du lymphome à cellules B.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 109970740 A
- WO 2011090760 A1
- CN 108329321 A
- CN 108484609 A
- US 2011039868 A
- WO 2016019233 A1
- WO 2020231990 A1


### Non-patent literature cited in the description


- **YUAN YIN et al.** Structure-based design and synthesis of 1Hpyrazolo[3,4-d]pyrimidin-4-amino derivatives as Janus kinase 3 inhibitors. *Bioorg. & Med. Chem.*, 01 September 2018, vol. 26 (17), 4774-4786